(19) Europäisches Patentamt
European Patent Office
Office européen des brevets

(11) Publication number: 0 237 138 A1

(12) EUROPEAN PATENT APPLICATION

(21) Application number: 87300109.3

(22) Date of filing: 07.01.87

(51) Int.Cl.[3]: C 07 D 417/04
C 07 D 277/36, C 07 D 417/14
C 07 D 403/04, C 07 D 409/04
C 07 D 413/04, A 61 K 31/425
A 61 K 31/415, A 61 K 31/55
A 61 K 31/42

(30) Priority: 07.01.86 JP 1847/86
12.08.86 JP 189850/86

(43) Date of publication of application:
16.09.87 Bulletin 87/38

(84) Designated Contracting States:
AT BE CH DE FR GB IT LI LU NL SE

(71) Applicant: YAMANOUCHI PHARMACEUTICAL CO. LTD.
No. 5-1 Nihonbashi-Honcho, 2-chome Chuo-ku
Tokyo(JP)

(72) Inventor: Niigata, Kunihiro
335-12, Ageo-shi Nakabun
Saitama(JP)

(72) Inventor: Okada, Minoru
10-20, Higashi Ohi 6-chome Shinagawa-ku
Tokyo(JP)

(72) Inventor: Yoneda, Takashi
Dai 2 Sanraizu Manshon 406 26-16
Takashimadaira 9-chome Itabashi-ku Tokyo(JP)

(74) Representative: Geering, Keith Edwin et al,
REDDIE & GROSE 16 Theobalds Road
London WC1X 8PL(GB)

(54) **Heterocyclic compounds, their preparation and, pharmaceutical compositions containing them.**

(57) A heterocyclic compound selected from those of formula (1), tautomers thereof, and pharmaceutically acceptable salts and esters of formula (I) compounds and tautomers:

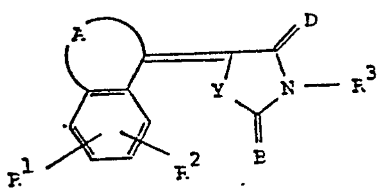

(I)

wherein A represents an alkylene or alkenylene chain which has 1 to 4 carbon atoms and which may have a hetero atom; B, D and Y are selected independently from oxygen and sulfur atoms and NH; $R^1$ and $R^2$ are selected independently from hydrogen and halogen atoms and $C_1$ to $C_5$ alkyl, $C_1$ to $C_5$ alkoxy, $C_1$ to $C_5$ alkylthio, phenyl, nitro, hydroxyl, sulfo, imidazolyl-$C_2$ to $C_5$ acylamimo, amino and carboxyl groups; and $R^3$ represents a hydrogen atom, an amino group, a $C_1$ to $C_5$ alkyl group which may be substituted by a carboxyl group, or a phenyl group which may be substituted by a carboxyl, a carboxy-$C_1$ to $C_5$ alkyl, or an imidazolyl-$C_1$ to $C_5$ alkylamide group. These compounds, particularly when D is oxygen, are aldose reductose inhibitors useful in treatment of chronic diabetic complications.

EP 0 237 138 A1

0237138

# HETEROCYCLIC COMPOUNDS, THEIR PREPARATION AND, PHARMACEUTICAL COMPOSITIONS CONTAINING THEM

The present invention relates to pharmaceutically useful heterocyclic compounds, more particularly, heterocyclic compounds having an aldose reductase-inhibitory activity, methods for their manufacture, and pharmaceutical compositions containing them.

Various compounds have heretofore been synthesized as remedial medicines for diabetes resulting from blood sugar increase due to insulin deficiency. However, medicines sufficiently satisfactory as preventatives or remedies for diabetic complications caused by polyols produced by the action of aldose reductase, for example diabetic cataract, neuropathy and retinopathy, have not been marketed.

It is disclosed in U.S. Patent 4,130,714 that spirohydantoin derivatives represented by the following formula are useful as aldose reductase inhibitors for inhibiting chronic diabetic symptoms:

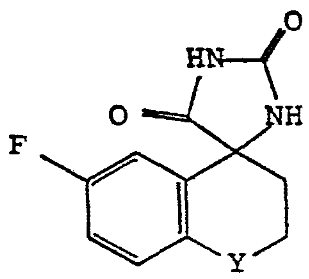

wherein Y represents an oxygen atom or a sulfur atom. Further, European Patent Publication (unexamined) No. 47,109 discloses that rhodanine derivatives represented by the following formula are useful as aldose reductase inhibitors:

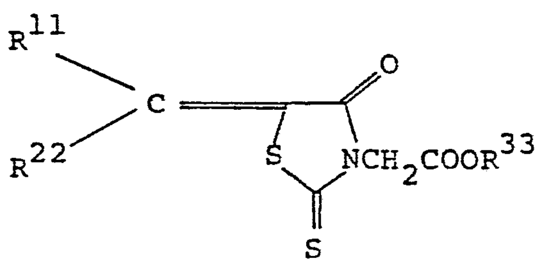

wherein $R^{11}$ and $R^{22}$ form a tetramethylene group or a pentamethylene group, or $R^{11}$ represents a hydrogen atom and $R^{22}$ represents a naphthyl group or a substituted phenyl group; and $R^{33}$ represents a hydrogen atom or an alkyl group, etc.

The heterocyclic compounds of the present invention are structurally quite different from those above, having no spiro ring and having different substituents at the 5-position of the rhodanine ring.

The present invention provides heterocyclic compounds of formula (I), tautomeric isomers thereof, and pharmaceutically acceptable salts and esters of said formula (I) compounds and tautomers:

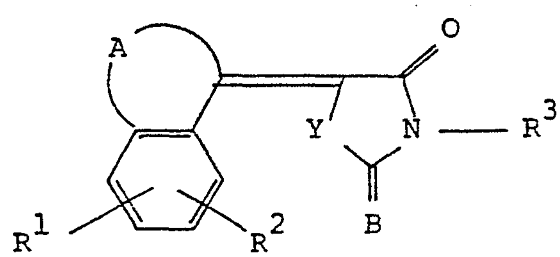

(I)

wherein:

A represents an alkvlene or alkenylene chain having 1 to 4 carbon atoms and which may have a hetero atom;

B, D and Y are selected independently (so that any two or all three may be the same or different) from oxygen and sulfur atoms and NH;

$R^1$ and $R^2$ are selected independently (so as to be the same or different) from hydrogen and halogen atoms and lower alkyl, lower alkoxy, lower alkylthio, phenyl, nitro, hydroxyl, sulfo, imidazolyl-lower acylamino, amino and carboxyl groups; and

$R^3$ represents a hydrogen atom, an amino group, a lower alkyl group which may be substituted by a carboxyl group, or a phenyl group which may be substituted by a carboxyl group, by a carboxy-lower alkyl group, or by an imidazolyl-lower alkylamide group.

The compounds according to the invention, especially those where D is oxygen, inhibit aldose reductase activity and may be useful to prevent or remedy chronic diabetic complications; at least some inhibit platelet aggregation and/or have lipid lowering activity.

Most at least of the preferred compounds are other than:

(a) those where A represents -COO-, $R^1$ and $R^2$ each represents a hydrogen atom, B and Y each represents a sulfur atom or an oxygen atom and $R^3$ represents a hydrogen atom or a phenyl group;

(b) those where A represents -NHCO-, $R^1$ and $R^2$ each represents a hydrogen atom, B and Y each represents a sulfur atom and $R^3$ represents a hydrogen atom or a methyl group; and

(c) those where A represents $-NR^4CO-$ (wherein $R^4$ represents a hydrogen atom, a methyl group or an acetyl group), $R^1$ represents a bromine atom or a hydrogen atom, $R^2$ and $R^3$ each represents a hydrogen atom, Y represents a sulfur atom and B represents an oxygen atom.

Compounds like (a), (b) and (c) are described in Chemical Abstract 74(5)22751a, Chem. Pharm. Bull (1976) 24(10), 2305-11, and Chemical Abstract 92(11)94286x.

Typical groups included in the definitions of the above formula (I) are explained hereunder.

The "hetero atom" in the definition of A includes oxygen, sulfur and nitrogen; the sulfur atom may be substituted by oxygen atom(s) to be present as a sulfinyl (-SO-) or sulfonyl ($-SO_2-$) group, and the nitrogen atom may be present as an imino group (-NH-). One of these hetero atoms may be present in any position of the alkylene or alkenylene chain. Substituents may be on the alkylene or alkenylene chain A, including e.g. lower alkyl, phenyl and oxo groups, halogen atoms and a 2,4-dioxo-5-thiazolidinylidene group (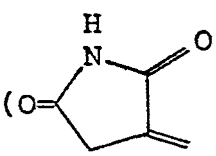).

One, two or more substituents may be on the alkylene or alkenylene chain. Typical examples of A

are as follows:

$-CH_2CH_2-$, $-CH_2CH_2CH_2-$, $-CH_2CH_2CH_2CH_2-$, $-CH_2CH{=}CHCH_2-$,
$-OCH_2-$, $-OCH_2CH_2-$, $-SCH_2-$, $-SCH_2CH_2-$, $-SCH_2CH_2CH_2-$,

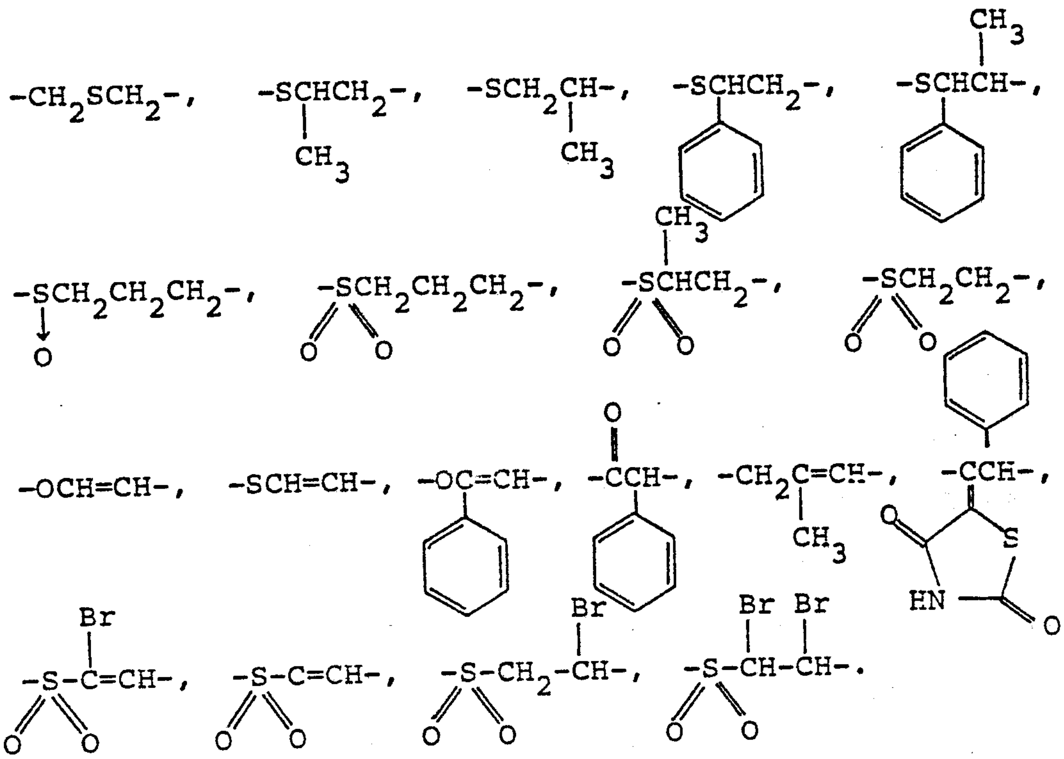

The preferred example of

A is $-SO_2CH_2CH_2$.

The preferred compounds of the present invention are of formula (I-a):

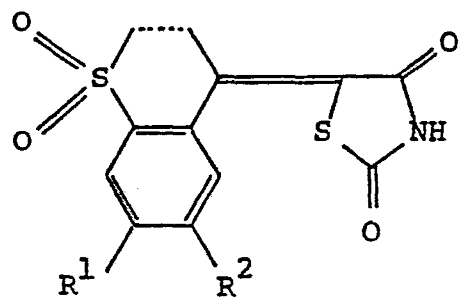

(I-a)

wherein $R^1$ and $R^2$ are the same or different and selected from hydrogen and halogen atoms, and the dotted line means a single or double bond.

The word "lower" as used in "lower alkyl group", "lower alkoxy group" and "lower alkylthio group" means herein a linear or branched carbon chain having 1 to 5 carbon atoms. Accordingly, lower alkyl includes, for example, methyl, ethyl, propyl, isopropyl, n-butyl, sec-butyl and pentyl; lower alkoxy (or lower alkylthio) includes, for example, methoxy (methylthio), ethoxy (ethylthio), propoxy (propylthio) and butoxy (butylthio). The word "halogen" herein includes chlorine, bromine, fluorine and iodine. $R^3$, when a "lower alkyl group which may be substituted by a carboxyl group", includes the above-

mentioned lower alkyl groups having 1 to 5 carbon atoms, unsubstituted or substituted by a carboxyl group at any desired position. Typical examples thereof are carboxymethyl ($-CH_2COOH$), carboxyethyl ($-CH_2CH_2COOH$) and carboxypropyl groups ($-CH_2CH_2CH_2COOH$). The carboxyl group may be in the form of an ester or salt thereof. The esters include lower alkylesters such as methyl-, ethyl- and propyl-ester, aralkyl-esters such as benzyl-ester, and cycloalkyl-esters having 5 to 6 carbon atoms.

The "phenyl group which may be substituted by a carboxy group (or a carboxy-lower alkyl group)" includes, for example, p-carboxyphenyl ( 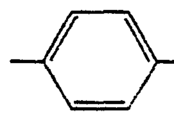 COOH), m-carboxyphenyl p-(carboxymethyl)phenyl ( 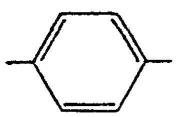 $CH_2COOH$) and p-(carboxyethyl)phenyl groups. The carboxyl group in these groups may also be in the form of an above ester or salt thereof.

The "imidazolyl-lower acylamino group" (lower acyl here having 2 to 5 carbon atoms) includes, for example, imidazolylacetamino, imidazolylpropionylamino and imidazolylbutylamino groups. The "imidazolyl-lower alkylamide" group in the above definition of $R^3$ includes for example imidazolylacetamide and imidazolylbutylamide.

The compounds (I) of the present invention include cis-trans isomers due to the a-moiety and b-moiety bound via the double bond, as shown below:

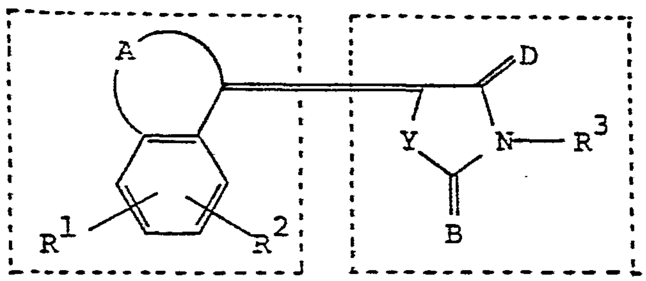

a b

In addition, prototropy may occur in the double bond between the above a-moiety and b-moiety, depending upon the nature of the adjacent atomic groups, or the double bond may be converted into a single bond when the compounds are processed by additional treatments as mentioned hereinafter. In these cases, the linking carbon atom in the b-moiety is asymmetric and the compounds have optical isomers. The present invention includes all tautomers and isomers, individually and in any combination.

The compounds of the present invention may form

salts and esters the present invention includes any and every pharmaceutically acceptable such salt and ester.

Examples of the salts are alkali metal salts such as lithium, sodium and potassium salts, alkaline earth metal salts such as magnesium and calcium salts, aluminum salts, and salts with an organic base such as ammonia, trimethylamine and triethylamine. These salts may be obtained in conventional manner.

Compounds of formula (I) can be obtained by various methods, and typical methods are illustrated hereinafter.

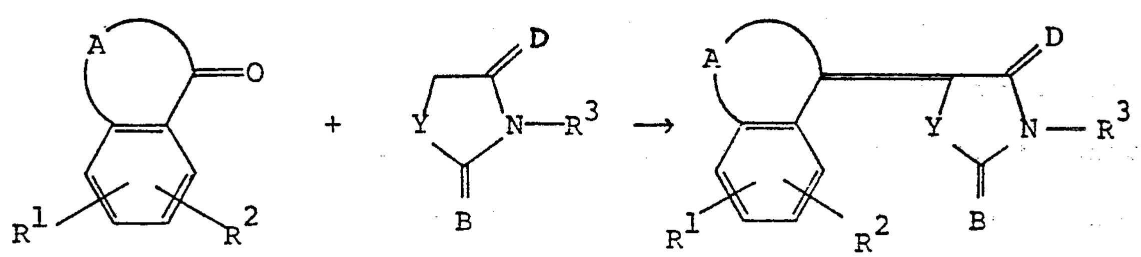

(II) (III) (I)

In the above formulae (II) and (III), $R^1$, $R^2$, $R^3$, A, D, B and Y have the same meanings as in formula (I).

According to the above reaction formula, compounds (I) can be prepared by reacting ketone compounds (II) and heterocyclic compounds (III).

The reaction is usually carried out in a solvent of acetic acid, or aromatic hydrocarbon such as toluene or xylene, or alcohol such as methanol or ethanol, or cyclic ether such as tetrahydrofuran or dioxane, with cooling or heating. In order to accelerate the reaction, a basic compound such as 1,8-diazabicyclo[5,4,0]-undeca-7-ene (DBU), 1,5-diazabicyclo[4,3,0]nona-5-ene (DBN), piperidine, diethylamine, ammonia, ammonium acetate or ammonium carbonate, or a Lewis acid such as boron trifluoride or titanium tetrachloride, is preferably added to the reaction system. When a basic compound is used as reaction accelerator, the starting compounds are usually reacted in a solvent such as acetic acid, aromatic hydrocarbons or alcohols, at 50°C to 200°C. When boron trifluoride, titanium tetrachloride or the like is used as a reaction accelerator, the compounds are usually reacted in a cyclic ether solvent such as dioxane, tetrahydrofuran, etc., or an alkyl halide solvent such as chloroform, ethylene chloride, etc. at room temperature or while cooled with ice.

The final products of the present invention can be

used as intermediates for the preparation of other final products. Some typical such reactions are mentioned below. In the preparation described above (reaction of ketone with heterocyclic) and in those which follow, the starting material where appropriate may be a tautomer, salt or ester, and the initial product may be tautomerised and/or converted to or from salt or ester form.

(i) Hydrolysis:

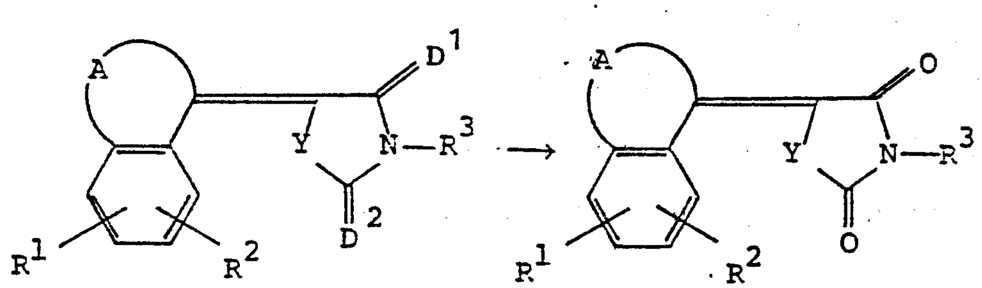

(IV) (I-1)

In the above formula (I-1), $R^1$, $R^2$, $R^3$, A and Y have the same meanings as in formula (I).

According to the above reaction formula, the imine compounds of the formula (IV) or salts thereof, wherein one of $D^1$ and $D^2$ represents an imino group and the other represents an oxygen atom, or both represent imino groups, can be hydrolyzed, e.g. in dilute acid. Sulfuric acid is generally used as the acid, and the reaction is preferably carried out while heating.

(ii) Oxidation:

This is to convert compounds of formula (I) wherein A represents an alkylene or alkenylene chain containing -S- or -SO- to the corresponding oxides (-SO- or $-SO_2-$). The oxidation is generally carried out by treating the compound with hydrogen peroxide. By appropriately regulating the concentration of the hydrogen peroxide and the reaction time, the corresponding mono-oxides or dioxides can be obtained.

Thus heterocyclic compounds of the following formula (I-2), tautomeric isomers thereof and pharmaceutically acceptable salts and esters of these compounds and tautomers:

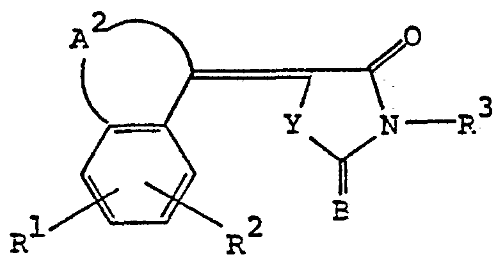

(I-2)

wherein $A^2$ represents a $C_1$ to $C_4$ alkylene or alkenylene chain which includes a sulfinyl or sulfonyl group, and B, Y, $R^1$, $R^2$ and $R^3$ are as in formula (I)

can be produced by oxidizing compounds of formula (V):

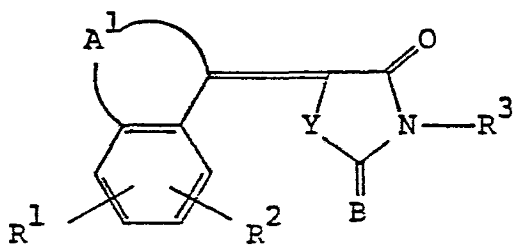

(V)

wherein

$A^1$ represents a $C_1$ to $C_4$ alkylene or alkenylene chain which includes a sulfur atom or a sulfinyl group.

(iii) (a) S → O conversion; (b) O → S conversion:

(a) This is to convert to oxygen the substituent B (when this is a sulfur atom) and/or a thioxo group at the 4-position.

This reaction is generally carried out by heating the starting compound in water together with bromo-acetic acid or chloro-acetic acid. Otherwise, the reaction can be carried out by heating the starting compound in methanol,

ethanol or acetic acid together with Lewis acid such as boron trifluoride, etc. Further, the reaction may also be carried out by treating the starting compound with an oxidizing agent such as chlorine, bromine, iodine, hydrogen peroxide, potassium permanganate, sodium periodate or sodium hypobromite in water.

(b) Conversely when oxygen B is to be converted to sulfur, the reaction can be carried out by treating the starting compound with a Lawesson's reagent.

Thus heterocyclic compounds of formula (I) wherein D is oxygen, tautomeric isomers thereof, and pharmaceutically acceptable salts and esters, can be produced by oxidizing compounds of formula (VI)

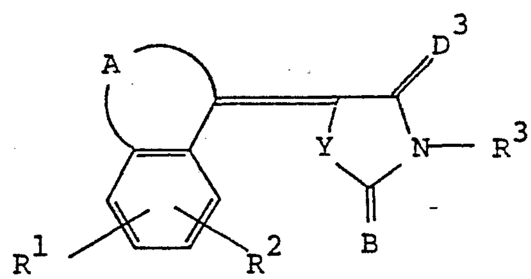

(VI)

wherein A, B, Y, $R^1$, $R^2$ and $R^3$ are as defined in formula (I); and $D^3$ represents an oxygen atom or a sulfur atom (provided that when B represents an oxygen atom or NH, $D^3$ represents a sulfur atom).

(iv) Isomerization:

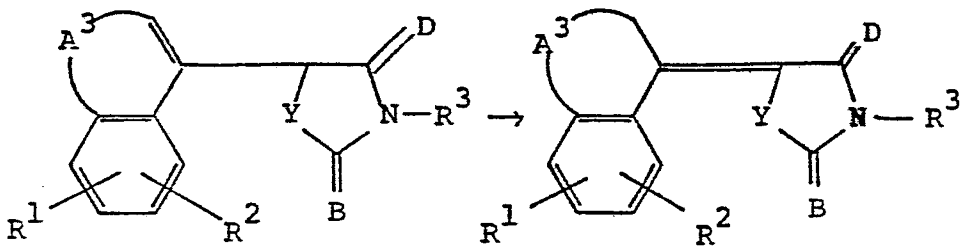

(VII) (I-3)

In the above formulae (I-3) and (VII), $A^3$ represents an alkylene or alkenylene chain having 1 to 3 carbon atoms which may have a hetero atom and/or which may have substituent(s); and B, D, Y, $R^1$, $R^2$ and $R^3$ are as defined in formula (I)

The reaction can be carried out by irradiating compound of formula (VII),

or heating compound (VII), or treating the same with a base such as ammonia, triethylamine, sodium hydroxide or ammonium hydroxide.

Isomerization of final product (I-3) of the present invention to compound (VII) can be carried out under the same conditions.

(V) Halogenation:

This is to introduce a halogen atom as a substituent on the alkylene chain or alkenylene chain of A. The halogenation is generally carried out by reacting the compound with a halogen such as chlorine or bromine in a solvent such as acetic acid or dioxane at room temperature or with heating (under reflux) at about the boiling point of the solvent.

(vi) Dehydrohalogenation:

This is to obtain a compound wherein A is an alkenylene chain, by dehydrohalogenating a compound wherein A is an alkylene chain substituted by halogen. The dehydrohalogenation can be carried out by suspending or dissolving the starting compound in a solvent such as acetic acid, dimethylformamide or dimethylsulfoxide and then reacting the said compound with a tertiary amine such as pyridine or triethylamine or with a weak base such as sodium carbonate or sodium acetate at room temperature or raised temperature.

The compounds of formula (I) of the present invention can be isolated and purified by extraction with an organic solvent such as ethyl acetate, recrystallization, or silica gel-chromatography. When the compounds have a free acid residue such as a carboxyl or sulfo group, or a free base residue such as an amino or imidazolyl group, they can be treated respectively with base or acid to form the corresponding salts. When the free carboxylic acids are to be esterified, they may be reacted with alcohol such as

ethanol or benzyl alcohol in conventional manner.

Particularly preferred compounds of the present invention are the following and their tautomers, and pharmaceutically acceptable salts and esters thereof;

4-(4-oxo-2-thioxo-5-thiazolidinylidene)thiochroman 1,1-dioxide

4-(2,4-dioxo-5-thiazolidinylidene)thiochroman 1,1-dioxide

(Z)-4-(2,4-dioxo-5-thiazolidinylidene)thiochroman 1,1-dioxide

(E)-4-(2,4-dioxo-5-thiazolidinylidene)thiochroman 1,1-dioxide

7-chloro-4-(4-oxo-2-thioxo-5-thiazolidinylidene)thiochroman 1,1-dioxide

4-(4-oxo-2-thioxo-5-thiazolidinylidene)thiochromene 1,1-dioxide

4-(2,4-dioxo-5-thiazolidinylidene)thiochromene 1,1-dioxide

7-chloro-4-(2,4-dioxo-5-thiazolidinylidene)thiochromene 1,1-dioxide

7-chloro-4-(4-oxo-2-thioxo-5-thiazolidinylidene)thiochroman 1,1-dioxide

7-chloro-4-(2,4-dioxo-5-thiazolidinylidene)thiochroman 1,1-dioxide

8-chloro-4-(2,4-dioxo-5-thiazolidinylidene)thiochroman 1,1-dioxide

6-chloro-4-(2,4-dioxo-5-thiazolidinylidene)thiochroman 1,1-dioxide

7-chloro-4-(2,4-dioxo-5-thiazolidinylidene)thiochroman

6,7-dichloro-4-(2,4-dioxo-5-thiazolidinylidene)thiochroman

7-bromo-4-(2,4-dioxo-5-thiazolidinylidene)thiochroman

7-chloro-4-(2-thio-4-oxo-5-thiazolidinylidene)thio-chroman

The compounds of the present invention have aldose reductase-inhibitory activity. Some have in addition platelet aggregation-inhibitory activity or lipid-lowering activity. Aldose reductase is an enzyme catalyzing the conversion of glucose into sorbitol in the living body. Accordingly, inhibition of the activity of the said enzyme inhibits abnormal increment of the sorbitol content in cells in diabetic patients. Therefore, the compounds of the present invention can be used as a preventative or remedy for chronic diabetic complications such as cataract, neuropathy, retinopathy or cerebrovascular insufficiency. The aldose reductase-inhibitory activity and the platelet aggregation-inhibitory activity were measured by the following test methods:

(1) Aldose reductase-inhibitory activity:

This was measured by the method developed by S. Hayman and J.H. Kinoshita (The Journal of Biological Chemistry, Vol. 240, 877 (1965)) as modified by S.D. Varma and J.H. Kinoshita (Biochemical Pharmacology, Vol. 25, 2505 (1976)).

The compound of the present invention, aldose reductase and nicotinamide adenine dinucleotide phosphate (NADPH) are dissolved in a buffer of pH 6.2 and incubated for 10 minutes at 25°C; glyceraldehyde is added thereto to start the reaction, and the rate of disappearance of NADPH is defined to be the enzyme activity of the aldose reductase.

For the preparation of the aldose reductase, a method by B. Wermuth, J.P. von Warburg, et al. (Method in Enzymology, Vol. 89, 181 (1982)) was partially modified and a human placenta aldose reductase was purified to a single protein.

(2) Platelet aggregation-inhibitory activity:

A male rabbit (Japanese white rabbit) having a weight of about 3.0 kg was anesthetized with pentobarbital, and blood was taken from the thoracic vena cava into 3.8% sodium citrate (10% of the blood). Platelet-rich (PRP) and platelet-poor (PPP) plasma were prepared by centrifuging for 10 minutes at 200 x g and for 20 minutes at 1200 x g, respectively. Platelet aggregation in PRP was monitored photometrically by Born's method in a Payton

aggregometer using PPP as a blank. Aggregation was induced by arachidonate and recorded for 5 min. Test compound was incubated with PRP for 2 minutes prior to the addition of arachidonate. The extent of platelet aggregation was expressed as a percent decrease in the optical density of PRP.

The results are given in the following table, where the enzyme activity of a variety of the compounds of the present invention is shown in various concentration levels in terms of the inhibition percentage (%).

Table

| Example No. | Aldose Reductase Inhibition | | Platelet Aggregation Inhibition | |
|---|---|---|---|---|
| | $10^{-7}$(M) (%) | $10^{-6}$(M) (%) | 200 (μM) (%) | 400 (μM) (%) |
| 7 | 65 | 93 | 100 | - |
| 11 | 40 | 89 | 17 | 56 |
| 49 | 23 | 65 | 57 | 69 |
| 54 | 15 | 30 | 65 | - |
| 66 | - | 72 | 17 | 96 |
| 67 | - | 64 | 9 | 95 |
| 95 | 19 | 65 | - | 43 |
| 99B | 42 | 87 | - | - |
| 105 | 29 | 83 | - | - |
| 119 | 37 | 53 | - | 84 |
| 146 | - | 56 | - | - |
| Sorbinil* | 15 | 53.6 | | |

Note: -: not tested.
*: known compound described in U.S. Patent 4,130,714.

Medicines which contain one or more of compounds of the invention as active ingredient can be prepared using pharmaceutically acceptable carriers, vehicles and additives, to form tablets, powders, fine granules, granules, capsules, pills, solutions, injections, eye drops, etc., and these may be administered orally

or parenterally.

Solid or liquid non-toxic substances suitable as pharmaceutically acceptable carriers and vehicles include lactose, magnesium stearate, starch, talc, gelatin, agar, pectine, gum arabi, olive oil, sesame oil, cacao butter, ethylene glycol and other conventional materials such as physiological salt solution.

The daily dosage of compound of the present invention in clinical use is appropriately determined according to the symptom, weight, age and sex of the patient to be treated; in general, the total dosage is 10 to 600 mg/day for an adult human, administered in a single dose or in separate sub-doses.

Compounds of the present invention and the manufacture thereof are explained in greater detail by reference to the following examples, which, however, are not intended to be interpreted as limiting the scope of the present invention. In addition, the manufacture of starting compounds as used in the examples is described in reference examples . The following abbreviations are used:

EtOAc: Ethyl acetate

AcOH: Acetic acid

$CH_2Cl_2$: Dichloromethane

| | |
|---|---|
| MeOH: | Methanol |
| DMSO: | Dimethylsulfoxide |
| $CHCl_3$ | Chloroform |
| EtOH: | Ethanol |
| <: | more than ... |
| dec.: | Decomposition |

REFERENCE EXAMPLE 1 (Raw material in Example 19):

Dimethylformamide solution containing 2.37 g of 4-(1-imidazolyl)butyric acid and 2.08 g of 1-hydroxybenzotriazole was warmed at 35°C, and 3.18 g of dicyclohexylcarbodiimide was added to the resulting solution, while stirred. After the whole was stirred for 20 minutes, 5 ml of dimethylformamide solution containing 2.3 g of 6-aminothiochroman-4-one was added dropwise thereto. After the addition, the whole was stirred for 2.5 hours at 40 to 45°C. The urea precipitated was filtered out, and the remaining filtrate was concentrated under reduced pressure, water was added thereto and the resulting solution was extracted with chloroform. The chloroform layer was washed with a saturated sodium hydrogencarbonate aqueous solution and then with a saturated salt solution, and thereafter dried with anhydrous magnesium sulfate. After the chloroform was distilled out under reduced pressure, the crystals obtained were washed with ether, to obtain 6-[4-(1-imidazolyl)butyramido]thiochroman-4-one.

Physico-chemical properties:

(i) m.p.: 160 to 163°C

(ii) Nuclear magnetic reasonance-spectrum (DMSO-$d_6$)

δ: 4.00 (2H, t, J = 7.2 Hz, N-$CH_2$-)

6.90 (1H, s, hydrogen in imidazole group)

7.18 (1H, s, hydrogen in imidazole group)

(iii) Mass spectrometric value (m/z) 315 ($M^+$)

<u>REFERENCE EXAMPLE 2 (Raw material in Example 32):</u>

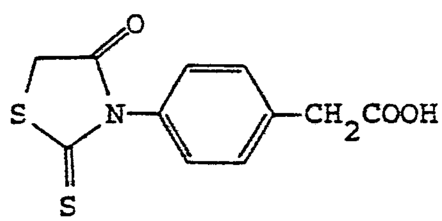

100 ml of water was added to 15.1 g of p-aminophenyl-acetic acid and suspended, and the resulting suspension was completely neutralized with sodium carbonate. To this was added 22.6 g of di(carboxymethyl)trithiocarbonate; after 16 hours heating under reflux, then cooling, and then acidifying with dilute sulfuric acid, the crystals precipitated were filtered and washed with water to obtain p-(4-oxo-2-thioxo-3-thiazolidinyl)phenyl-acetic acid.

Physico-chemical properties:

(i) m.p.: 183 to 187°C (decomposition)

(ii) Nuclear magnetic reasonance-spectrum (DMSO-$d_6$)

δ: 3.64 (2H, s, $-CH_2COOH$)

4.38 (2H, s, 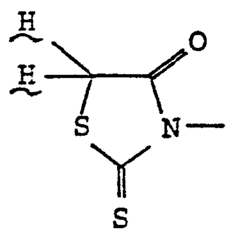 )

7.18 (2H, d, J=9 Hz, hydrogen in benzene ring)

7.42 (2H, d, J=9 Hz, hydrogen in benzene ring)

(iii) Mass spectrometric value (m/z) 267 ($M^+$)

EXAMPLE 1

50 ml of acetic acid and 0.5 g of DBU (1.8-diazabicyclo[5,4,0]undeca-7-ene) were put in an egg-plant type flask having a capacity of 200 ml, which was provided with a Dean-Stark dehydration device and which was filled with zeolite. After the contents were neutralized, 1.91 g of rhodanine-3-acetic acid and then 1.7 g of 5,7-dimethyl-1-tetralone were added. The whole was heated and stirred in a hot bath at 180 to 200°C for 8 hours; then the acetic acid was distilled out under reduced pressure, and the residue was dissolved in 200 ml of ethyl acetate and twice washed with 200 ml of 5% sodium hydrogencarbonate aqueous solution. The ethyl acetate was

distilled out under reduced pressure, and the residue was subjected to silica gel chromatography (silica gel: 100 g); the unreacted 5,7-dimethyl-1-tetralone was first eluted out with an eluent of chloroform and then the product was eluted out with an eluent of chloroform containing a small amount of methanol, to obtain 1.2 g (34.6%) of 5-(5,7-dimethyl-1,2,3,4-tetrahydro-1-naphthylidene)-4-oxo-2-thioxo-3-thiazolidine-acetic acid.

The physico-chemical properties of the product are shown in the following Table.

In the same manner as the Example 1, the following compounds of Examples 2 to 19 were obtained.

| Example No. | I |
|---|---|
| Chemical name | 5-(5,7-dimethyl-1,2,3,4-tetrahydro-1-naphthylidene)-4-oxo-2-thioxo-3-thiazolidine-acetic acid |
| Structural formula | 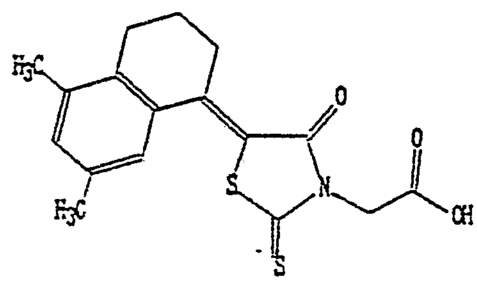 |
| m.p. (°C) | 201-204 |
| Solvent for recrystallization | Ether |
| Molecular formula | C17 H17 N O3 S2 |

| Elementary analysis | C | H | N | S |
|---|---|---|---|---|
| Calculated value (%) | 58.77 | 4.93 | 4.03 | 18.45 |
| Measured value (%) | 58.60 | 4.77 | 4.05 | 18.68 |

| Mass spectrometric value | (m/z) 347($M^+$),202 |
|---|---|

| Example No. | 2 | | | |
|---|---|---|---|---|
| Chemical name | 4-oxo-5-(4-thiochromanylidene)-2-thioxo-3-thiazolidine-acetic acid | | | |
| Structural formula | 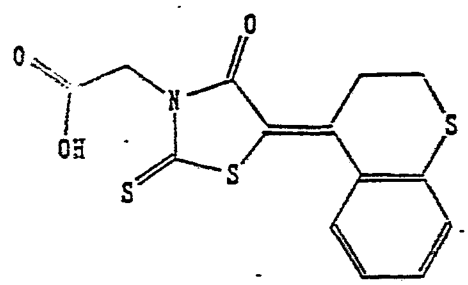 | | | |
| m.p. (°C) | 204-206 | | | |
| Solvent for recrystallization | EtOAc | | | |
| Molecular formula | C14 H11 N O3 S3 | | | |
| Elementary analysis | C | H | N | S |
| Calculated value (%) | 49.83 | 3.29 | 4.15 | 28.50 |
| Measured value (%) | 49.97 | 3.16 | 4.17 | 28.50 |
| Mass spectrometric value | (m/z) 337(M+),187 | | | |

| Example No. | 3 |
| --- | --- |
| Chemical name | 5-(4-chromanylidene)-4-oxo-2-thioxo-3-thiazolidine-acetic acid |
| Structural formula | 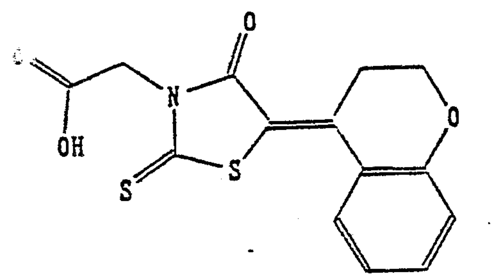 |
| m.p. (°C) | 226-229 |
| Solvent for recrystallization | EtOAc |
| Molecular formula | C14 H11 N O4 S2 |
| Elementary analysis | C H N S |
| Calculated value (%) | 52.32 3.45 4.36 19.95 |
| Measured value (%) | 52.32 3.30 4.35 19.83 |
| Mass spectrometric value | (m/z) 321($M^+$),176 |

| Example No. | 4 | | | |
|---|---|---|---|---|
| Chemical name | 4-oxo-5-(1,2,3,4-tetrahydro-1-naphthylidene)-2-thioxo-3-thiazolidineacetic acid | | | |
| Structural formula | 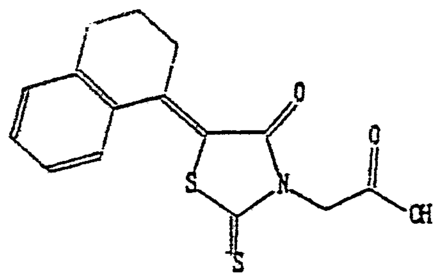 | | | |
| m.p. (°C) | 236-240 | | | |
| Solvent for recrystallization | EtOAc | | | |
| Molecular formula | C15 H13 N 03 S2 | | | |
| Elementary analysis | C | H | N | S |
| Calculated value (%) | 56.41 | 4.10 | 4.39 | 20.08 |
| Measured value (%) | 56.60 | 4.00 | 4.29 | 20.26 |
| Mass spectrometric value | (m/z) 319(M⁺),174 | | | |

| Example No. | 5 | | | | |
|---|---|---|---|---|---|
| Chemical name | 5-(6-chloro-4-chromanylidene)-4-oxo-2-thioxo-3-thiazolidine-acetic acid | | | | |
| Structural formula | 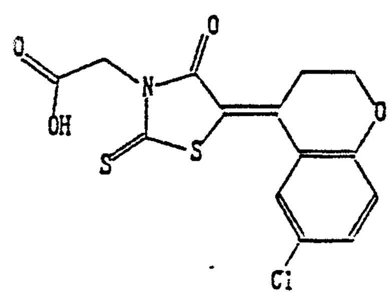 | | | | |
| m.p. (°C) | 238-240 | | | | |
| Solvent for recrystallization | AcOH | | | | |
| Molecular formula | C14 H10 Cl N O4 S2 | | | | |
| Elementary analysis | C | H | N | S | Cl |
| Calculated value (%) | 47.26 | 2.83 | 3.94 | 18.02 | 9.96 |
| Measured value (%) | 47.32 | 2.75 | 3.81 | 18.28 | 9.93 |
| Mass spectrometric value | (m/z) 356(M+1) | | | | |

| Example No. | 6 | | | |
|---|---|---|---|---|
| Chemical name | 4-oxo-5-(2-phenyl-4-chromanylidene)-2-thioxo-3-thiazolidineacetic acid | | | |
| Structural formula | 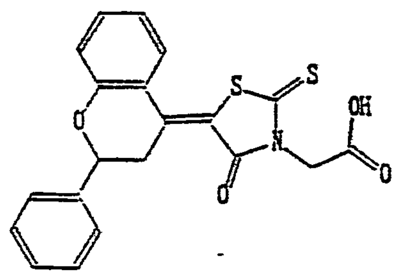 | | | |
| m.p. (°C) | 203-204 | | | |
| Solvent for recrystallization | $CH_2Cl_2$ | | | |
| Molecular formula | C20 H15 N 04 S2 | | | |
| Elementary analysis | C | H | N | S |
| Calculated value (%) | 60.44 | 3.80 | 3.52 | 16.10 |
| Measured value (%) | 60.25 | 3.58 | 3.53 | 16.13 |
| Mass spectrometric value | (m/z) 397(M+),279,203 | | | |

| Example No. | 7 |
|---|---|
| Chemical name | 5-(6-methoxy-1,2,3,4-tetrahydro-1-natphylidene)-4-oxo-2-thioxo-3-thiazolidine-acetic acid |
| Structural formula | 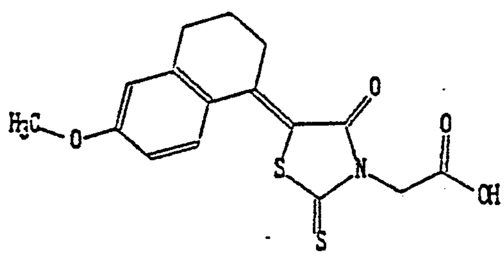 |
| m.p. (°C) | 175-190 |
| Solvent for recrystallization | MeOH |
| Molecular formula | C16 H15 N 04 S2 |
| Elementary analysis | C H N S |
| Calculated value (%) | 55.00 4.33 4.01 18.35 |
| Measured value (%) | 55.05 4.20 3.96 18.64 |
| Mass spectrometric value | (m/z) 349(M⁺),204 |

| Example No. | 8 |
|---|---|
| Chemical name | 5-(1-indanylidene)-4-oxo-2-thioxo-thiazolidine |
| Structural formula | 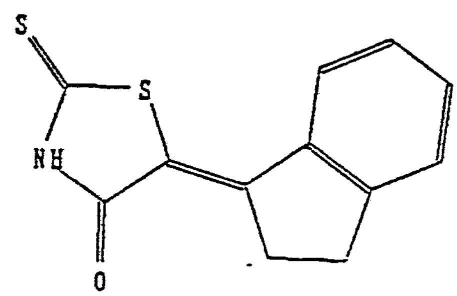 |
| m.p. (°C) | 275 |
| Solvent for recrystallization | DMSO |
| Molecular formula | C12 H9 N O S2 |
| Elementary analysis | C H N S |
| Calculated value (%) | 58.28 3.67 5.66 25.92 |
| Measured value (%) | 58.27 3.40 5.63 26.08 |
| Mass spectrometric value | (m/z) 247(M⁺),160 |

| Example No. | 9 |
| --- | --- |
| Chemical name | 4-oxo-5-(2-phenyl-4H-chromen-4-ylidene)-2-thioxo-3-thiazolidine |
| Structural formula | 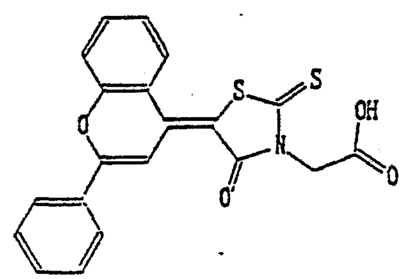 |
| m.p. (°C) | 200< |
| Solvent for recrystallization | EtOAc |
| Molecular formula | C20 H13 N 04 S2 |
| Elementary analysis | C H N |
| Calculated value (%) | 60.74 3.31 3.54 |
| Measured value (%) | 60.48 3.09 3.50 |
| Mass spectrometric value | (m/z) 395($M^+$),250 |

| | | | | |
|---|---|---|---|---|
| Example No. | 10 | | | |
| Chemical name | 5-(7-methoxy-1,2,3,4-tetrahydro-1-naphthylidene)-4-oxo-2-thioxo-3-thiazolidine-acetic acid | | | |
| Structural formula | 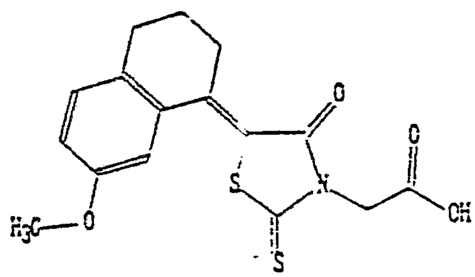 | | | |
| m.p. (°C) | 173-175 | | | |
| Solvent for recrystallization | Benzene | | | |
| Molecular formula | C16 H15 N O4 S2 | | | |
| Elementary analysis | C | H | N | S |
| Calculated value (%) | 55.00 | 4.33 | 4.01 | 18.35 |
| Measured value (%) | 54.91 | 4.17 | 3.99 | 18.19 |
| Mass spectrometric value | (m/z) 349(M⁺),204 | | | |

| Example No. | 11 | | | |
|---|---|---|---|---|
| Chemical name | 5-(5-methoxy-1,2,3,4-tetrahydro-1-naphthylidene)-4-oxo-2-thioxo-3-thiazolidine-acetic acid | | | |
| Structural formula | 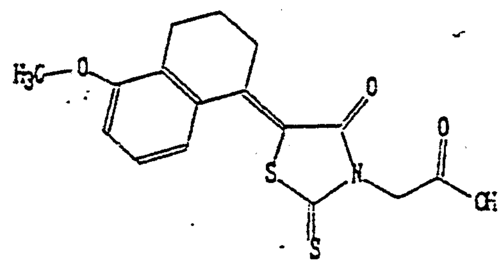 | | | |
| m.p. (°C) | 185-187 | | | |
| Solvent for recrystallization | Ether | | | |
| Molecular formula | C16 H15 N 04 S2 | | | |
| Elementary analysis | C | H | N | S |
| Calculated value (%) | 55.00 | 4.33 | 4.01 | 18.35 |
| Measured value (%) | 55.43 | 4.18 | 3.86 | 18.24 |
| Mass spectrometric value | (m/z) 349($M^+$),204 | | | |

| Example No. | 12 | | | |
|---|---|---|---|---|
| Chemical name | 5-(5-hydroxy-1-indanylidene)-4-oxo-2-thioxo-3-thiazolidine-acetic acid | | | |
| Structural formula | 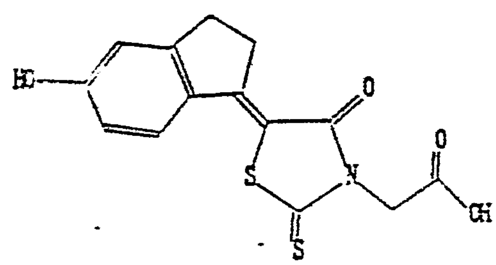 | | | |
| m.p. (°C) | 250< | | | |
| Solvent for recrystallization | AcOH | | | |
| Molecular formula | C14 H11 N 04 S2 | | | |
| Elementary analysis | C | H | N | S |
| Calculated value (%) | 52.32 | 3.45 | 4.36 | 19.96 |
| Measured value (%) | 52.01 | 3.29 | 4.26 | 19.77 |
| Mass spectrometric value | (m/z) 321(M⁺),176 | | | |

| Example No. | 13 |
| --- | --- |
| Chemical name | 5-(4-hydroxy-1-indanylidene)-4-oxo-2-thioxo-3-thiazolidine-acetic acid |
| Structural formula | 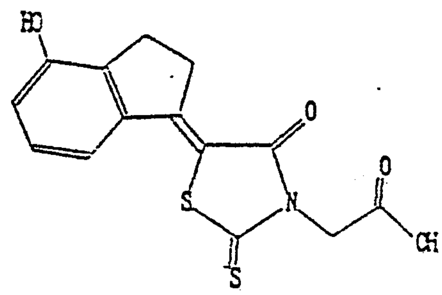 |
| m.p. (°C) | 250< |
| Solvent for recrystallization | AcOH |
| Molecular formula | C14 H11 N 04 S2 |
| Elementary analysis | C H N S |
| Calculated value (%) | 52.32 3.45 4.36 19.96 |
| Measured value (%) | 52.14 3.25 4.28 19.95 |
| Mass spectrometric value | (m/z) 321($M^+$),176 |

| Example No. | 14 |
|---|---|
| Chemical name | 5-(5,6-dimethoxy-1-indanylidene)-4-oxo-2-thioxo-3-thiazolidine-acetic acid |
| Structural formula | 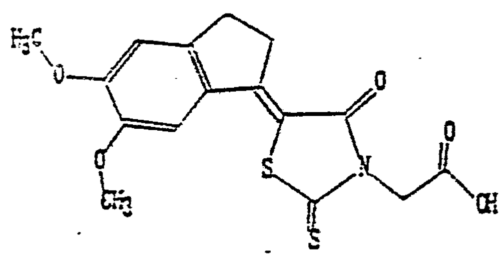 |
| m.p. (°C) | 307 |
| Solvent for recrystallization | AcOH |
| Molecular formula | C16 H15 N 05 S2 |

| Elementary analysis | C | H | N | S |
|---|---|---|---|---|
| Calculated value (%) | 52.59 | 4.14 | 3.83 | 17.55 |
| Measured value (%) | 52.46 | 4.00 | 3.76 | 17.66 |

| Mass spectrometric value | (m/z) 365(M⁺),220 |
|---|---|

| Example No. | 15 |
|---|---|
| Chemical name | 5-(5-methyl-1-indanylidene)-4-oxo-2-thioxo-3-thiazolidine-acetic acid |
| Structural formula | 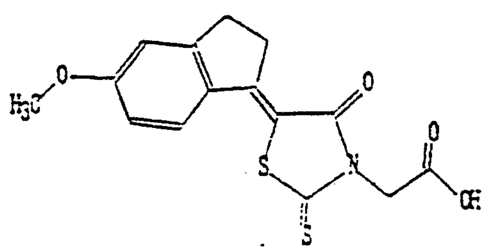 |
| m.p. (°C) | 265-268 |
| Solvent for recrystallization | AcOH |
| Molecular formula | C15 H13 N O4 S2 |
| Elementary analysis | C H N S |
| Calculated value (%) | 53.72 3.91 4.18 19.12 |
| Measured value (%) | 53.50 3.70 4.22 19.17 |
| Mass spectrometric value | (m/z) 335(M⁺),190 |

| Example No. | 16 | | | |
|---|---|---|---|---|
| Chemical name | 4-oxo-5-(3-oxo-2-phenyl-1-indan-ylidene)-2-thioxo-3-thiazolidine-acetic acid | | | |
| Structural formula | 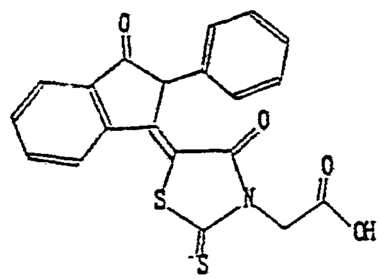 | | | |
| m.p. (°C) | 254-256 | | | |
| Solvent for recrystallization | MeOH | | | |
| Molecular formula | C20 H13 N O4 S2 | | | |
| Elementary analysis | C | H | N | S |
| Calculated value (%) | 60.74 | 3.31 | 3.54 | 16.22 |
| Measured value (%) | 60.64 | 3.28 | 3.53 | 16.23 |
| Mass spectrometric value | (m/z) 395(M⁺),250 | | | |

| | |
|---|---|
| Example No. | 17 |
| Chemical name | p-[4-oxo-5-(4-thiochromanylidene)-2-thioxo-3-thiazolidinyl]benzoic acid |
| Structural formula | 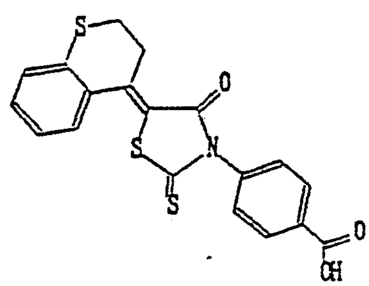 |
| m.p. (°C) | 286-289(dec.) |
| Solvent for recrystallization | Washing with $CHCl_3$ |
| Molecular formula | C19 H13 N O3 S3 |
| Elementary analysis | C H N S |
| Calculated value (%) | 57.12 3.28 3.51 24.08 |
| Measured value (%) | 56.96 3.02 3.48 23.80 |
| Mass spectrometric value | (m/z) 399($M^+$),192 |

| Example No. | 18 |
| --- | --- |
| Chemical name | 1-(2,4-dioxo-5-thiazolidin-ylidene)-3-(4-oxo-2-thioxo-5-thiazolidinylidene)-2phenylindane |
| Structural formula | 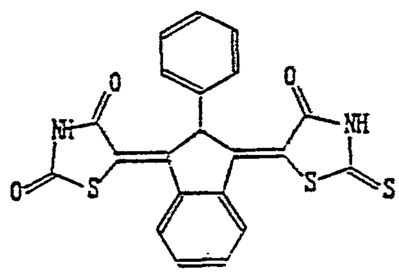 |
| m.p. (°C) | 250< |
| Solvent for recrystallization | Isopropanol |
| Molecular formula | C21 H12 N2 O3 S3 |
| Elementary analysis<br>Calculated value (%)<br>Measured value (%) | C H N<br>57.78 2.77 6.42<br>57.66 2.71 6.00 |
| Mass spectrometric value | (m/z) 436($M^+$) |

| Example No. | 19 |
|---|---|
| Chemical name | 5-[6-[4-(1-imidazolyl)butyramido]-4-thiochromanylidene]-4-oxo-2-thioxo-thiazolidine |
| Structural formula | 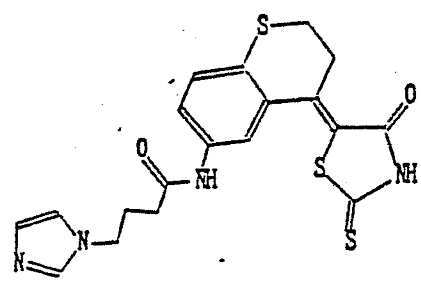 |
| m.p. (°C) | 235-240(dec.) |
| Solvent for recrystallization | Washing with $CHCl_3$ |
| Molecular formula | C19 H18 N4 O2 S3 |
| Elementary analysis | C H N S |
| Calculated value (%) | 53.00 4.21 13.01 22.34 |
| Measured value (%) | 52.63 4.13 12.99 21.65 |
| Mass spectrometric value | (m/z) 430($M^+$) |

EXAMPLE 20

1.33 g of rhodanine, 1.64 g of thiochroman-4-one, 0.8 g of ammonium acetate and 50 ml of acetic acid were put into an egg-plant type flask having a capacity of 200 ml which was provided with a Dean-Stark dehydration device and which was filled with zeolite, and then the whole was heated and stirred in an oil bath at 180 to 200°C for 8 hours. After cooling, excess saturated aqueous sodium hydrogencarbonate was added to make the resulting solution alkaline, and then the solution was extracted with 300 ml of ethyl acetate. Next, this was twice washed with 100 ml of saturated aqueous sodium hydrogencarbonate, and then ethyl acetate was distilled out under reduced pressure. The resulting residue was subjected to silica gel chromatography (silica gel:100 g); unreacted thiochroman-4-one was first eluted with an eluent of chloroform and then the desired product was eluted with an eluent of chloroform containing a small amount of methanol to obtain 0.8 g (28.6%) of 4-oxo-5-(4-thiochromanylidene)-2-thioxo-thiazolidine.

Physico-chemical properties of the product are shown in the following Table.

In the same manner as Example 20, the following compounds of Examples 21 to 54 were obtained.

| Example No. | 20 | | | |
|---|---|---|---|---|
| Chemical name | 4-oxo-5-(4-thiochromanylidene)-2-thioxo-thiazolidine | | | |
| Structural formula | 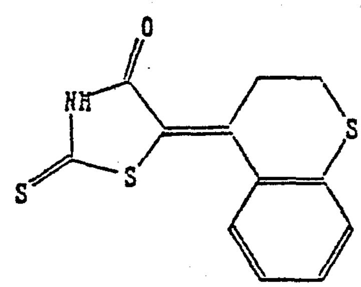 | | | |
| m.p. (°C) | 199-200 | | | |
| Solvent for recrystallization | $CHCl_3$ | | | |
| Molecular formula | $C_{12}H_9NOS_3$ | | | |
| Elementary analysis | C | H | N | S |
| Calculated value (%) | 51.59 | 3.25 | 5.01 | 34.43 |
| Measured value (%) | 51.62 | 3.18 | 4.99 | 34.38 |
| Mass spectrometric value | (m/z) 279(M⁺),187 | | | |

| Example No. | 21 | | | |
|---|---|---|---|---|
| Chemical name | 5-(4-hydroxy-1-indanylidene)-4-oxo-2-thioxo-thiazolidine | | | |
| Structural formula | 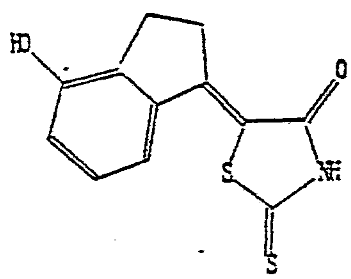 | | | |
| m.p. (°C) | 277-280(dec.) | | | |
| Solvent for recrystallization | MeOH | | | |
| Molecular formula | C12 H9 N O2 S2 | | | |
| Elementary analysis | C | H | N | S |
| Calculated value (%) | 54.73 | 3.44 | 5.32 | 24.35 |
| Measured value (%) | 54.63 | 3.30 | 5.35 | 23.94 |
| Mass spectrometric value | (m/z) 263($M^+$),176 | | | |

| Example No. | 22 | | | | |
|---|---|---|---|---|---|
| Chemical name | 5-(6-chloro-4-chromanylidene)-4-oxo-2-thioxo-thiazolidine | | | | |
| Structural formula | 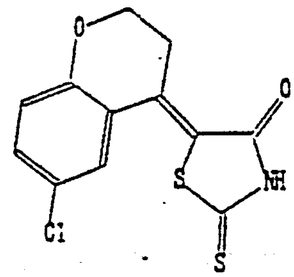 | | | | |
| m.p. (°C) | 210-213(dec.) | | | | |
| Solvent for recrystallization | EtOAc-n-hexane | | | | |
| Molecular formula | C12 H8 Cl N O2 S2 | | | | |
| Elementary analysis | C | H | N | S | Cl |
| Calculated value (%) | 48.08 | 3.36 | 4.67 | 21.39 | 11.83 |
| Measured value (%) | 48.02 | 3.22 | 4.57 | 21.28 | 12.38 |
| Mass spectrometric value | (m/z) 300(M⁺),210 | | | | |

| Example No. | 23 |
|---|---|
| Chemical name | 5-(7-methoxy-1,2,3,4-tetrahydro-1-naphtylidene)-4-oxo-2-thioxo-thiazolidine |
| Structural formula | 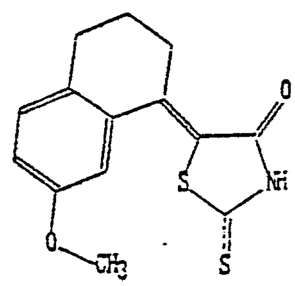 |
| m.p. (°C) | 238-241 (dec.) |
| Solvent for recrystallization | AcOH |
| Molecular formula | C14 H13 N O2 S2 |
| Elementary analysis | C H N S |
| Calculated value (%) | 57.71 4.50 4.81 22.01 |
| Measured value (%) | 57.73 4.43 4.78 21.93 |
| Mass spectrometric value | (m/z) 291 ($M^+$), 204 |

| Example No. | 24 | | | | |
|---|---|---|---|---|---|
| Chemical name | 5-(6-fluoro-4-chromanylidene)-4-oxo-2-thioxo-thiazolidine | | | | |
| Structural formula | 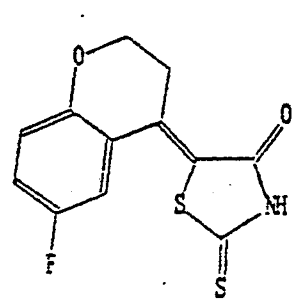 | | | | |
| m.p. (°C) | 176-178 | | | | |
| Solvent for recrystallization | EtOH | | | | |
| Molecular formula | C12 H8 F N O2 S2 | | | | |
| Elementary analysis | C | H | N | S | F |
| Calculated value (%) | 51.23 | 2.87 | 4.98 | 22.80 | 6.75 |
| Measured value (%) | 51.12 | 2.74 | 4.93 | 22.94 | 6.70 |
| Mass spectrometric value | (m/z) 281(M$^+$),194 | | | | |

| Example No. | 25 | | | |
|---|---|---|---|---|
| Chemical name | 3-ethyl-4-oxo-5-(4-thiochroman-ylidene)-2-thioxo-thiazolidine | | | |
| Structural formula | 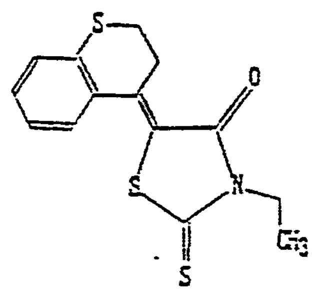 | | | |
| m.p. (°C) | 112-113 | | | |
| Solvent for recrystallization | EtOAc-n-hexane | | | |
| Molecular formula | C14 H13 N O S3 | | | |
| Elementary analysis | C | H | N | S |
| Calculated value (%) | 54.69 | 4.26 | 4.56 | 31.29 |
| Measured value (%) | 54.81 | 4.11 | 4.56 | 30.74 |
| Mass spectrometric value | (m/z) 307(M⁺),192 | | | |

| Example No. | 26 | | | |
|---|---|---|---|---|
| Chemical name | 4-oxo-5-(3-oxo-2-phenyl-1-indan-ylidene)-2-thioxo-thiazolidine | | | |
| Structural formula | 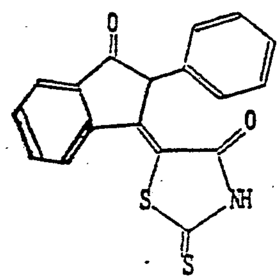 | | | |
| m.p. (°C) | 243-245 | | | |
| Solvent for recrystallization | Benzene | | | |
| Molecular formula | C18 H11 N O2 S2 | | | |
| Elementary analysis | C | H | N | S |
| Calculated value (%) | 64.07 | 3.29 | 4.15 | 19.01 |
| Measured value (%) | 64.13 | 3.16 | 4.09 | 18.79 |
| Mass spectrometric value | (m/z) 337(M+),250 | | | |

| Example No. | 27 | | | | |
|---|---|---|---|---|---|
| Chemical name | 5-(6-chloro-4-thiochromanylidene)-4-oxo-2-thioxo-thiazolidine | | | | |
| Structural formula | 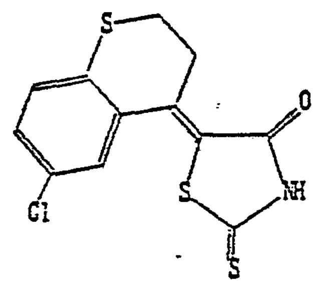 | | | | |
| m.p. (°C) | 144-146 | | | | |
| Solvent for recrystallization | Washing with $CHCl_3$ | | | | |
| Molecular formula | C12 H8 Cl N O S3 | | | | |
| Elementary analysis | C | H | N | S | Cl |
| Calculated value (%) | 45.92 | 2.57 | 4.46 | 30.65 | 11.30 |
| Measured value (%) | 45.74 | 2.41 | 4.45 | 30.40 | 11.49 |
| Mass spectrometric value | (m/z) 313(M⁺),226 | | | | |

| Example No. | 28 |
|---|---|
| Chemical name | 5-(4-chromanylidene)-4-oxo-2-thioxo-thiazolidine |
| Structural formula | 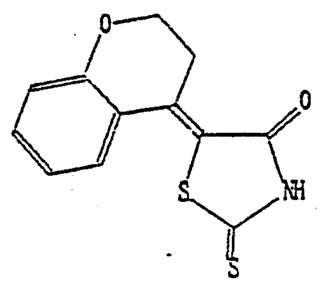 |
| m.p. (°C) | 175-177 |
| Solvent for recrystallization | Benzene |
| Molecular formula | C12 H9 N 02 S2 |
| Elementary analysis | C H N S |
| Calculated value (%) | 54.73 3.44 5.32 24.35 |
| Measured value (%) | 54.58 3.20 5.30 24.30 |
| Mass spectrometric value | (m/z) 263($M^+$),176 |

| Example No. | 29 | | | |
|---|---|---|---|---|
| Chemical name | 5-(6-nitro-4-thiochromanylidene)-4-oxo-2-thioxo-thiazolidine | | | |
| Structural formula | 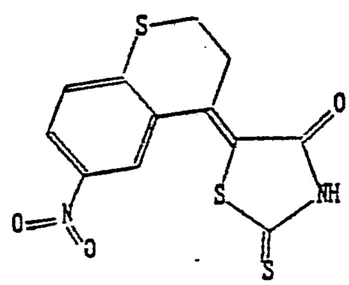 | | | |
| m.p. (°C) | 195-200(dec.) | | | |
| Solvent for recrystallization | Washing with $CHCl_3$ | | | |
| Molecular formula | C12 H8 N2 O3 S3 | | | |
| Elementary analysis | C | H | N | S |
| Calculated value (%) | 44.43 | 2.49 | 8.64 | 29.65 |
| Measured value (%) | 43.71 | 2.52 | 8.40 | 28.71 |
| Mass spectrometric value | (m/z) 324($M^+$) | | | |

| Example No. | 30 | | | |
|---|---|---|---|---|
| Chemical name | 4-oxo-5-(1,2,3,4-tetrahydro-1-naphthylidene)-2-thioxo-thiazolidine | | | |
| Structural formula | 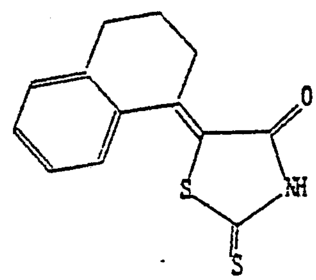 | | | |
| m.p. (°C) | 186-187 | | | |
| Solvent for recrystallization | iso-propanol | | | |
| Molecular formula | C13 H11 N O S2 | | | |
| Elementary analysis | C | H | N | S |
| Calculated value (%) | 59.74 | 4.24 | 5.36 | 24.54 |
| Measured value (%) | 59.92 | 4.33 | 5.28 | 24.66 |
| Mass spectrometric value | (m/z) 261($M^+$),174 | | | |

| Example No. | 31 |
|---|---|
| Chemical name | 5-(6-methoxy-4-thiochromanylidene)-4-oxo-2-thioxo-thiazolidine |
| Structural formula | 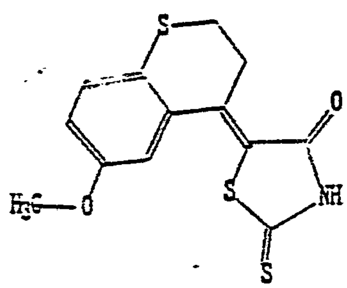 |
| m.p. (°C) | 175-177 |
| Solvent for recrystallization | Washing with $CHCl_3$ |
| Molecular formula | C13 H11 N 02 S3 |
| Elementary analysis | C H N S |
| Calculated value (%) | 50.41 3.58 4.53 31.09 |
| Measured value (%) | 50.41 3.53 4.39 31.12 |
| Mass spectrometric value | (m/z) 309 ($M^+$) |

| Example No. | 82 |
|---|---|
| Chemical name | p-[4-oxo-5-(4-thiochromanylidene)-2-thioxo-3-thiazolidinyl]phenyl-acetic acid |
| Structural formula | 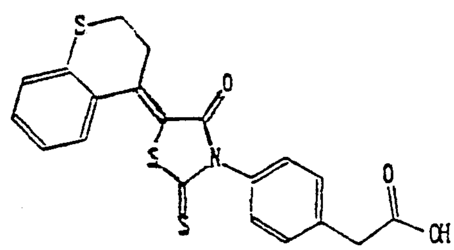 |
| m.p. (°C) | 207-210 |
| Solvent for recrystallization | Washing with isopropanol |
| Molecular formula | $C_{20}H_{15}NO_3S_3$ |
| Elementary analysis | C H N S |
| Calculated value (%) | 58.21 3.53 3.77 17.27 |
| Measured value (%) | 57.58 3.45 3.63 17.03 |
| Mass spectrometric value | (m/z) 371($M^+$),150 |

| Example No. | 33 | | | |
|---|---|---|---|---|
| Chemical name | 2,4-dioxo-5-(4-thiochromanylidene)-thiazolidine | | | |
| Structural formula | 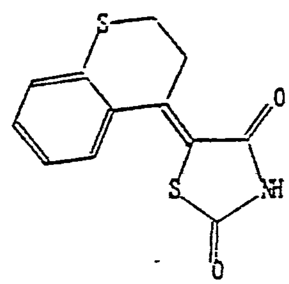 | | | |
| m.p. (°C) | 145-146 | | | |
| Solvent for recrystallization | Benzene | | | |
| Molecular formula | C12 H9 N O2 S2 | | | |
| Elementary analysis | C | H | N | S |
| Calculated value (%) | 54.73 | 3.44 | 5.32 | 24.35 |
| Measured value (%) | 54.74 | 3.47 | 5.25 | 24.21 |
| Mass spectrometric value | (m/z) 263(M⁺),202 | | | |

| Example No. | 34 | | | | |
|---|---|---|---|---|---|
| Chemical name | 5-(6-fluoro-4-thiochromanylidene)-4-oxo-2-thioxo-thiazolidine | | | | |
| Structural formula | 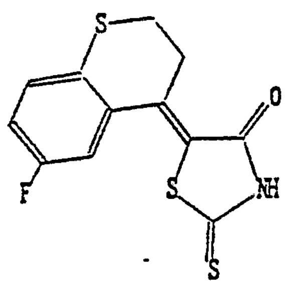 | | | | |
| m.p. (°C) | 165-168(dec.) | | | | |
| Solvent for recrystallization | Washing with $CHCl_3$ | | | | |
| Molecular formula | C12 H8 F N O S3 | | | | |
| Elementary analysis | C | H | N | S | F |
| Calculated value (%) | 48.46 | 2.71 | 4.71 | 32.35 | 6.39 |
| Measured value (%) | 47.96 | 2.64 | 4.57 | 32.32 | 6.14 |
| Mass spectrometric value | (m/z) 297($M^+$) | | | | |

| | |
|---|---|
| Example No. | 35 |
| Chemical name | 4-oxo-5-(4-thiochromanylidene)-2-thioxo-imidazolidine |
| Structural formula | 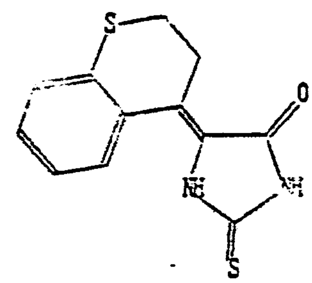 |
| m.p. (°C) | 242 - 243 |
| Solvent for recrystallization | AcOH |
| Molecular formula | C12 H10 N2 O S2 |
| Elementary analysis | C H N S |
| Calculated value (%) | 55.79 3.68 13.94 21.28 |
| Measured value (%) | 55.78 3.51 13.91 21.12 |
| Mass spectrometric value | (m/z) 301 ($M^+$), 214 |

| Example No. | 36 |
|---|---|
| Chemical name | 5-(5-methoxy-1,2,3,4-tetrahydro-1-naphthylidene)-4-oxo-2-thioxo-thiazolidine |
| Structural formula | 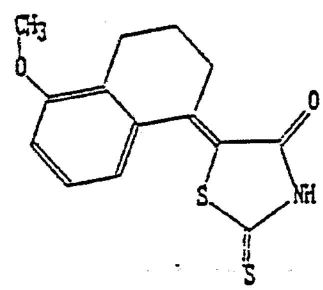 |
| m.p. (°C) | 176 - 177 |
| Solvent for recrystallization | AcOH |
| Molecular formula | C14 H13 N O2 S2 |
| Elementary analysis | C H N S |
| Calculated value (%) | 57.71 4.50 4.81 22.01 |
| Measured value (%) | 57.80 4.26 4.78 21.95 |
| Mass spectrometric value | (m/z) 291 ($M^+$), 204 |

| Example No. | 37 |
|---|---|
| Chemical name | 5-(6-methoxy-1,2,3,4-tetrahydro-1-naphthylidene)-4-oxo-2-thioxo-thiazolidine |
| Structural formula | 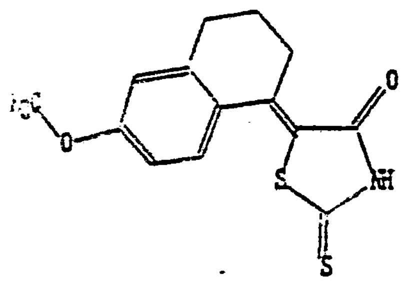 |
| m.p. (°C) | 208 - 210 |
| Solvent for recrystallization | AcOH |
| Molecular formula | $C_{14}H_{13}NO_2S_2$ |
| Elementary analysis | C / H / N / S |
| Calculated value (%) | 57.71 / 4.50 / 4.81 / 22.01 |
| Measured value (%) | 57.72 / 4.43 / 4.71 / 21.01 |
| Mass spectrometric value | (m/z) 291($M^+$), 204 |

| Example No. | 38 | | | |
|---|---|---|---|---|
| Chemical name | 5-(5,7-dimethyl-1,2,3,4-tetrahydro-1-natphylidene)-4-oxo-2-thioxo-thiazolidine | | | |
| Structural formula | 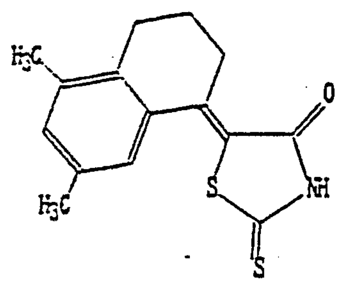 | | | |
| m.p. (°C) | 245 - 246 | | | |
| Solvent for recrystallization | Acetic acid | | | |
| Molecular formula | C15 H15 N O S2 | | | |
| Elementary analysis | C | H | N | S |
| Calculated value (%) | 62.25 | 5.22 | 4.84 | 22.16 |
| Measured value (%) | 62.05 | 5.05 | 4.62 | 22.09 |
| Mass spectrometric value | (m/z) 289 ($M^+$), 202 | | | |

| Example No. | 39 | | |
|---|---|---|---|
| Chemical name | 5-(6-amino-4-thiochromanylidene)-4-oxo-2-thioxo-thiazolidine | | |
| Structural formula | 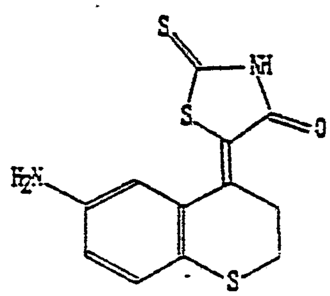 | | |
| m.p. (°C) | 220 < | | |
| Solvent for recrystallization | Benzene | | |
| Molecular formula | C12 H10 N2 O S3 | | |
| Elementary analysis | C | H | N |
| Calculated value (%) | 48.95 | 3.42 | 9.51 |
| Measured value (%) | 48.66 | 3.52 | 9.40 |
| Mass spectrometric value | (m/z) 294 (M$^+$), 207, 162 | | |

| | |
|---|---|
| Example No. | 40 |
| Chemical name | 5-(2-methyl-4-thiochromanyl-idene)-4-oxo-2-thioxo-thiazolidine |
| Structural formula | 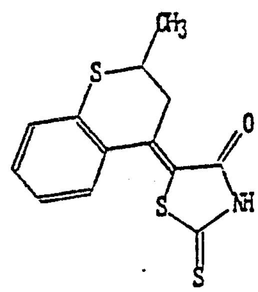 |
| m.p. (°C) | 173-175 |
| Solvent for recrystallization | Ether |
| Molecular formula | C13 H11 N O S3 |
| Elementary analysis | C  H  N  S |
| Calculated value (%) | 53.21  3.78  4.77  32.78 |
| Measured value (%) | 53.07  3.84  4.76  32.53 |
| Mass spectrometric value | (m/z) 293 ($M^+$), 201 |

| Example No. | 41 |
| --- | --- |
| Chemical name | 4-oxo-5-(6,7,8,9-tetrahydro-5H-benzocyclohepten-5-ylidene)-2-thioxo-thiazolidine |
| Structural formula | 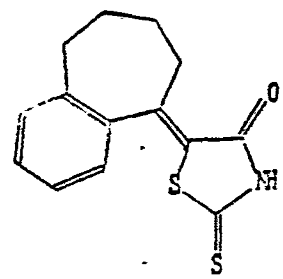 |
| m.p. (°C) | 177-178 |
| Solvent for recrystallization | Benzene |
| Molecular formula | C14 H13 N O S2 |
| Elementary analysis | C H N S |
| Calculated value (%) | 61.06 4.76 5.09 23.29 |
| Measured value (%) | 60.90 4.68 5.07 23.22 |
| Mass spectrometric value | (m/z) 275($M^+$), 188 |

| Example No. | 42 | | | |
|---|---|---|---|---|
| Chemical name | 4-oxo-5-(2-phenyl-4-thiochroman-ylidene)-2-thioxo-thiazolidine | | | |
| Structural formula | 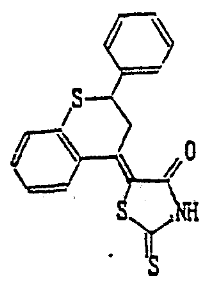 | | | |
| m.p. (°C) | 227-230 | | | |
| Solvent for recrystallization | $CHCl_3$ | | | |
| Molecular formula | C18 H13 N O S3 | | | |
| Elementary analysis | C | H | N | S |
| Calculated value (%) | 60.81 | 3.69 | 3.94 | 27.06 |
| Measured value (%) | 60.73 | 3.52 | 3.81 | 26.89 |
| Mass spectrometric value | (m/z) 355 ($M^+$), | | | |

| Example No. | 43 | | | |
|---|---|---|---|---|
| Chemical name | 4-(4-oxo-2-thioxo-5-thiazolidin-ylidene)thiochroman 1,1-dioxide | | | |
| Structural formula | 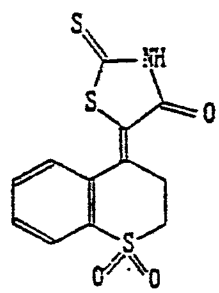 | | | |
| m.p. (°C) | 273 | | | |
| Solvent for recrystallization | Toluene | | | |
| Molecular formula | $C_{12}H_9NO_3S_3$ | | | |
| Elementary analysis | C | H | N | S |
| Calculated value (%) | 46.28 | 2.91 | 4.50 | 30.89 |
| Measured value (%) | 46.35 | 2.89 | 4.48 | 30.74 |
| Mass spectrometric value | (m/z) 311($M^+$), 160 | | | |

| Example No. | 44 |
|---|---|
| Chemical name | 5-(3,4-dihydro-1H-2-benzothiopyran-4-ylidene)-4-oxo-2-thioxothiazolidine |
| Structural formula | 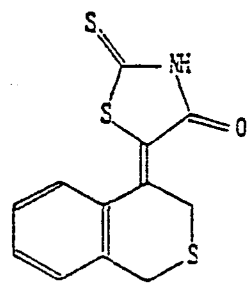 |
| m.p. (°C) | 192-194 |
| Solvent for recrystallization | $CHCl_3$ |
| Molecular formula | $C_{12}H_9NOS_3$ |
| Elementary analysis | C H N S |
| Calculated value (%) | 51.58 3.25 5.01 34.43 |
| Measured value (%) | 51.32 3.11 5.11 34.38 |
| Mass spectrometric value | (m/z) 279 ($M^+$), 188 |

| Example No. | 45 |
|---|---|
| Chemical name | 5-(3-benzo[b]thienyl)-4-oxo-2-thioxo-thiazolidine |
| Structural formula | 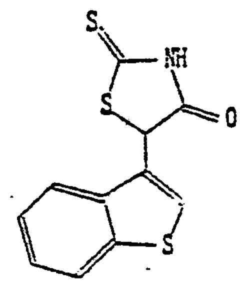 |
| m.p. (°C) | 149-150 |
| Solvent for recrystallization | $CHCl_3$ |
| Molecular formula | C11 H7 N O S3 |
| Elementary analysis | C  H  N  S |
| Calculated value (%) | 49.79  2.66  5.28  36.25 |
| Measured value (%) | 49.55  2.46  5.28  36.27 |
| Mass spectrometric value | (m/z) 265(M⁻), 174 |

| | |
|---|---|
| Example No. | 46 |
| Chemical name | 4-oxo-5-(2,2,3,4-tetrahydro-1-benzothiepin-5-ylidene)-2-thioxo-thiazolidine |
| Structural formula | 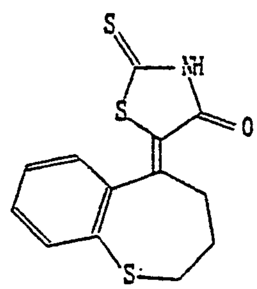 |
| m.p. (°C) | 174-175 |
| Solvent for recrystallization | $CHCl_3$ - ether |
| Molecular formula | C13 H11 N O S3 |
| Elementary analysis | C H N S |
| Calculated value (%) | 53.21 3.78 4.77 32.78 |
| Measured value (%) | 53.16 3.60 4.72 32.91 |
| Mass spectrometric value | (m/z) 293 ($M^-$), 178, 134 |

| Example No. | 47 | | | |
|---|---|---|---|---|
| Chemical name | 2-methyl-4-(4-oxo-2-thioxo-5-thiazolidinylidene)thiochroman 1,1-dioxide | | | |
| Structural formula | 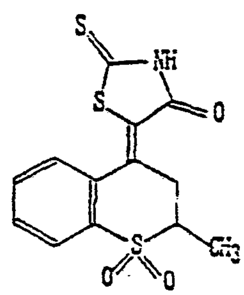 | | | |
| m.p. (°C) | 225-230 | | | |
| Solvent for recrystallization | $CHCl_3$ | | | |
| Molecular formula | C13 H11 N O3 S3 | | | |
| Elementary analysis | C | H | N | S |
| Calculated value (%) | 47.98 | 3.41 | 4.30 | 29.56 |
| Measured value (%) | 47.90 | 3.24 | 4.26 | 29.38 |
| Mass spectrometric value | (m/z) 325 (M⁺), 174, 161 | | | |

| Example No. | 48 | | | |
|---|---|---|---|---|
| Chemical name | 2,4-dioxo-5-(2,3,4,5-tetrahydro-1-benzothiepin-5-ylidene)thiazolidine | | | |
| Structural formula | 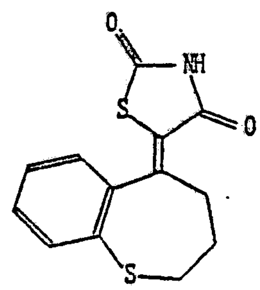 | | | |
| m.p. (°C) | 132-134 | | | |
| Solvent for recrystallization | $CHCl_3$ – ether | | | |
| Molecular formula | C13 H11 N O2 S2 | | | |
| Elementary analysis | C | H | N | S |
| Calculated value (%) | 56.29 | 4.00 | 5.05 | 23.12 |
| Measured value (%) | 56.39 | 3.75 | 5.02 | 23.24 |
| Mass spectrometric value | (m/z) 277($M^+$), 178, 134 | | | |

| Example No. | 49 | | | |
|---|---|---|---|---|
| Chemical name | 2,3,4,5-tetrahydro-5-(4-oxo-2-thioxo-5-thiazolidinylidene)-1-benzothiepine 1,1-dioxide | | | |
| Structural formula | 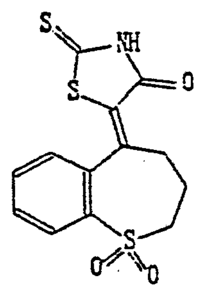 | | | |
| m.p. (°C) | 293-296 | | | |
| Solvent for recrystallization | EtOAc | | | |
| Molecular formula | C13 H11 N 03 S3 | | | |
| Elementary analysis | C | H | N | S |
| Calculated value (%) | 47.98 | 3.41 | 4.30 | 29.56 |
| Measured value (%) | 48.09 | 3.29 | 4.12 | 29.37 |
| Mass spectrometric value | (m/z) 325 ($M^+$), 238 | | | |

| Example No. | 50 | | | |
|---|---|---|---|---|
| Chemical name | 4-(4-oxo-2-thioxo-5-thiazolidin-ylidene)-2-phenyl-thiochroman 1,1-dioxide | | | |
| Structural formula | 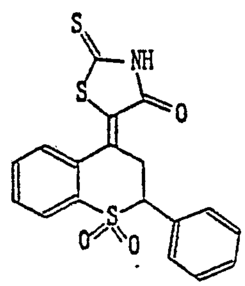 | | | |
| m.p. (°C) | 258-271 | | | |
| Solvent for recrystallization | EtOAc | | | |
| Molecular formula | C18 H13 N O3 S3 | | | |
| Elementary analysis | C | H | N | S |
| Calculated value (%) | 55.79 | 3.38 | 3.61 | 24.83 |
| Measured value (%) | 55.69 | 3.48 | 3.85 | 24.91 |
| Mass spectrometric value | (m/z) 387 ($M^+$), 264, 235 | | | |

| Example No. | 51 | | | |
|---|---|---|---|---|
| Chemical name | 4-(3-carboxymethyl-4-oxo-2-thioxo-5-thiazolidinylidene)thiochroman 1,1-dioxide | | | |
| Structural formula | 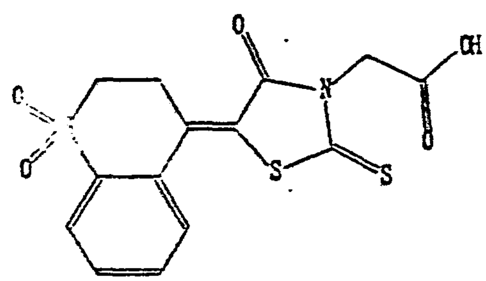 | | | |
| m.p. (°C) | 135-140 | | | |
| Solvent for recrystallization | Toluene | | | |
| Molecular formula | C14 H11 N O5 S3 | | | |
| Elementary analysis | C | H | N | S |
| Calculated value (%) | 45.52 | 3.00 | 3.79 | 26.04 |
| Measured value (%) | 45.35 | 3.01 | 3.72 | 25.96 |
| Mass spectrometric value | (m/z) 369 (M+), | | | |

| Example No. | 52 |
|---|---|
| Chemical name | 4-(3-ethyl-4-oxo-2-thioxo-5-thiazolidinylidene)thiochroman 1,1-dioxide |
| Structural formula | 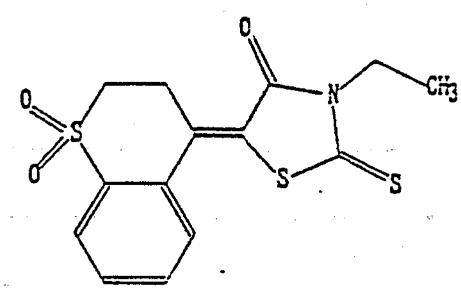 |
| m.p. (°C) | 177-179 |
| Solvent for recrystallization | $CHCl_3$ |
| Molecular formula | C14 H13 N O3 S3 |
| Elementary analysis | C H N S |
| Calculated value (%) | 49.54 3.86 4.13 28.34 |
| Measured value (%) | 49.30 3.74 4.19 28.58 |
| Mass spectrometric value | (m/z) 339 ($M^+$), 160 |

| Example No. | 53 |
|---|---|
| Chemical name | 4-(3-amino-4-oxo-2-thioxo-5-thiazolidinylidene)thiochroman 1,1-dioxid |
| Structural formula | 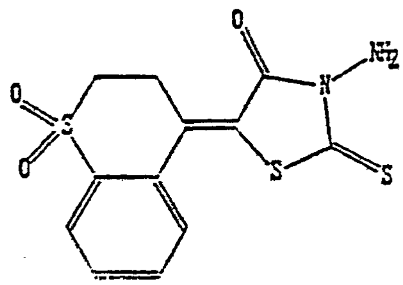 |
| m.p. (°C) | 192-195 |
| Solvent for recrystallization | MeOH |
| Molecular formula | C12 H10 N2 O3 S3 |
| Elementary analysis | C H N S |
| Calculated value (%) | 44.16 3.09 8.58 29.47 |
| Measured value (%) | 44.43 3.04 8.31 29.24 |
| Mass spectrometric value | (m/z) 326 ($M^+$), 187 |

| Example No. | 54 | | | |
|---|---|---|---|---|
| Chemical name | 4-[3-(p-carboxyphenyl)-4-oxo-2-thioxo-5-thiazolidinylidene]thiochroman 1,1-dioxide | | | |
| Structural formula | 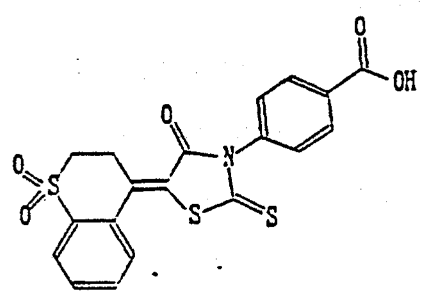 | | | |
| m.p. (°C) | 250< | | | |
| Solvent for recrystallization | $CHCl_3$ | | | |
| Molecular formula | C19 H13 N O5 S3 | | | |
| Elementary analysis | C | H | N | S |
| Calculated value (%) | 52.89 | 3.04 | 3.24 | 22.29 |
| Measured value (%) | 52.87 | 3.12 | 3.31 | 22.34 |
| Mass spectrometric value | (m/z) 431 ($M^+$), | | | |

EXAMPLE 55

1.10 g of hydantoin, 1.92 g of 5-nitroisatin, 0.8 g of ammonium acetate and 70 ml of acetic acid were put into an egg-plant type flask having a capacity of 200 ml, which was provided with a Dean-Stark dehydration device and which was filled with zeolite, and then the whole was heated and stirred in an oil bath at 150 to 180°C for 12 hours. The crystals as freshly precipitated were taken out by filtration while heating and thereafter washed with a small amount of hot acetic acid and then with 10 ml of acetic acid, to obtain 0.5 g (17.2%) of the product 5-nitro-3-(5-oxo-2-thioxo-4-imidazolidinylidene)-2-indoline.

Physico-chemical properties of the product are shown in the following Table. In the same manner as Example 55, the following compounds of Examples 56 to 95 were obtained.

| Example No. | 55 | | | |
|---|---|---|---|---|
| Chemical name | 5-nitro-3-(5-oxo-2-thioxo-4-imidazolidinylidene)-2-indoline | | | |
| Structural formula | 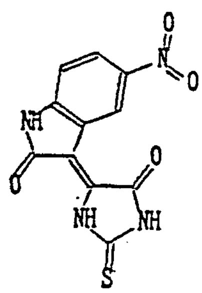 | | | |
| m.p. (°C) | 250< | | | |
| Solvent for recrystallization | AcOH | | | |
| Molecular formula | C11 H6 N4 O4 S | | | |
| Elementary analysis | C | H | N | S |
| Calculated value (%) | 45.52 | 2.08 | 19.30 | 11.05 |
| Measured value (%) | 45.59 | 2.03 | 19.28 | 10.77 |
| Mass spectrometric value | (m/z) 290 ($M^+$), 203 | | | |

| Example No. | 56 | | | |
|---|---|---|---|---|
| Chemical name | 4-oxo-5-(2-oxo-3-indolinylidene)-2-thioxo-3-thiazolidine-acetic acid | | | |
| Structural formula | 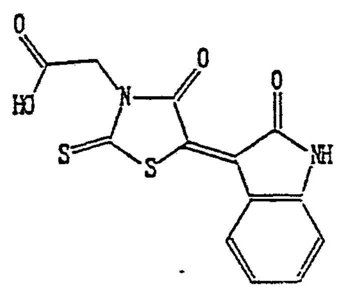 | | | |
| m.p. (°C) | 300< | | | |
| Solvent for recrystallization | AcOH | | | |
| Molecular formula | C13 H8 N2 O4 S2 | | | |
| Elementary analysis | C | H | N | S |
| Calculated value (%) | 48.74 | 2.52 | 8.74 | 20.02 |
| Measured value (%) | 48.04 | 2.46 | 8.32 | 19.52 |
| Mass spectrometric value | (m/z) 320 (M⁻), 175 | | | |

| Example No. | 57 | | | | |
|---|---|---|---|---|---|
| Chemical name | 5-(5-bromo-2-oxo-3-indolinylidene)-4-oxo-2-thioxo-3-thiazolidine-acetic acid | | | | |
| Structural formula | 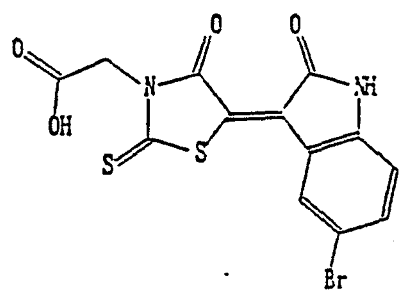 | | | | |
| m.p. (°C) | 250< | | | | |
| Solvent for recrystallization | DMSO+AcOH | | | | |
| Molecular formula | C13 H7 Br N2 04 S2 | | | | |
| Elementary analysis | C | H | N | S | Br |
| Calculated value (%) | 39.11 | 1.77 | 7.02 | 16.06 | 20.01 |
| Measured value (%) | 39.22 | 1.50 | 7.00 | 16.24 | 19.77 |
| Mass spectrometric value | (m/z) 398 (M−1), 400(M+1) | | | | |

| Example No. | 58 |
| --- | --- |
| Chemical name | 5-(5-nitro-2-oxo-3-indolinylidene)-4-oxo-2-thioxo-3-thiazilidine-acetic acid |
| Structural formula | 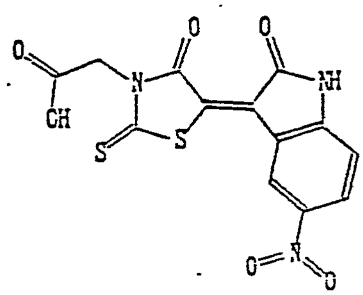 |
| m.p. (°C) | 300< |
| Solvent for recrystallization | DMSO+AcOH |
| Molecular formula | C13 H7 N3 O6 S2 |
| Elementary analysis | C H N S |
| Calculated value (%) | 42.74 1.93 11.50 17.55 |
| Measured value (%) | 42.88 1.66 11.52 17.39 |
| Mass spectrometric value | (m/z) 365(M⁺), 220 |

| | | | | |
|---|---|---|---|---|
| Example No. | 59 | | | |
| Chemical name | 4-oxo-5-(2-oxo-5-sodiooxysulfonyl-3-indolinylidene)-2-thioxo-3-thiazolidine-acetic acid | | | |
| Structural formula | 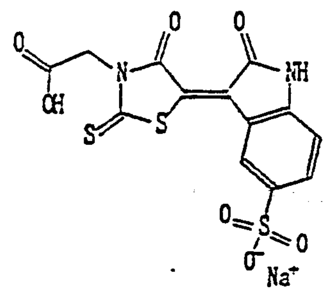 | | | |
| m.p. (°C) | 300< | | | |
| Solvent for recrystallization | DMSO+AcOH | | | |
| Molecular formula | C13 H7 N2 Na O7 S3 | | | |
| Elementary analysis | C | H | N | S |
| Calculated value (%) | 36.97 | 1.67 | 6.63 | 22.77 |
| Measured value (%) | 36.69 | 1.88 | 6.50 | 22.65 |
| Mass spectrometric value | ———— | | | |

| | | | | | |
|---|---|---|---|---|---|
| Example No. | 60 | | | | |
| Chemical name | 5-(5-chloro-2-oxo-3-indolin-ylidene)-4-oxo-2-thioxo-3-thiazolidine-acetic acid | | | | |
| Structural formula | 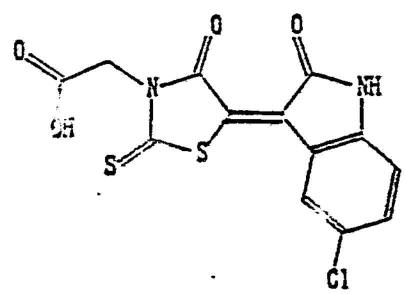 | | | | |
| m.p. (°C) | 300< | | | | |
| Solvent for recrystallization | DMSO+AcOH | | | | |
| Molecular formula | C13 H7 Cl N2 O4 S2 | | | | |
| Elementary analysis | C | H | N | S | Cl |
| Calculated value (%) | 44.01 | 1.99 | 7.90 | 18.07 | 9.99 |
| Measured value (%) | 43.94 | 1.72 | 7.82 | 18.29 | 9.63 |
| Mass spectrometric value | (m/z) 354 (M⁺), 209 | | | | |

| Example No. | 61 |
| --- | --- |
| Chemical name | 5-(5-methyl-2-oxo-3-indolin-ylidene)-4-oxo-2-thioxo-3-thiazolidine-acetic acid |
| Structural formula | 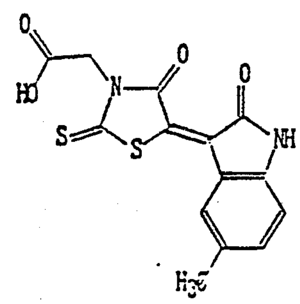 |
| m.p. (°C) | 300< |
| Solvent for recrystallization | DMSO+AcOH |
| Molecular formula | C14 H10 N2 04 S2 |
| Elementary analysis<br>Calculated value (%)<br>Measured value (%) | C H N S<br>50.29 3.01 8.38 19.18<br>50.29 2.80 8.30 19.36 |
| Mass spectrometric value | (m/z) 334 ($M^+$), 189 |

| Example No. | 62 | | | |
|---|---|---|---|---|
| Chemical name | 5-(1-methyl-2-oxo-3-indolin-ylidene)-4-oxo-2-thioxo-3-thiazolidine-acetic acid | | | |
| Structural formula | 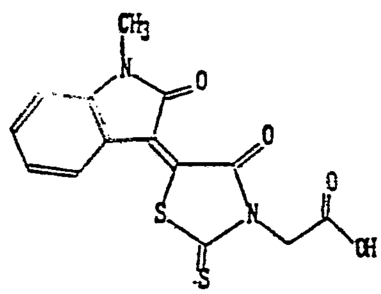 | | | |
| m.p. (°C) | 200< | | | |
| Solvent for recrystallization | AcOH | | | |
| Molecular formula | $C_{14}H_{10}N_2O_4S_2$ | | | |
| Elementary analysis | C | H | N | S |
| Calculated value (%) | 50.29 | 3.01 | 8.38 | 19.18 |
| Measured value (%) | 50.23 | 2.86 | 8.38 | 19.44 |
| Mass spectrometric value | (m/z) 334(M⁻), 189 | | | |

| | |
|---|---|
| Example No. | 63 |
| Chemical name | 3-(3-amino-4-oxo-2-thioxo-5-thiazolidinylidene)-2-indolinone |
| Structural formula | 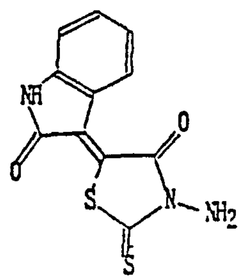 |
| m.p. (°C) | 200< |
| Solvent for recrystallization | AcOH |
| Molecular formula | C11 H7 N3 O2 S2 |
| Elementary analysis | C H N S |
| Calculated value (%) | 47.64 2.54 15.15 23.13 |
| Measured value (%) | 47.77 2.47 15.37 23.27 |
| Mass spectrometric value | (m/z) 277 (M⁺), 175 |

| Example No. | 64 |
|---|---|
| Chemical name | 3-(2-imino-4-oxo-5-thiazolidinylidene)-2-indolinone |
| Structural formula | 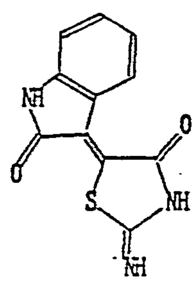 |
| m.p. (°C) | 300< |
| Solvent for recrystallization | AcOH |
| Molecular formula | C11 H7 N3 O2 S |
| Elementary analysis | C H N S |
| Calculated value (%) | 53.87 2.88 17.13 13.07 |
| Measured value (%) | 53.99 2.92 17.03 12.81 |
| Mass spectrometric value | (m/z) 245 (M+), 175 |

| Example No. | 65 | | | |
|---|---|---|---|---|
| Chemical name | 3-(5-oxo-2-thioxo-4-imidazolidin-ylidene)-2-indolinone | | | |
| Structural formula | 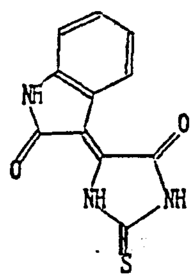 | | | |
| m.p. (°C) | 250 < | | | |
| Solvent for recrystallization | AcOH | | | |
| Molecular formula | $C_{11}H_7N_3O_2S$ | | | |
| Elementary analysis | C | H | N | S |
| Calculated value (%) | 53.87 | 2.88 | 17.13 | 13.07 |
| Measured value (%) | 54.02 | 2.87 | 17.30 | 12.99 |
| Mass spectrometric value | (m/z) 245 ($M^+$), 158 | | | |

| | | | | | |
|---|---|---|---|---|---|
| Example No. | 66 | | | | |
| Chemical name | 5-chloro-3-(4-oxo-2-thioxo-5-thiazolidinylidene)-2-indolinone | | | | |
| Structural formula | 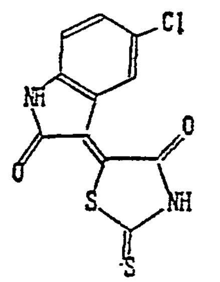 | | | | |
| m.p. (°C) | 250< | | | | |
| Solvent for recrystallization | AcOH | | | | |
| Molecular formula | C11 H5 Cl N2 O2 S2 | | | | |
| Elementary analysis | C | H | N | S | Cl |
| Calculated value (%) | 44.52 | 1.70 | 9.44 | 21.61 | 11.95 |
| Measured value (%) | 44.74 | 1.89 | 9.49 | 21.60 | 11.85 |
| Mass spectrometric value | (m/z) 296 (M⁻), 209 | | | | |

| Example No. | 67 |
|---|---|
| Chemical name | 5-bromo-3-(4-oxo-2-thioxo-5-thiazolidinylidene)-2-indolinone |
| Structural formula | 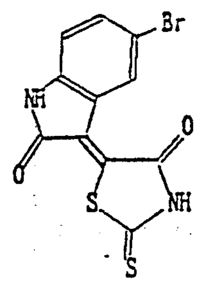 |
| m.p. (°C) | 250< |
| Solvent for recrystallization | AcOH |
| Molecular formula | C11 H5 Br N2 O2 S2 |
| Elementary analysis | C H N S Br |
| Calculated value (%) | 38.72 1.48 8.21 18.80 23.42 |
| Measured value (%) | 38.93 1.22 8.24 18.94 23.50 |
| Mass spectrometric value | (m/z) 340 (M−1), 342 (M+1) |

| Example No. | 68 | | | |
|---|---|---|---|---|
| Chemical name | 5-nitro-3-(4-oxo-2-thioxo-5-thiazolidinylidene)-2-indolinone | | | |
| Structural formula | 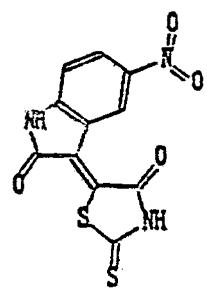 | | | |
| m.p. (°C) | 250< | | | |
| Solvent for recrystallization | AcOH | | | |
| Molecular formula | C11 H5 N3 O4 S2 | | | |
| Elementary analysis | C | H | N | S |
| Calculated value (%) | 42.99 | 1.64 | 13.67 | 20.87 |
| Measured value (%) | 42.94 | 1.41 | 13.65 | 20.57 |
| Mass spectrometric value | (m/z) 307 ($M^+$), 220 | | | |

| Example No. | 69 | | | |
|---|---|---|---|---|
| Chemical name | 5-methyl-3-(4-oxo-2-thioxo-5-thiazolidinylidene)-2-indolinone | | | |
| Structural formula | 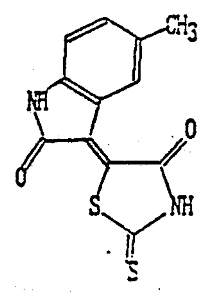 | | | |
| m.p. (°C) | 250< | | | |
| Solvent for recrystallization | AcOH | | | |
| Molecular formula | C12 H8 N2 O2 S2 | | | |
| Elementary analysis | C | H | N | S |
| Calculated value (%) | 52.16 | 2.92 | 10.14 | 23.21 |
| Measured value (%) | 52.18 | 2.72 | 10.17 | 23.03 |
| Mass spectrometric value | (m/z) 276 (M⁻), 189 | | | |

| Example No. | 70 | | | |
|---|---|---|---|---|
| Chemical name | Sodium 2-oxo-3-(4-oxo-2-thioxo-5-thiazolidinylidene)-5-indoline-sulfonate | | | |
| Structural formula | 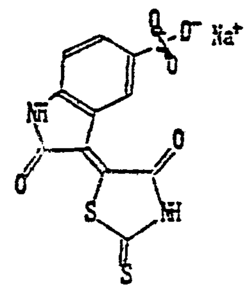 | | | |
| m.p. (°C) | 250< | | | |
| Solvent for recrystallization | AcOH | | | |
| Molecular formula | C11 H5 N2 Na O5 S3 | | | |
| Elementary analysis | C | H | N | S |
| Calculated value (%) | 36.26 | 1.38 | 7.69 | 26.40 |
| Measured value (%) | 35.92 | 1.17 | 7.69 | 26.06 |
| Mass spectrometric value | (m/z) 365 (M⁻), | | | |

| Example No. | 71 |
|---|---|
| Chemical name | 5-chloro-3-(2,4-dioxo-5-thiazolidinylidene)-2-indolinone |
| Structural formula | 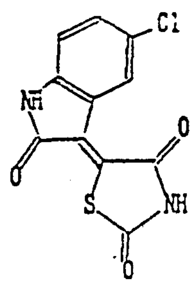 |
| m.p. (°C) | 250< |
| Solvent for recrystallization | AcOH |
| Molecular formula | C11 H5 Cl N2 O3 S |
| Elementary analysis | C H N S |
| Calculated value (%) | 47.07 1.80 9.98 11.42 |
| Measured value (%) | 47.08 1.76 9.92 11.38 |
| Mass spectrometric value | (m/z) 280 (M⁻), 209 |

| Example No. | 72 |
| --- | --- |
| Chemical name | 3-(2,4-dioxo-5-thiazolidinylidene)-5-nitro-2-indolinone |
| Structural formula | 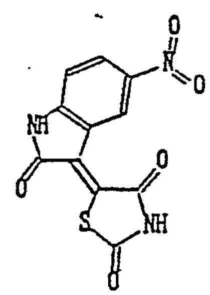 |
| m.p. (°C) | 250< |
| Solvent for recrystallization | AcOH |
| Molecular formula | $C_{11}H_5N_3O_5S$ |
| Elementary analysis | C H N S |
| Calculated value (%) | 45.36 1.73 14.43 11.01 |
| Measured value (%) | 45.76 1.76 14.40 10.82 |
| Mass spectrometric value | (m/z) 291 ($M^+$), 220 |

| Example No. | 73 | | | |
|---|---|---|---|---|
| Chemical name | 3-(2,4-dioxo-5-thiazolidin-ylidene)-5-methyl-2-indolinone | | | |
| Structural formula | 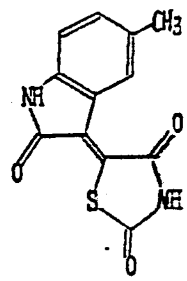 | | | |
| m.p. (°C) | 250< | | | |
| Solvent for recrystallization | AcOH | | | |
| Molecular formula | $C_{12}H_8N_2O_3S$ | | | |
| Elementary analysis | C | H | N | S |
| Calculated value (%) | 55.38 | 3.10 | 10.76 | 12.32 |
| Measured value (%) | 55.58 | 3.17 | 10.63 | 12.25 |
| Mass spectrometric value | (m/z) 260 ($M^+$), 189 | | | |

| Example No. | 74 |
| --- | --- |
| Chemical name | Sodium 2-oxo-3-(2,4-dioxo-5-thiazolidinylidene)-5-indoline-sulfonate |
| Structural formula | 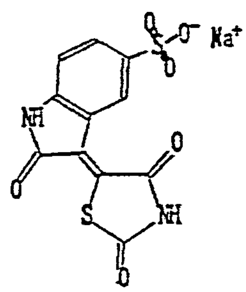 |
| m.p. (°C) | 250< |
| Solvent for recrystallization | AcOH |
| Molecular formula | C11 H5 N2 Na O6 S2 |
| Elementary analysis | C H N |
| Calculated value (%) | 37.93 1.45 8.04 |
| Measured value (%) | 37.91 1.75 7.97 |
| Mass spectrometric value | (m/z) 349 (M+1) |

| Example No. | 75 |
| --- | --- |
| Chemical name | 5-chloro-3-(2,5-dioxo-4-imidazolidinylidene)-2-indolinone |
| Structural formula | 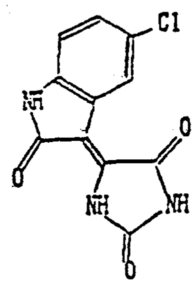 |
| m.p. (°C) | 250< |
| Solvent for recrystallization | AcOH |
| Molecular formula | C11 H6 Cl N3 O3 |

| Elementary analysis | C | H | N | S |
| --- | --- | --- | --- | --- |
| Calculated value (%) | 50.11 | 2.29 | 15.94 | 13.45 |
| Measured value (%) | 50.08 | 1.96 | 15.62 | 13.35 |

| Mass spectrometric value | (m/z) 263 ($M^+$) |
| --- | --- |

| Example No. | 76 | | |
|---|---|---|---|
| Chemical name | 3-(2,5-dioxo-4-imidazolidin-ylidene)-5-nitro-2-indolinone | | |
| Structural formula | 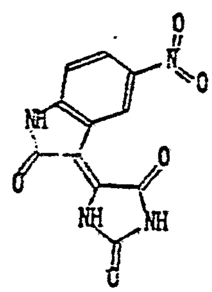 | | |
| m.p. (°C) | 250< | | |
| Solvent for recrystallization | AcOH | | |
| Molecular formula | C11 H6 N4 O5 | | |
| Elementary analysis | C | H | N |
| Calculated value (%) | 48.19 | 2.21 | 20.43 |
| Measured value (%) | 48.29 | 2.24 | 20.41 |
| Mass spectrometric value | (m/z) 274 ($M^+$) | | |

| Example No. | 77 |
| --- | --- |
| Chemical name | 3-(2,5-dioxo-4-imidazolidin-ylidene)-5-methyl-2-indolinone |
| Structural formula | 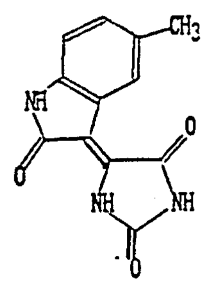 |
| m.p. (°C) | 250< |
| Solvent for recrystallization | AcOH |
| Molecular formula | C12 H9 N3 O3 |
| Elementary analysis | C  H  N |
| Calculated value (%) | 59.26  3.73  17.28 |
| Measured value (%) | 59.38  3.63  17.27 |
| Mass spectrometric value | (m/z) 243 ($M^+$), 172 |

| Example No. | 78 | | | |
|---|---|---|---|---|
| Chemical name | Sodium 3-(2,5-dioxo-4-imidazolidinylidene)-2-oxo-5-indolinesulfonate | | | |
| Structural formula | 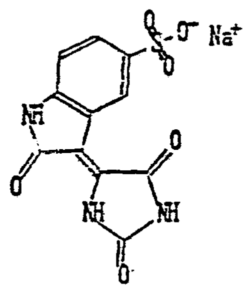 | | | |
| m.p. (°C) | 250< | | | |
| Solvent for recrystallization | AcOH | | | |
| Molecular formula | C11 H6 N3 Na O6 S | | | |
| Elementary analysis | C | H | N | S |
| Calculated value (%) | 39.89 | 1.83 | 12.69 | 9.68 |
| Measured value (%) | 39.82 | 2.04 | 12.37 | 9.52 |
| Mass spectrometric value | (m/z) 332 (M+1) | | | |

| Example No. | 79 | | | | |
|---|---|---|---|---|---|
| Chemical name | 5-chloro-3-(5-oxo-2-thioxo-4-imidazolidinylidene)-2-indolinone | | | | |
| Structural formula | 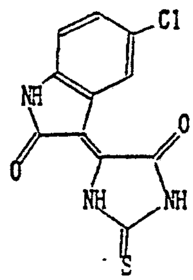 | | | | |
| m.p. (°C) | 250< | | | | |
| Solvent for recrystallization | AcOH | | | | |
| Molecular formula | C11 H6 Cl N3 O2 S | | | | |
| Elementary analysis | C | H | N | S | Cl |
| Calculated value (%) | 47.24 | 2.16 | 15.02 | 11.46 | 12.68 |
| Measured value (%) | 47.28 | 2.05 | 15.06 | 11.69 | 12.62 |
| Mass spectrometric value | (m/z) 279 ($M^+$), 192 | | | | |

| | | | | |
|---|---|---|---|---|
| Example No. | 80 | | | |
| Chemical name | 5-bromo-3-(2,5-dioxo-4-imidazolidinylidene)-2-indolinone | | | |
| Structural formula | 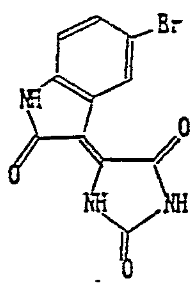 | | | |
| m.p. (°C) | 250< | | | |
| Solvent for recrystallization | AcOH | | | |
| Molecular formula | C11 H6 Br N3 O3 | | | |
| Elementary analysis | C | H | N | Br |
| Calculated value (%) | 42.88 | 1.96 | 13.64 | 25.94 |
| Measured value (%) | 42.69 | 1.71 | 13.06 | 25.78 |
| Mass spectrometric value | (m/z) 307(M−1), 309 (M+1) | | | |

| Example No. | 81 | | | | |
|---|---|---|---|---|---|
| Chemical name | 5-bromo-3-(5-oxo-2-thioxo-4-imidazolidinylidene)-2-indolinone | | | | |
| Structural formula | 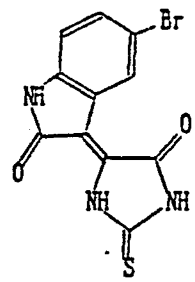 | | | | |
| m.p. (°C) | 250< | | | | |
| Solvent for recrystallization | AcOH | | | | |
| Molecular formula | C11 H6 Br N3 O2 S | | | | |
| Elementary analysis | C | H | N | S | Br |
| Calculated value (%) | 40.76 | 1.87 | 12.96 | 9.89 | 24.65 |
| Measured value (%) | 40.74 | 1.86 | 12.95 | 9.81 | 24.58 |
| Mass spectrometric value | (m/z) 323 (M−1), 325 (M+1) | | | | |

| Example No. | 82 | | | |
|---|---|---|---|---|
| Chemical name | 5-methyl-3-(5-oxo-2-thioxo-4-imidazolidinylidene)-2-indolinone | | | |
| Structural formula | 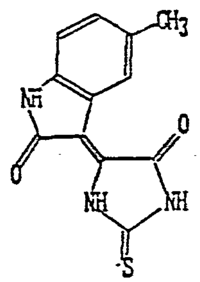 | | | |
| m.p. (°C) | 250< | | | |
| Solvent for recrystallization | AcOH | | | |
| Molecular formula | C12 H9 N3 O2 S | | | |
| Elementary analysis | C | H | N | S |
| Calculated value (%) | 55.59 | 3.50 | 16.21 | 12.37 |
| Measured value (%) | 55.61 | 3.46 | 16.10 | 12.61 |
| Mass spectrometric value | (m/z) 259 ($M^+$), 172 | | | |

| Example No. | 83 | | | |
|---|---|---|---|---|
| Chemical name | Sodium 2-oxo-3-(5-oxo-2-thioxo-4-imidazolidinylidene)-5-indoline-sulfonate | | | |
| Structural formula | 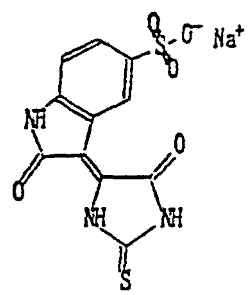 | | | |
| m.p. (°C) | 250< | | | |
| Solvent for recrystallization | AcOH | | | |
| Molecular formula | C11 H6 N3 Na O5 S2 | | | |
| Elementary analysis | C | H | N | S |
| Calculated value (%) | 38.04 | 1.74 | 12.10 | 18.47 |
| Measured value (%) | 37.86 | 1.67 | 12.10 | 18.19 |
| Mass spectrometric value | (m/z) 348 (M+1) | | | |

| Example No. | 84 | | | | |
|---|---|---|---|---|---|
| Chemical name | 5-chloro-3-(2-imino-4-oxo-5-thiazolidinylidene)-2-indolinone | | | | |
| Structural formula | 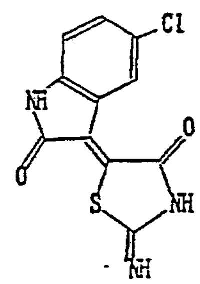 | | | | |
| m.p. (°C) | 250< | | | | |
| Solvent for recrystallization | AcOH | | | | |
| Molecular formula | $C_{11}H_6ClN_3O_2S$ | | | | |
| Elementary analysis | C | H | N | S | Cl |
| Calculated value (%) | 47.24 | 2.16 | 15.02 | 11.46 | 12.68 |
| Measured value (%) | 47.33 | 2.24 | 14.94 | 11.18 | 12.50 |
| Mass spectrometric value | (m/z) 279 ($M^+$), 209 | | | | |

| Example No. | 85 | | | |
|---|---|---|---|---|
| Chemical name | 3-(2-imino-4-oxo-5-thiazolidinylidene)-5-nitro-2-indolinone-acetic acid | | | |
| Structural formula | 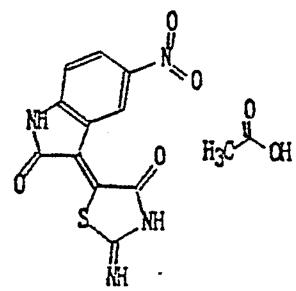 | | | |
| m.p. (°C) | 250 < | | | |
| Solvent for recrystallization | AcOH | | | |
| Molecular formula | C13 H10 N4 O6 S | | | |
| Elementary analysis | C | H | N | S |
| Calculated value (%) | 44.57 | 2.88 | 15.99 | 9.15 |
| Measured value (%) | 44.63 | 2.66 | 16.12 | 8.83 |
| Mass spectrometric value | (m/z) 290 (M⁺), 220 | | | |

| Example No. | 86 |
|---|---|
| Chemical name | 5-bromo-3-(2-imino-4-oxo-5-thiazolidinylidene)-2-indolinone |
| Structural formula | 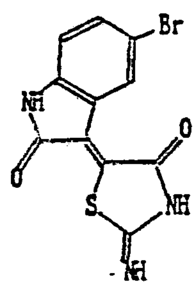 |
| m.p. (°C) | 250 < |
| Solvent for recrystallization | AcOH |
| Molecular formula | C11 H6 Br N3 O2 S |
| Elementary analysis | C H N S Br |
| Calculated value (%) | 40.76 1.87 12.96 9.89 24.65 |
| Measured value (%) | 40.96 1.84 12.98 9.74 24.53 |
| Mass spectrometric value | (m/z) 323 (M−1), 325 (M+1) |

| Example No. | 87 | | | |
|---|---|---|---|---|
| Chemical name | 3-(2-imino-4-oxo-5-thiazolidin-ylidene)-5-methyl-2-indolinone | | | |
| Structural formula | 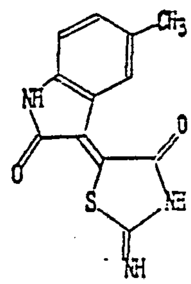 | | | |
| m.p. (°C) | 250< | | | |
| Solvent for recrystallization | AcOH | | | |
| Molecular formula | $C_{12}H_9N_3O_2S$ | | | |
| Elementary analysis | C | H | N | S |
| Calculated value (%) | 55.59 | 3.50 | 16.21 | 12.37 |
| Measured value (%) | 55.54 | 3.49 | 16.23 | 12.14 |
| Mass spectrometric value | (m/z) 259 ($M^+$), 189 | | | |

| Example No. | 88 |
|---|---|
| Chemical name | Sodium 3-(2-imino-4-oxo-5-thiazolidinylidene)-2-oxo-5-indoline-sulfonate |
| Structural formula | 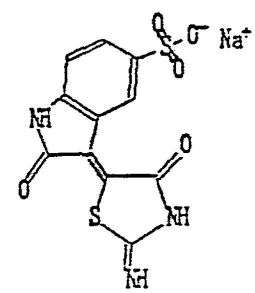 |
| m.p. (°C) | 250< |
| Solvent for recrystallization | AcOH |
| Molecular formula | C11 H6 N3 Na O5 S2 |
| Elementary analysis | C   H   N   S |
| Calculated value (%) | 38.04   1.74   12.10   18.47 |
| Measured value (%) | 37.86   1.81   11.90   18.21 |
| Mass spectrometric value | (m/z) 348 (M+1) |

| | | | | | |
|---|---|---|---|---|---|
| Example No. | 89 | | | | |
| Chemical name | 3-(3-amino-4-oxo-2-thioxo-5-thiazolidinylidene)-5-chloro-2-indolinone | | | | |
| Structural formula | 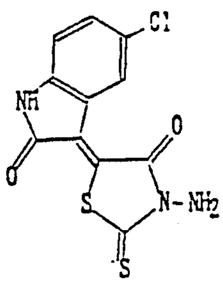 | | | | |
| m.p. (°C) | 250< | | | | |
| Solvent for recrystallization | AcOH | | | | |
| Molecular formula | C11 H6 Cl N3 O2 S2 | | | | |
| Elementary analysis | C | H | N | S | Cl |
| Calculated value (%) | 42.38 | 1.94 | 13.48 | 20.57 | 11.37 |
| Measured value (%) | 42.46 | 1.95 | 13.48 | 20.29 | 11.57 |
| Mass spectrometric value | (m/z) 311 (M⁻), 209 | | | | |

| Example No. | 90 |
|---|---|
| Chemical name | 3-(3-amino-4-oxo-2-thioxo-5-thiazolidinylidene)-5-nitro-2-indolinone |
| Structural formula | 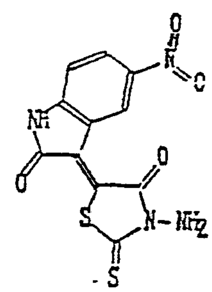 |
| m.p. (°C) | 250< |
| Solvent for recrystallization | AcOH |
| Molecular formula | C11 H5 N4 O4 S2 |
| Elementary analysis | C H N S |
| Calculated value (%) | 40.99 1.88 17.38 19.90 |
| Measured value (%) | 40.99 1.88 17.38 19.72 |
| Mass spectrometric value | (m/z) 322 ($M^+$), 220 |

| Example No. | 91 |
|---|---|
| Chemical name | 3-(3-amino-4-oxo-2-thioxo-5-thiazolidinylidene)-5-bromo-2-indolinone |
| Structural formula | 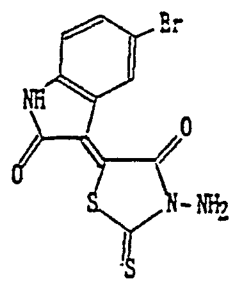 |
| m.p. (°C) | 250< |
| Solvent for recrystallization | AcOH |
| Molecular formula | C11 H6 Br N3 O2 S2 |

| Elementary analysis | C | H | N | S | Br |
|---|---|---|---|---|---|
| Calculated value (%) | 37.09 | 1.70 | 11.80 | 18.00 | 22.43 |
| Measured value (%) | 37.15 | 1.82 | 11.81 | 17.76 | 22.54 |

| Mass spectrometric value | (m/z) 355 (M−1), 357 (M+1) |
|---|---|

| Example No. | 92 | | | |
|---|---|---|---|---|
| Chemical name | 3-(3-amino-4-oxo-2-thioxo-5-thiazolidinylidene)-5-methyl-2-indolinone | | | |
| Structural formula | 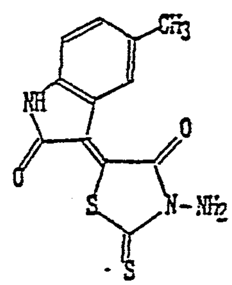 | | | |
| m.p. (°C) | 250< | | | |
| Solvent for recrystallization | AcOH | | | |
| Molecular formula | $C_{12}H_9N_3O_2S_2$ | | | |
| Elementary analysis | C | H | N | S |
| Calculated value (%) | 49.47 | 3.11 | 14.42 | 22.01 |
| Measured value (%) | 49.48 | 3.15 | 14.31 | 22.00 |
| Mass spectrometric value | (m/z) 291 ($M^-$), 189 | | | |

| Example No. | 93 |
|---|---|
| Chemical name | Sodium 3-(3-amino-4-oxo-2-thioxo-5-thiazolidinylidene)-2-oxo-5-indoline-sulfonate |
| Structural formula | 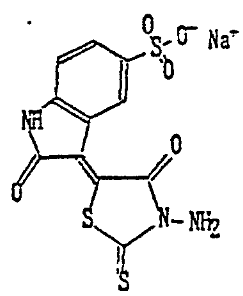 |
| m.p. (°C) | 250< |
| Solvent for recrystallization | AcOH |
| Molecular formula | C11 H6 N3 Na 05 S3 |

| Elementary analysis | C | H | N | S |
|---|---|---|---|---|
| Calculated value (%) | 34.83 | 1.59 | 11.08 | 25.36 |
| Measured value (%) | 34.83 | 1.71 | 11.38 | 25.21 |

| Mass spectrometric value | ———— |
|---|---|

| Example No. | 94 | | | |
|---|---|---|---|---|
| Chemical name | 4-(5-oxo-2-thioxo-4-imidazolidinylidene)-thiochroman 1,1-dioxide | | | |
| Structural formula | 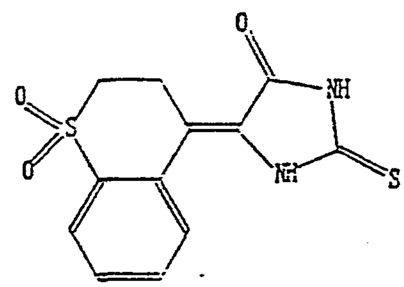 | | | |
| m.p. (°C) | 250< | | | |
| Solvent for recrystallization | AcOH | | | |
| Molecular formula | C12 H10 N2 O3 S2 | | | |
| Elementary analysis | C | H | N | S |
| Calculated value (%) | 48.97 | 3.42 | 9.52 | 21.79 |
| Measured value (%) | 48.87 | 3.30 | 9.42 | 21.71 |
| Mass spectrometric value | (m/z) 294 (M⁺), 230 | | | |

| Example No. | 95 |
|---|---|
| Chemical name | 4-(2-imino-4-oxo-5-thiazolidinylidene)thiochroman 1,1-dioxide |
| Structural formula | 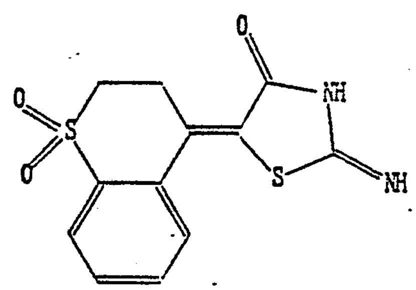 |
| m.p. (°C) | 200<, (colored above 240) |
| Solvent for recrystallization | AcOH |
| Molecular formula | C12 H10 N2 O3 S2 |
| Elementary analysis | C H N S |
| Calculated value (%) | 48.97 3.42 9.52 21.79 |
| Measured value (%) | 48.90 3.45 9.24 22.03 |
| Mass spectrometric value | (m/z) 294 ($M^+$), 230, 160 |

## EXAMPLE 96

5.26 g of 2,4-dioxo-5-(4-thiochromanylidene)thiazolidine as obtained in Example 33 was dissolved in 50 ml of acetic acid, and 1.9 g of 35% hydrogen peroxide aqueous solution was added thereto while stirring. After stirring overnight at room temperature, the crystals precipitated were filtered from the filtrate (A). The crystals were fully washed with water and then with 30 ml of methanol, to obtain 2.5 g of 4-(2,4-dioxo-5-thiazolidinylidene)thiochroman 1-oxide. Physico-chemical properties of the product are shown in the following Table.

## EXAMPLE 97

In the same manner as Example 96, the product shown below was obtained from the starting compound 2,4-dioxo-5-(2,3,4,5-tetrahydro-1-benzothiepin-5-ylidene)thiazolidine as obtained in Example 48. Physico-chemical properties of the product are shown in the following Table.

| Example No. | 96 | | | |
|---|---|---|---|---|
| Chemical name | 4-(2,4-dioxo-5-thiazolidinylidene)-thiochroman 1-oxide | | | |
| Structural formula | 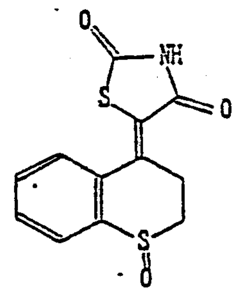 | | | |
| m.p. (°C) | 207-211 | | | |
| Solvent for recrystallization | MeOH | | | |
| Molecular formula | C12 H9 N O3 S2 | | | |
| Elementary analysis | C | H | N | S |
| Calculated value (%) | 51.60 | 3.25 | 5.01 | 22.96 |
| Measured value (%) | 51.62 | 3.06 | 4.99 | 22.78 |
| Mass spectrometric value | (m/z) 280 (M+1), | | | |

| Example No. | 97 | | | |
|---|---|---|---|---|
| Chemical name | 5-(2,4-dioxo-5-thiazolidinylidene)-2,3,4,5-tetrahydro-1-benzothiepine 1-oxide | | | |
| Structural formula | 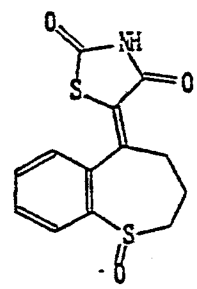 | | | |
| m.p. (°C) | 257-258 | | | |
| Solvent for recrystallization | AcOH or MeOH | | | |
| Molecular formula | C13 H11 N O3 S2 | | | |
| Elementary analysis | C | H | N | S |
| Calculated value (%) | 53.22 | 3.78 | 4.77 | 21.86 |
| Measured value (%) | 53.24 | 3.58 | 4.67 | 21.94 |
| Mass spectrometric value | (m/z) 293 ($M^+$), 203, 161 | | | |

EXAMPLE 98

The filtrate (A) from Example 96 and the methanol-washed waste were combined and 10 g of 35% hydrogen peroxide aqueous solution was added thereto and stirred for one full day. After completion of reaction, 100 ml of water was added and crystals were taken out by filtration and fully washed with water and then with 30 ml of methanol to obtain 2.4 g of 4-(2,4-dioxo-5-thiazolidinylidene)thiochroman 1,1-dioxide.

Physico-chemical properties:

(i) m.p.: 212 to 220°C

(ii) Elementary analysis (in the form of $C_{12}H_9NO_4S_2$)

| | C | H | N | S |
|---|---|---|---|---|
| Theoretical value | 48.80 | 3.07 | 4.74 | 21.71 |
| Measured value | 48.90 | 3.13 | 4.58 | 21.65 |

(iii) Mass spectrometric value (m/z) 295 ($M^+$), 230, 160, 115

EXAMPLE 99 (A)

2.0 g of 4-(2,4-dioxo-5-thiazolidinylidene)thiochroman 1,1-dioxide as obtained in Example 98 was dissolved in 70 ml of acetic acid under heating, filtered by treatment with 0.5 g of carbon and left in this condition overnight. Crystals (B) were taken out by filtration from the remaining filtrate (C). Crystals (B) were repeatedly recrystallized from acetic acid to obtain 50 mg of (E)-4-(2,4-dioxo-5-thiazolidinylidene)thiochroman 1,1-dioxide.

Physico-chemical properties are shown in the following Table.

EXAMPLE 99 (B)

The filtrate (C) from Example 99 (A) was left for 3 hours, and the crystals precipitated were taken out by filtration and repeatedly recrystallized from acetic acid to obtain 100 mg of (Z)-4-(2,4-dioxo-5-thiazolidinylidene)thiochroman 1,1-dioxide.

Physico-chemical properties are shown in the following Table.

| Example No. | 99(A) |
|---|---|
| Chemical name | (E)-4-(2,4-dioxo-5-thiazolidinylidene)-thiochroman 1,1-dioxide |
| Structural formula | 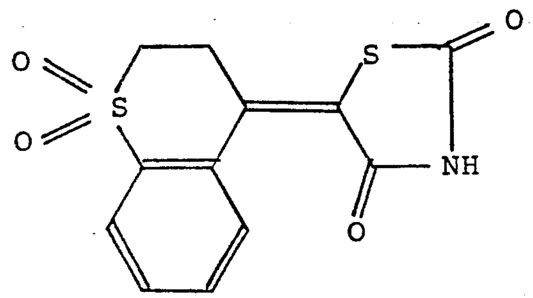 |
| m.p. (°C) | 239-240 |
| Solvent for recrystallization | AcOH |
| Molecular formula | $C_{12}H_9NO_4S_2$ |
| Elementary analysis<br>-----------<br>Calculated value (%)<br>-----------<br>Measured value (%) | C H N S<br><br>48.80 3.07 4.74 21.71<br><br>48.90 2.90 4.53 21.85 |
| Mass spectrometric value | (m/z) 295($M^+$), 230, 160, 115 |
| NMR-spectrum δ(ppm) $d_6$-DMSO, TMS internal standard | 3.04 (2H, t, $-CH_2-$)<br>3.55 (2H, t, $-CH_2-$) |

| | |
|---|---|
| Example No. | 99(B) |
| Chemical name | (Z)-4-(2,4-dioxo-5-thiazolidinylidene)-thiochroman 1,1-dioxide |
| Structural formula | 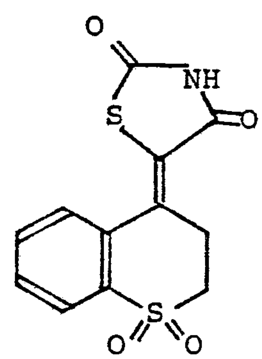 |
| m.p. (°C) | 219-220 |
| Solvent for recrystallization | AcOH |
| Molecular formula | $C_{12}H_9NO_4S_2$ |
| Elementary analysis | C H N S |
| Calculated value (%) | 48.80 3.07 4.74 21.71 |
| Measured value (%) | 48.69 2.90 4.53 21.85 |
| Mass spectrometric value | (m/z) 295($M^+$), 230, 160, 115 |
| NMR-spectrum δ(ppm) $d_6$-DMSO, TMS internal standard | 3.5 to 3.9 (4H, m, $-CH_2CH_2-$) |

EXAMPLE 100

1 g of 2,4-dioxo-5-(4-thiochromanylidene)thiazolidine as obtained in Example 33 was dissolved in 50 ml of acetic acid and then catalytically hydrogenated with 2 g of 10% palladium-carbon as a catalyst. After the acetic acid was distilled out, the product was subjected to silicagel chromatography (silicagel: 100 g, eluent: chloroform) to obtain 50 mg of caramel-like 2,4-dioxo-5-(4-thiochromanyl)-thiazolidine. Physico-chemical properties of the product are shown in the following Table.

EXAMPLE 101

20 ml of acetic acid, 1.33 g of rhodanine, 1.0 g of ammonium acetate and 1.7 g of 3-methylthiochroman-4-one were put into a glass tube and sealed and heated in an oil bath at 200°C for 10 hours. After cooling, an excess aqueous sodium hydrogencarbonate was added to make the content alkaline, and the reaction mixture was extracted with 300 ml of ethyl acetate. The resulting extract was twice washed with 100 ml of saturated aqueous sodium hydrogencarbonate; then the ethyl acetate was distilled out under reduced pressure, and the residue was subjected to silicagel chromatography (silicagel: 100 g, eluent: chloroform containing 1% methanol) to obtain 100 mg of 5-(3-methylthiochroman-4-ylidene)-4-oxo-2-thioxo-thiazolidine. Physico-chemical properties of the

product are shown in the following Table.

EXAMPLE 102

3.55 g 4-oxo-5-(2-phenyl-4-thiochromanylidene)-2-thioxo-thiazolidine as obtained in Example 42 was dissolved in 50 ml of acetic acid, and 1 ml of 30% hydrogen peroxide aqueous solution was added thereto and the whole was stirred for one full day at room temperature. 100 ml of water was added thereto, and the whole was stirred further for 3 hours, and then the crystals precipitated were taken out by filtration, washed with 300 ml of water and dried, and thereafter recrystallized from chloroform, to obtain 2.0 g of 2,4-dioxo-5-(2-phenyl-4-thiochromanylidene)-thiazolidine. Physico-chemical properties of the product are shown in the following Table.

EXAMPLE 103

In the same manner as Example 102, 100 mg of 5-(2-methyl-4-thiochromanylidene)-2,4-dioxothiazolidine was obtained from 150 mg of 5-(2-methyl-4-thiochromanylidene)-4-oxo-2-thioxothiazolidine of Example 40. Physico-chemical properties of the product are shown in the following Table.

EXAMPLE 104

In the same manner as Example 102, 50 mg of 4-(2,4-dioxo-5-thiazolidinylidene)thiochromane 1,1-dioxide was obtained from 300 mg of 4-(4-oxo-2-thioxo-5-thiazolidinyli-

dene)thiochroman 1,1-dioxide of Example 43. The physico-chemical properties of the product were the same as obtained in Example 98.

EXAMPLE 105

263 mg of 2,4-dioxo-5-(4-thiochromanylidene)thiazolidine as obtained in Example 33 and 300 mg of Lawesson's reagent were heated in 20 ml of toluene for 5 hours under reflux. After cooling, 50 ml of ethyl acetate was added thereto, and the reaction mixture was twice washed with 50 ml of water. The organic layer was dried under reduced pressure, and the residue was subjected to silicagel chromatography (silicagel: 100 g, eluent: chloroform containing 1% methanol) to obtain 150 mg of 4-oxo-5-(4-thiochromanylidene)-2-thioxo-thiazolidine. The physico-chemical properties of the product were the same as obtained in Example 20.

EXAMPLES 106 AND 107

In the same manner as Example 98, the compounds of Examples 106 and 107 were obtained, from starting compounds 5-(2,4-dioxo-5-thiazolidinylidene)-2,3,4,5-tetrahydro-1-benzothiepine 1-oxide as obtained in Example 97 and 2,4-dioxo-5-(2-phenyl-4-thiochromanylidene)thiazolidine as obtained in Example 103, respectively. Physico-chemical properties of these products are shown in the following Table.

EXAMPLE 108

0.25 g of dicyclohexylcarbodiimide was added to 4 ml of dimethylformamide solution containing 0.4 g of p-[4-oxo-5-(4-thiochromanylidene)-2-thioxo-3-thiazolidinyl]benzoic acid as obtained in Example 17 and 0.16 g of 1-hydroxybenzotriazole at room temperature and stirred for 30 minutes. To the resulting solution was dropwise added 1 ml of dimethylformamide solution containing 0.13 g of 1-(3-aminopropyl)-imidazole, and after addition, the whole was stirred for 1 hour at room temperature. The urea precipitated was filtered off; water was added to the filtrate, and this was extracted with ethyl acetate. The organic layer was washed with saturated aqueous sodium hydrogencarbonate and then with saturated salt solution, and thereafter dried with anhydrous magnesium sulfate. The solvent was distilled out under reduced pressure, and the resulting residue was subjected to silicagel chromatography. The crystals obtained from the eluate with an eluent of chloroform/methanol (10/1, by volume ratio) were washed with chloroform to obtain 0.2 g of 3-[p-[N-[3-(1-imidazolyl)propyl]carbamoyl]phenyl]-4-oxo-5-(4-thiochromanylidene)-2-thioxo-thiazolidine. Physico-chemical properties of the product are shown in the following Table.

EXAMPLE 109

1.33 g of rhodanine and 1.96 g of thiochroman-4-one

1,1-dioxide were added to 20 ml of tetrahydrofuran solution containing 5.68 g of boron trifluoride/ethylether complex. While stirring under cooling with ice, 2.02 g of triethylamine was dropwise added to the solution. After the addition, the whole was stirred for 72 hours at room temperature. The reaction mixture was poured into ice-water, and the crystals precipitated were taken out by filtration, washed with ethyl acetate and then recrystallized from a mixed solvent of ethanol/dimethylformamide, to obtain 1.40 g (45.0%) of the product 4-(4-oxo-2-thioxo-5-thiazolidinylidene)thiochroman 1,1-dioxide. The physico-chemical properties of the product were the same as obtained in Example 43.

EXAMPLE 110 TO 112

In the same manner as Example 109, the products of the Examples 110 to 112 were obtained. The physico-chemical properties of the products were shown in the following Table.

EXAMPLE 113

In the same manner as Example 20, the compound of Example 113 was obtained. The physico-chemical property thereof was shown in the following Table.

EXAMPLE 114

In the same manner as Example 55, the compound of Example 114 was obtained. The physico-chemical property thereof was shown in the following Table.

| Example No. | 100 | | | |
|---|---|---|---|---|
| Chemical name | 2,4-dioxo-5-(4-thiochromanyl)thiazolidine | | | |
| Structural formula | 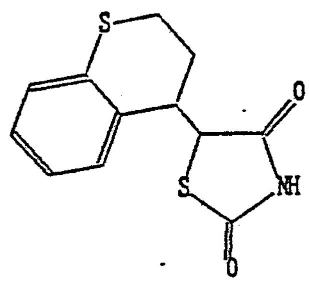 | | | |
| m.p. (°C) | — | | | |
| Solvent for recrystallization | Amorphous powder | | | |
| Molecular formula | C12 H11 N O2 S2 | | | |
| Elementary analysis | C | H | N | S |
| Calculated value (%) | 54.32 | 4.18 | 5.28 | 24.16 |
| Measured value (%) | 54.22 | 4.00 | 5.19 | 14.08 |
| Mass spectrometric value | (m/z) 265 (M+), 149, 116 | | | |

| Example No. | 101 | | | |
|---|---|---|---|---|
| Chemical name | 5-(3-methylthiochroman-4-ylidene)-4-oxo-2-thioxo-thiazolidine | | | |
| Structural formula | 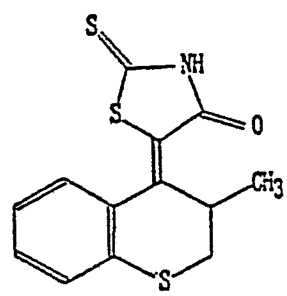 | | | |
| m.p. (°C) | 197-203 | | | |
| Solvent for recrystallization | $CHCl_3$ | | | |
| Molecular formula | C13 H11 N 0 S3 | | | |
| Elementary analysis | C | H | N | S |
| Calculated value (%) | 53.21 | 3.78 | 4.77 | 32.78 |
| Measured value (%) | 53.56 | 3.60 | 4.64 | 32.52 |
| Mass spectrometric value | (m/z) 293 ($M^+$), 201 | | | |

| | | | | |
|---|---|---|---|---|
| Example No. | 102 | | | |
| Chemical name | 2,4-dioxo-5-(2-phenyl-4-thiochromanylidene)thiazolidine | | | |
| Structural formula | 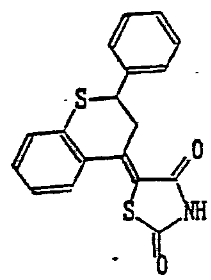 | | | |
| m.p. (°C) | 175-176 | | | |
| Solvent for recrystallization | $CHCl_3$ | | | |
| Molecular formula | C18 H13 N 02 S2 | | | |
| Elementary analysis | C | H | N | S |
| Calculated value (%) | 63.69 | 3.86 | 4.13 | 18.89 |
| Measured value (%) | 63.94 | 3.81 | 4.10 | 18.72 |
| Mass spectrometric value | (m/z) 339 ($M^+$), 248 | | | |

| Example No. | 103 | | | |
|---|---|---|---|---|
| Chemical name | 5-(2-methyl-4-thiochromanylidene)-2,4-dioxo-thiazolidine | | | |
| Structural formula | 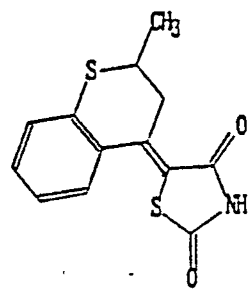 | | | |
| m.p. (°C) | 136-137 | | | |
| Solvent for recrystallization | Ether | | | |
| Molecular formula | C13 H11 N O2 S2 | | | |
| Elementary analysis | C | H | N | S |
| Calculated value (%) | 56.29 | 4.00 | 5.05 | 23.12 |
| Measured value (%) | 56.13 | 3.93 | 5.19 | 22.94 |
| Mass spectrometric value | (m/z) 277 ($M^+$), 202 | | | |

| Example No. | 106 | | | |
|---|---|---|---|---|
| Chemical name | 5-(2,4-dioxo-5-thiazolidinylidene)-2,3,4,5-tetrahydro-2H-benzothiepine 1,1-dioxide | | | |
| Structural formula | 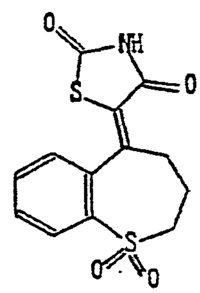 | | | |
| m.p. (°C) | 250< | | | |
| Solvent for recrystallization | MeOH | | | |
| Molecular formula | C13 H11 N 04 S2 | | | |
| Elementary analysis | C | H | N | S |
| Calculated value (%) | 50.47 | 3.58 | 4.53 | 20.73 |
| Measured value (%) | 50.59 | 3.66 | 4.61 | 20.83 |
| Mass spectrometric value | (m/z) 309 ($M^+$), 238, 173 | | | |

| | |
|---|---|
| Example No. | 107 |
| Chemical name | 4-(2,4-dioxo-5-thiazolidinylidene)-2-phenyl-thiochroman 1,1-dioxide |
| Structural formula | 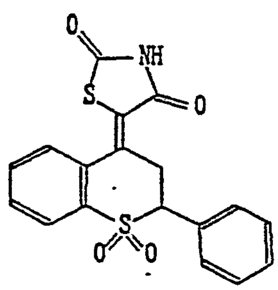 |
| m.p. (°C) | 241-242 |
| Solvent for recrystallization | EtOAc |
| Molecular formula | C18 H13 N 04 S2 |
| Elementary analysis | C　H　N |
| Calculated value (%) | 58.21　3.53　3.77 |
| Measured value (%) | 58.06　3.41　3.83 |
| Mass spectrometric value | (m/z) 372 (M+1) |

| Example No. | 108 | | | |
|---|---|---|---|---|
| Chemical name | 3-[p-[N-[3-(1-imidazolyl)propyl]-carbamoyl]phenyl]-4-oxo-5-(4-thiochromanylidene)-2-thioxo-thiazolidene | | | |
| Structural formula | 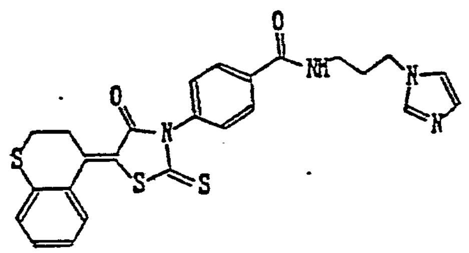 | | | |
| m.p. (°C) | 152-154 (dec.) | | | |
| Solvent for recrystallization | | | | |
| Molecular formula | C25 H22 N4 O2 S3 | | | |
| Elementary analysis | C | H | N | S |
| Calculated value (%) | 59.27 | 4.38 | 11.06 | 18.98 |
| Measured value (%) | 58.98 | 4.21 | 11.21 | 19.05 |
| Mass spectrometric value | (m/z) 507 (M⁺+1) | | | |

| Example No. | 110 | | | | |
|---|---|---|---|---|---|
| Chemical name | 6-chloro-4-(4-oxo-2-thioxo-5-thiazolidinylidene)thiochroman 1,1-dioxide | | | | |
| Structural formula | 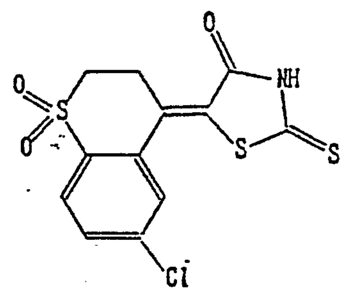 | | | | |
| m.p. (°C) | 245-247 (dec.) | | | | |
| Solvent for recrystallization | EtOAc | | | | |
| Molecular formula | C12 H8 Cl N O3 S3 | | | | |
| Elementary analysis | C | H | Cl | N | S |
| Calculated value (%) | 41.67 | 2.33 | 10.25 | 405 | 27.81 |
| Measured value (%) | 41.51 | 2.38 | 10.39 | 3.90 | 27.90 |
| Mass spectrometric value | (m/z) 345 (M$^+$) | | | | |

| Example No. | 111 | | | | |
|---|---|---|---|---|---|
| Chemical name | 7-chloro-4-(4-oxo-2-thioxo-5-thiazolidinylidene)thiochroman 1,1-dioxide | | | | |
| Structural formula | 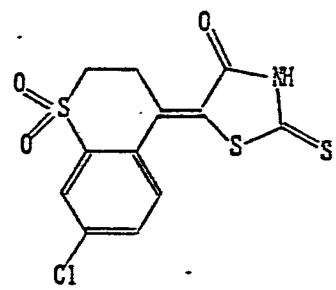 | | | | |
| m.p. (°C) | 270-273 (dec.) | | | | |
| Solvent for recrystallization | Dioxane | | | | |
| Molecular formula | C12 H8 Cl N O3 S3 | | | | |
| Elementary analysis | C | H | Cl | N | S |
| Calculated value (%) | 41.67 | 2.33 | 10.25 | 4.05 | 27.81 |
| Measured value (%) | 41.40 | 2.29 | 10.51 | 3.95 | 27.60 |
| Mass spectrometric value | (m/z) 345 (M+) | | | | |

| Example No. | 112 | | | | |
|---|---|---|---|---|---|
| Chemical name | 8-chloro-4-(4-oxo-2-thioxo-5-thiazolidinylidene)thiochroman 1,1-dioxide | | | | |
| Structural formula | 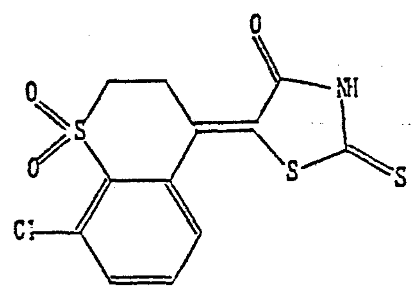 | | | | |
| m.p. (°C) | 265-270 (dec.) | | | | |
| Solvent for recrystallization | Dioxane | | | | |
| Molecular formula | C12 H8 Cl N O3 S3 | | | | |
| Elementary analysis | C | H | Cl | N | S |
| Calculated value (%) | 41.67 | 2.33 | 10.25 | 4.05 | 27.81 |
| Measured value (%) | 41.64 | 2.17 | 10.43 | 3.92 | 27.58 |
| Mass spectrometric value | (m/z) 345 ($M^{+}$) | | | | |

| Example No. | 113 | | | |
|---|---|---|---|---|
| Chemical name | 4-[3-(2-carboxyethyl)-4-oxo-2-thioxo-5-thiazolidinylidene)-thiochroman 1,1-dioxide | | | |
| Structural formula | 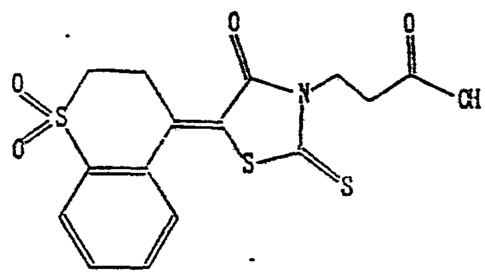 | | | |
| m.p. (°C) | Amorphous powder | | | |
| Solvent for recrystallization | — | | | |
| Molecular formula | C15 H13 N O5 S3 | | | |
| Elementary analysis | C | H | N | S |
| Calculated value (%) | 46.98 | 3.42 | 3.65 | 25.09 |
| Measured value (%) | 46.77 | 3.41 | 3.47 | 24.80 |
| Mass spectrometric value | (m/z) 383 (M$^+$), 160 | | | |

| Example No. | 114 | | | |
|---|---|---|---|---|
| Chemical name | 3-(4-oxo-2-thioxo-5-oxazolidinylidene)-2-indolinone | | | |
| Structural formula | 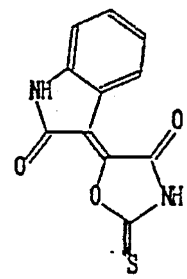 | | | |
| m.p. (°C) | 200< | | | |
| Solvent for recrystallization | AcOH | | | |
| Molecular formula | C11 H6 N2 O3 S | | | |
| Elementary analysis | C | H | N | S |
| Calculated value (%) | 53.65 | 2.46 | 11.38 | 13.02 |
| Measured value (%) | 53.58 | 2.44 | 11.02 | 13.01 |
| Mass spectrometric value | (m/z) 246 ($M^+$), 175 | | | |

EXAMPLE 115

27.8 g of 4-(4-oxo-2-thioxo-5-thiazolidinylidene)-thiochroman 1,1-dioxide was added to 2000 ml of acetic acid and suspended, and 30 ml of bromine was gradually and dropwise added thereto while stirring under reflux. After completion of the reaction, the whole was left cooled. The crystals precipitated were taken out by filtration and washed with 50 ml of hot acetic acid and dried to obtain 21 g of 3-bromo-4-(4-oxo-2-thioxo-5-thiazolidinylidene)thiochroman 1,1-dioxide.

Physico-chemical properties of the product are shown in the following Table.

EXAMPLES 116 TO 118

In the same manner as Example 115, the compounds of Examples 116 to 118 were obtained. Physico-chemical properties of these compounds are given in the following Table.

EXAMPLE 119

27.8 g of 3-bromo-4-(4-oxo-2-thioxo-5-thiazolidin-ylidene)thiochroman 1,1-dioxide was suspended in 200 ml of acetic acid and 8 g of pyridine and stirred for 10 hours under reflux. After cooling, crystals were taken out by filtration, washed with 50 ml of hot acetic acid and dried to obtain 14.3 g of 4-(4-oxo-2-thioxo-5-thiazolidin-ylidene)thiochroman 1,1-dioxide. Physico-chemical properties of the product are shown in the following Table.

EXAMPLES 120 TO 123

In the same manner as Example 119, the compounds of Examples 120 to 123 were obtained. Physico-chemical properties of these compounds are given in the following Table.

EXAMPLE 124

3.45 g of 7-chloro-4-(4-oxo-2-thioxo-5-thiazolidinylidene)thiochroman 1,1-dioxide was suspended in 50 ml of water and, while stirring, 5 g of bromine was gradually and dropwise added thereto. After the addition, the whole was continuously stirred for 47 hours, and then the insoluble product was taken out by filtration and recrystallized from acetic acid to obtain 3.1 g of 7-chloro-4-(2,4-dioxo-5-thiazolidinylidene)thiochroman 1,1-dioxide. Physico-chemical properties of the product are shown in the following Table.

EXAMPLE 125

10 g of 4-(2,4-dioxo-5-thiazolidinylidene)thiochromene 1,1-dioxide was dissolved in 20 ml of bromine and left for 2 days. The excess bromine was distilled out under reduced pressure, and 30 ml of acetic acid was added to the crystalline residue and stirred for one day to powder the said residue, and then the resulting powdery product was taken out by filtration, washed with 20 ml of hot acetic

acid and dried, to obtain 9.5 g of 2,3-dibromo-4-(2,4-dioxo-5-thiazolidinylidene)thiochroman 1,1-dioxide. Physico-chemical properties of the product are shown in the following Table.

EXAMPLE 126

6.23 g of 4-(4-oxo-2-thioxo-5-thiazolidinylidene)-thiochroman 1,1-dioxide was dissolved in 200 ml of 0.5N-sodium hydroxide aqueous solution in a nitrogen-stream atmosphere at 5°C or lower. The whole was further stirred for 15 minutes at 15°C, and then 10 ml of concentrated hydrochloric acid was added to make the resulting solution acidic. Thereafter, the solution was extracted with 300 ml of ethyl acetate and then twice washed with 200 ml of saturated salt solution. 50 g of anhydrous sodium sulfate and 5 g of active charcoal were added to the ethyl acetate extract and then immediately filtered. The filtrate was concentrated to 50 ml and the crystals precipitated were taken by filtration to obtain 3.1 g of 4-(4-oxo-thioxo-5-thiazolidinyl)-2H-benzothiopyran 1,1-dioxide. Physico-chemical properties of the product are shown in the following Table.

EXAMPLE 127

2.1 g of 4-oxothiochroman-8-carboxylic acid, 1.5 g of rhodanine and 1.4 g of ammonium acetate were added to 40 ml of acetic acid and stirred for 8 hours at 140°C under

reflux. The crystals precipitated were taken out by filtration while heating and washed with 50 ml of hot acetic acid to obtain 1.5 g of 4-(4-oxo-2-thioxo-5-thiazolidinylidene)thiochroman-8-carboxylic acid. Physico-chemical properties of the product are shown in the following Table.

EXAMPLES 128 AND 129

In the same manner as Example 127, the compounds of Examples 128 and 129 were obtained. Physico-chemical properties of these compounds are given in the following Table.

| Example No. | 115 | | | | |
|---|---|---|---|---|---|
| Chemical name | 3-bromo-4-(4-oxo-2-thioxo-5-thiazolidinylidene)thiochroman 1,1-dioxide | | | | |
| Structural formula | 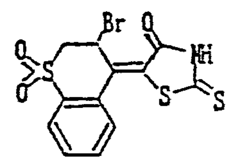 | | | | |
| m.p. (°C) | 240 (dec.) | | | | |
| Solvent for recrystallization | Dioxane | | | | |
| Molecular formula | $C_{12}H_8NO_3S_3Br$ | | | | |
| Elementary analysis | C | H | N | S | Br |
| Calculated value (%) | 36.93 | 2.07 | 3.59 | 24.65 | 20.47 |
| Measured value (%) | 37.04 | 2.17 | 3.42 | 24.79 | 20.43 |
| Mass spectrometric value | 391 (M+2), 389 (M⁺), 309, 222 | | | | |

| Example No. | 116 | | | | |
|---|---|---|---|---|---|
| Chemical name | 3-bromo-4-(2,4-dioxo-5-thiazolidinylidene)thiochroman 1,1-dioxide | | | | |
| Structural formula | 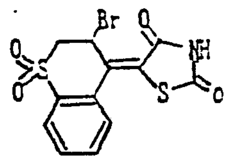 | | | | |
| m.p. (°C) | 330 (dec.) | | | | |
| Solvent for recrystallization | AcOH | | | | |
| Molecular formula | $C_{12}H_8NO_4S_2Br$ | | | | |
| Elementary analysis | C | H | N | S | Br |
| Calculated value (%) | 38.51 | 2.15 | 3.74 | 17.14 | 21.35 |
| Measured value (%) | 38.33 | 2.21 | 3.67 | 16.92 | 21.34 |
| Mass spectrometric value | 375 (M+2), 373 ($M^+$), 293 | | | | |

| | |
|---|---|
| Example No. | 117 |
| Chemical name | 3-bromo-7-chloro-4-(4-oxo-2-thioxo-5-thiazolidinylidene)thiochroman 1,1-dioxide |
| Structural formula | 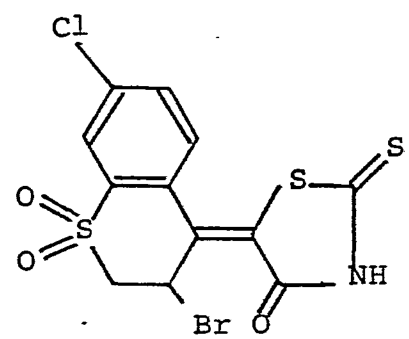 |
| m.p. (°C) | 200 < |
| Solvent for recrystallization | |
| Molecular formula | $C_{12}H_7NO_3S_3BrCl$ |

| Elementary analysis | C | H | N | S | Br |
|---|---|---|---|---|---|
| Calculated value (%) | 33.93 | 1.66 | 3.30 | 22.65 | 18.81 |
| Measured value (%) | 33.84 | 1.52 | 3.24 | 22.55 | 19.00 |

| | |
|---|---|
| Mass spectrometric value | |

| | |
|---|---|
| Example No. | 118 |
| Chemical name | 3-bromo-7-chloro-4-(2,4-dioxo-5-thiazolidinylidene)thiochroman 1,1-dioxide |
| Structural formula | 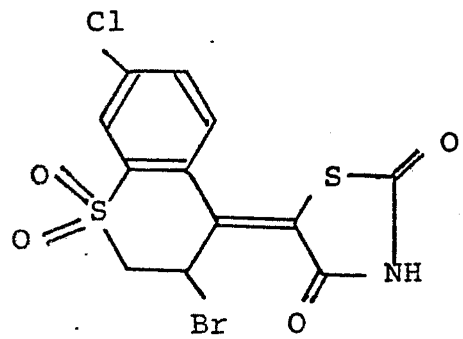 |
| m.p. (°C) | 200 < |
| Solvent for recrystallization | |
| Molecular formula | $C_{12}H_7NO_4S_2BrCl$ |

| Elementary analysis | C | H | N | S | Br |
|---|---|---|---|---|---|
| Calculated value (%) | 35.27 | 1.73 | 3.43 | 15.69 | 19.55 |
| Measured value (%) | 35.21 | 1.69 | 3.50 | 15.73 | 19.23 |
| Mass spectrometric value | | | | | |

| Example No. | 119 |
|---|---|
| Chemical name | 4-(4-oxo-2-thioxo-5-thiazolidinyl-idene)thiochromene 1,1-dioxide |
| Structural formula | 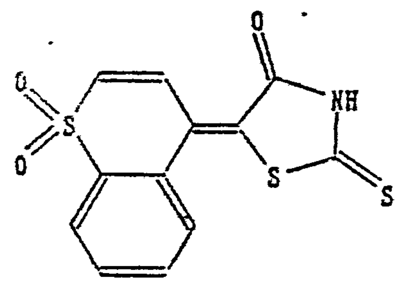 |
| m.p. (°C) | 269 (dec) |
| Solvent for recrystallization | AcOH |
| Molecular formula | C12 H7 N 03 S3 |

| Elementary analysis | C | H | N | S |
|---|---|---|---|---|
| Calculated value (%) | 46.59 | 2.28 | 4.53 | 31.09 |
| Measured value (%) | 46.77 | 2.38 | 4.46 | 31.12 |

| Mass spectrometric value | 309 ( $M^+$ ), 222 |
|---|---|

| Example No. | 120 |
|---|---|
| Chemical name | 4-(2,4-dioxo-5-thiazolidinylidene)-thiochromene 1,1-dioxide |
| Structural formula | 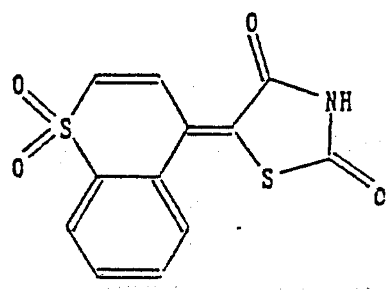 |
| m.p. (°C) | >250 (dec.) |
| Solvent for recrystallization | AcOH |
| Molecular formula | $C_{12}H_7NO_4S_2$ |
| Elementary analysis | C H N S |
| Calculated value (%) | 49.14 2.41 4.78 21.86 |
| Measured value (%) | 48.94 2.43 4.74 21.70 |
| Mass spectrometric value | 293 ( $M^+$ ), 222 |

| | |
|---|---|
| Example No. | 121 |
| Chemical name | 7-chloro-4-(2,4-dioxo-5-thiazolidinylidene)thiochromene 1,1-dioxide |
| Structural formula | 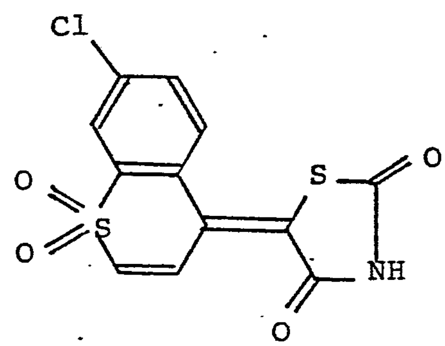 |
| m.p. (°C) | 250 < |
| Solvent for recrystallization | |
| Molecular formula | $C_{12}H_6ClNO_4S_2$ |
| Elementary analysis | C H N S Cl |
| Calculated value (%) | 43.97 1.85 4.27 19.57 10.82 |
| Measured value (%) | 43.74 1.91 4.21 19.78 10.79 |
| Mass spectrometric value | 329(M+), 327(M+), 256 |

| | | | | | |
|---|---|---|---|---|---|
| Example No. | 122 | | | | |
| Chemical name | 7-chloro-4-(4-oxo-2-thioxo-5-thiazolidinylidene)thiochromene 1,1-dioxide | | | | |
| Structural formula | 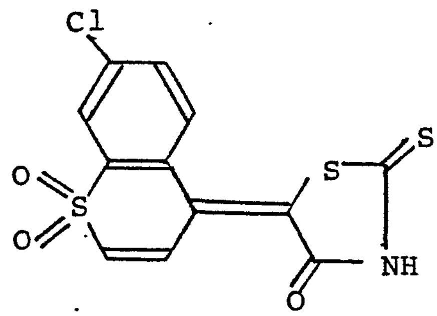 | | | | |
| m.p. (°C) | 200< | | | | |
| Solvent for recrystallization | | | | | |
| Molecular formula | $C_{12}H_6ClO_3S_3$ | | | | |
| Elementary analysis | C | H | N | S | Cl |
| Calculated value (%) | 41.92 | 1.76 | 4.07 | 27.98 | 10.31 |
| Measured value (%) | 41.77 | 1.75 | 3.99 | 28.16 | 10.34 |
| Mass spectrometric value | 343(M⁻), 256 | | | | |

| | |
|---|---|
| Example No. | 123 |
| Chemical name | 2-bromo-4-(2,4-dioxo-5-thiazolidinylidene)thiochromene 1,1-dioxide |
| Structural formula | 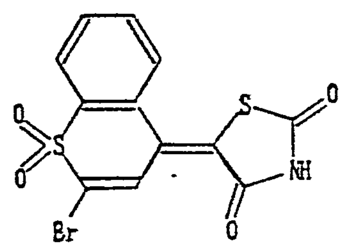 |
| m.p. (°C) | 280 – 285 ( dec ) |
| Solvent for recrystallization | |
| Molecular formula | $C_{12}H_5NO_4S_2Br$ |
| Elementary analysis | C / H / N / S / Br |
| Calculated value (%) | 38.83 / 1.36 / 3.77 / 17.28 / 21.53 |
| Measured value (%) | 38.88 / 1.64 / 3.47 / 17.03 / 21.39 |
| Mass spectrometric value | |

| | | | | | |
|---|---|---|---|---|---|
| Example No. | 124 | | | | |
| Chemical name | 7-chloro-4-(2,4-dioxo-5-thiazolidinylidene)thiochroman 1,1-dioxide | | | | |
| Structural formula | 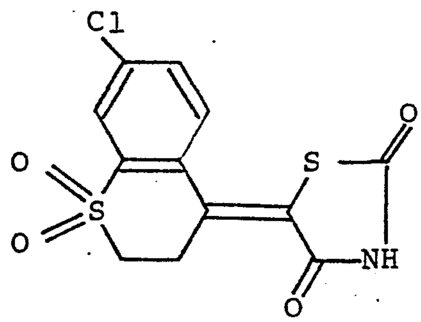 | | | | |
| m.p. (°C) | 245 (dec) | | | | |
| Solvent for recrystallization | | | | | |
| Molecular formula | $C_{12}H_8ClNO_4S_2$ | | | | |
| Elementary analysis | C | H | N | S | Cl |
| Calculated value (%) | 43.70 | 2.45 | 4.25 | 19.45 | 10.75 |
| Measured value (%) | 43.49 | 2.55 | 4.14 | 19.22 | 10.88 |
| Mass spectrometric value | 330 ($M^{+1}$) | | | | |

| Example No. | 125 | | | | |
|---|---|---|---|---|---|
| Chemical name | 2,3-dibromo-4-(2,4-dioxo-5-thiazolidinylidene)thiochroman 1,1-dioxide | | | | |
| Structural formula | 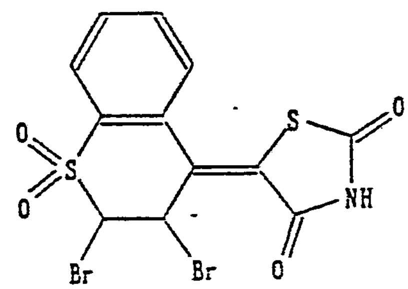 | | | | |
| m.p. (°C) | 225 – 230 ( dec ) | | | | |
| Solvent for recrystallization | | | | | |
| Molecular formula | C12 H7 Br2 N O4 S2 | | | | |
| Elementary analysis | C | H | N | S | Cl |
| Calculated value (%) | 31.81 | 1.56 | 3.09 | 14.15 | 35.27 |
| Measured value (%) | 31.85 | 1.52 | 2.81 | 13.91 | 35.53 |
| Mass spectrometric value | 455(M+4), 453(M+2), 451($M^+$) | | | | |

| Example No. | 126 |
|---|---|
| Chemical name | 4-(4-oxo-2-thioxo-5-thiazolidinyl)-2H-benzothiopyran 1,1-dioxide |
| Structural formula | 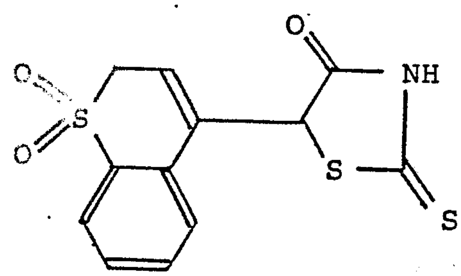 |
| m.p. (°C) | 255(dec), (after partially molten at 199°C and then solidified) |
| Solvent for recrystallization | EtOAc or acetone |
| Molecular formula | $C_{12}H_9NO_3S_3$ |
| Elementary analysis | C H N S |
| Calculated value (%) | 46.28 2.91 4.50 30.89 |
| Measured value (%) | 46.26 3.04 4.32 30.59 |
| Mass spectrometric value | 311($M^+$), 246, 160 |
| NMR-spectrum $\delta$(ppm) $d_6$-DMSO, TMS internal standard | 4.36(2H, d, $-SO_2CH_2-$)<br>6.28(1H, s, >—H)<br>6.52(1H, t, =<$CH_2$– H) |

| Example No. | 127 |
|---|---|
| Chemical name | 4-(4-oxo-2-thioxo-5-thiazolidinylidene)thiochroman-8-carboxylic acid |
| Structural formula | 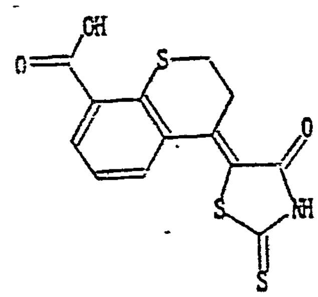 |
| m.p. (°C) | 231 |
| Solvent for recrystallization | AcOH |
| Molecular formula | C13 H9 N O3 S3 |
| Elementary analysis | C H N S |
| Calculated value (%) | 48.28 2.80 4.33 29.74 |
| Measured value (%) | 48.34 2.89 4.29 29.53 |
| Mass spectrometric value | 323 ($M^+$), 277, 231 |

| Example No. | 128 |
|---|---|
| Chemical name | 4-(4-oxo-2-thioxo-5-thiazolidinyl-idene)thiochroman-8-carboxylic acid 1,1-dioxide acetic·acid |
| Structural formula | 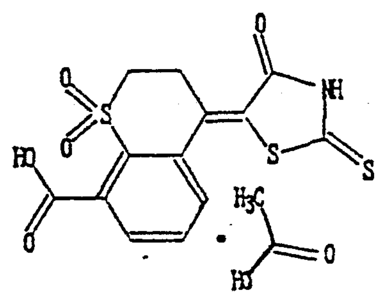 |
| m.p. (°C) | 275 (dec) |
| Solvent for recrystallization | AcOH |
| Molecular formula | C15 H13 N 07 S3 |
| Elementary analysis | C / H / N / S |
| Calculated value (%) | 43.36 / 3.15 / 3.37 / 23.15 |
| Measured value (%) | 43.30 / 3.07 / 3.23 / 23.30 |
| Mass spectrometric value | 355 ($M^+$), 291, 204 |

| Example No. | 129 |
|---|---|
| Chemical name | 3,4-dihydro-7-methoxy-4-(4-oxo-2-thioxo-5-thiazolidinylidene)-2H-1-benzothiopyran 1,1-dioxide |
| Structural formula | 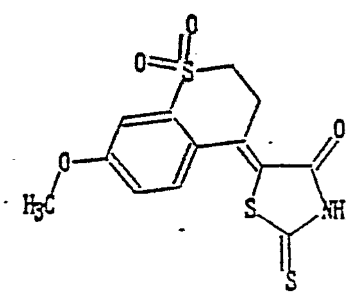 |
| m.p. (°C) | 286-288 (dec) |
| Solvent for recrystallization | Dioxane |
| Molecular formula | C13 H11 N O4 S3 |
| Elementary analysis | C H N S |
| Calculated value (%) | 45.73 3.25 4.10 28.17 |
| Measured value (%) | 45.70 3.12 4.02 28.16 |
| Mass spectrometric value | 341 ( $M^+$ ) |

EXAMPLE 130

2.95 g of (Z)-4-(2,4-dioxo-5-thiazolidinylidene)-thiochroman 1,1-dioxide as obtained in Example 99 (B) was dissolved in 10 ml of 28% $NH_4OH$, left for 3 days, and then made acidic with dilute hydrochloric acid. The crystals as precipitated were taken out by filtration and subjected to silicagel column-chromatography (silicagel: 300 g, eluent: chloroform containing 5% acetic acid). The resulting eluate was crystallized from ethyl acetate to obtain 1.1 g of 4-(2,4-dioxo-5-thiazolidinyl)-2H-1-benzopyran 1,1-dioxide.

Physico-chemical properties of the product are as follows:

| Example No. | 130 | | | |
|---|---|---|---|---|
| Chemical name | 4-(2,4-dioxo-5-thiazolidinyl)-2H-1-benzopyran 1,1-dioxide | | | |
| Structural formula | 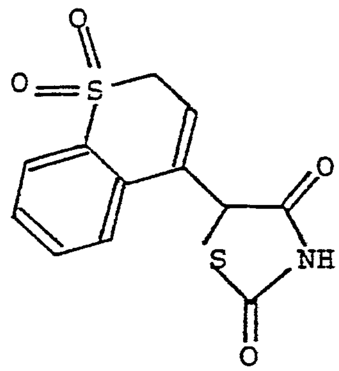 | | | |
| m.p. (°C) | 196 to 199 | | | |
| Solvent for recrystallization | Ethyl acetate | | | |
| Molecular formula | $C_{12}H_9NO_4S_2$ | | | |
| Elementary analysis | C | H | N | S |
| Calculated value (%) | 48.80 | 3.07 | 4.74 | 21.71 |
| Measured value (%) | 48.63 | 3.14 | 4.61 | 21.70 |
| Mass spectrometric value | 295($M^+$), 230, 160, 115 | | | |
| NMR-spectrum δ(ppm) $d_6$-DMSD, TMS internal standard | 4.36(2H, d, $-SO_2CH_2-$)<br>6.17(1H, s, >—H)<br>6.57(1H, t, =<$CH_2$ H) | | | |

EXAMPLE 131

2.95 g of (Z)-4-(2,4-dioxo-5-thiazolidinylidene)-thiochroman 1,1-dioxide as obtained in Example 99 (B) was heated for 3 days under reflux while irradiating with UV-lamp (UVL-400P, by Ricoh). Acetic acid was distilled out, and then the residue was subjected to silicagel column-chromatography (silicagel: 300 g, eluent: chloroform containing 5% acetic acid). The resulting eluate was crystallized from ethyl acetate to obtain 0.5 g of 4-(2,4-dioxo-5-thiazolidinyl)-2H-1-benzopyran 1,1-dioxide. The physicochemical properties of the product were the same as obtained in Example 130.

EXAMPLE 132

2.95 g of 4-(2,4-dioxo-5-thiazolidinyl)-2H-1-benzopyran 1,1-dioxide as obtained in Example 130 was dissolved in 60 ml of acetic acid under heating and irradiated with UV-lamp (UVL-400P, by Richo) for 3 days and then cooled. The crystals precipitated were repeatedly recrystallized from acetic acid to obtain 0.2 g of (E)-4-(2,4-dioxo-5-thiazolidinylidene)thiochroman 1,1-dioxide. The mother liquor was dried and the resulting solid was repeatedly recrystallized from dioxane to obtain 0.5 g of (Z)-4-(2,4-dioxo-5-thiazolidinylidene)thiochroman 1,1-dioxide. The physicochemical properties of the products were the same as obtained in Examples 99 (A) and 99 (B), respectively.

EXAMPLE 133

2.95 g of 4-(2,4-dioxo-5-thiazolidinyl)-2H-1-benzopyran 1,1-dioxide as obtained in Example 130 was heated and melted at 205°C in an argon atmosphere for 3 hours. After cooling, the product was recrystallized from dioxane and then heated and dried to obtain 1.5 g of (Z)-4-(2,4-dioxo-5-thiazolidinylidene)thiochroman 1,1-dioxide. The mother liquor after recrystallization was dried and the resulting residue was repeatedly recrystallized from acetic acid to obtain 0.1 g of (E)-4-(2,4-dioxo-5-thiazolidinylidene)thiochroman 1,1-dioxide. The physico-chemical properties of these products were the same as those obtained in Examples 99 (B) and 99 (A), respectively.

EXAMPLE 134

4-(2,4-dioxo-5-thiazolidinyl)-2H-1-benzopyran, 1,1-dioxide as obtained in Example 130 was dissolved in 28% $NH_4OH$ and left for 3 days. Thereafter, the solution was made acidic with dilute hydrochloric acid and the crystals precipitated were taken out by filtration, recrystallized from dioxane and taken out by filtration, and then heated and dried to obtain 1.1 g of (Z)-4-(2,4-dioxo-5-thiazolidinylidene)thiochroman 1,1-dioxide. The mother liquor after crystallization was repeatedly recrystallized from acetic acid to obtain 0.2 g of (E)-4-(2,4-dioxo-5-thiazolidinylidene)thiochroman 1,1-dioxide. The physico-chemical properties of these products were the same as those obtained in Examples 99 (B) and 99 (A), respectively.

EXAMPLE 135

2.95 g of (Z)-4-(2,4-dioxo-5-thiazolidinylidene)-thiochroman 1,1-dioxide as obtained in Example 99 (B) was dissolved in 600 g of acetic acid under heating, irradiated with UV-lamp (UVL-400P, by Ricoh) for 3 days, and then cooled. The crystals precipitated were repeatedly recrystallized from acetic acid to obtain 0.5 g of (E)-4-(2,4-dioxo-5-thiazolidinylidene)thiochroman 1,1-dioxide. The physico-chemical properties of the product were the same as obtained in Example 99 (A).

EXAMPLE 136

2.95 g of (E)-4-(2,4-dioxo-5-thiazolidinylidene)-thiochroman 1,1-dioxide as obtained in Example 99 (A) was dissolved in 50 ml of dioxane under heating, irradiated with UV-lamp (UVL-400P, by Ricoh) for 3 days, concentrated to 20 ml and cooled. The crystals precipitated were taken out by filtration, heated and dried to obtain 1.5 g of (Z)-4-(2,4-dioxo-5-thiazolidinylidene)thiochroman 1,1-dioxide. The physico-chemical properties of the product were the same as obtained in Example 99 (B).

EXAMPLE 137

2.95 g of (E)-4-(2,4-dioxo-5-thiazolidinylidene)-

thiochroman 1,1-dioxide as obtained in Example 99 (A) was dissolved in 60 ml of acetic acid and heated for one week under reflux, and then acetic acid was distilled out. The residue was recrystallized from dioxane, heated and dried to obtain 1.2 g of (Z)-4-(2,4-dioxo-5-thiazolidinylidene)thiochroman 1,1-dioxide. The physico-chemical properties of the product were the same as obtained in Example 99 (B).

EXAMPLE 138

3.11 g of 4-(4-oxo-2-thioxo-5-thiazolidinylidene)-thiochroman 1,1-dioxide as obtained in Example 43 was suspended in 50 ml of water and stirred for 3 days in a chlorine gas atmosphere. After completion of the reaction, crystals were taken out by filtration and recrystallized from dioxane, heated and dried to obtain 2.0g of (Z)-4-(2,4-dioxo-5-thiazolidinylidene)thiochroman 1,1-dioxide. The physico-chemical properties of the product were the same as obtained in the Example 99 (B).

EXAMPLE 139

3.11 g of 4-(4-oxo-2-thioxo-5-thiazolidinylidene)-thiochroman 1,1-dioxide as obtained in Example 43 was suspended in 50 ml of water, 10 g of NBS was added and the mix was closely sealed and stirred for 3 days. After the completion of the reaction, crystals were taken out by filtration and recrystallized from dioxane, heated and dried to obtain 1.9 g of (Z)-4-(2,4-dioxo-5-thiazolidinylidene)-

thiochroman 1,1-dioxide. The physico-chemical properties of the product were the same as obtained in Example 99 (B).

EXAMPLE 140

3.11 g of 4-(4-oxo-2-thioxo-5-thiazolidinylidene)-thiochroman 1,1-dioxide as obtained in Example 43 was suspended in 50 ml of water, 10 g of $NaBrO_2 \cdot 3H_2O$ was added and the mix was closely sealed and stirred for 3 days. After completion of the reaction, crystals were taken out by filtration and recrystallized from dioxane, heated and dried to obtain 1.5 g of (Z)-4-(2,4-dioxo-5-thiazolidinylidene)-thiochroman 1,1-dioxide. The physico-chemical properties of the product were the same as obtained in Example 99 (B).

EXAMPLE 141

2.9 g of 4-(2-imino-4-oxo-5-thiazolidinylidene)thiochroman 1,1-dioxide as obtained in Example 95 was heated and stirred together with 15 ml of 14N-$H_2SO_4$ for 10 hours in a steam bath. After completion of the reaction, 100 ml of water was added and the resulting mixture was thrice extracted with 100 ml of ethyl acetate. The ethyl acetate was distilled out under reduced pressure, and the residue was recrystallized from dioxane, heated and dried to obtain 1.9 g of (Z)-4-(2,4-dioxo-5-thiazolidinylidene)thiochroman 1,1-dioxide. The physico-chemical properties of the product were the same as obtained in Example 99 (B).

EXAMPLE 142

2.79 g of 4-oxo-5-(4-thiochromanylidene)-2-thioxothiazolidine as obtained in Example 20 was dissolved in 15 ml of acetic acid, and 10 ml of 35%-hydrogen peroxide solution was added thereto and stirred for 3 days at room temperature. After completion of the reaction, 100 ml of water was added; cooling with ice gave crystals which were taken out by filtration, recrystallized from dioxane, heated and dried to obtain 1.3 g of (Z)-4-(2,4-dioxo-5-thiazolidinylidene)thiochroman 1,1-dioxide. The physico-chemical properties of the product were the same as obtained in Example 99 (B).

EXAMPLE 143

3.11 g of 4-(4-oxo-2-thioxo-5-thiazolidinylidene)-thiochroman 1,1-dioxide as obtained in Example 43 was blended with 10 g of mono-bromo-acetic acid and 10 ml of water and heated and stirred for 10 hours under reflux. Thereafter, 100 ml of water was added followed by cooling. The crystals precipitated were taken out by filtration, recrystallized from dioxane, heated and dried to obtain 2.1 g of (Z)-4-(2,4-dioxo-5-thiazolidinylidene)thiochroman 1,1-dioxide. The physico-chemical properties of the product were the same as obtained in Example 99 (B).

EXAMPLE 144

2.95 g of (Z)-4-(2,4-dioxo-5-thiazolidinylidene)-

thiochroman 1,1-dioxide as obtained in Example 99 (B) and 20 ml of 0.1N aqueous sodium hydroxide were stirred for 2 days at room temperature. The precipitate as formed was taken out by filtration, washed with water and recrystallized from an appropriate amount of water to obtain 2.0 g of sodium (Z)-4-(2,4-dioxo-5-thiazolidinylidene)thiochroman 1,1-dioxide. The physico-chemical properties of the product were as follows:

(i) m.p.: 200°C or higher

(ii) Elementary analysis (in the form of $C_{12}H_8NO_4S_2Na$):

| | C (%) | H (%) | N (%) | S (%) |
|---|---|---|---|---|
| Theoretical value | 45.42 | 2.54 | 4.41 | 20.21 |
| Measured value | 45.40 | 2.46 | 4.33 | 20.49 |

(iii) Mass spectrometric value (m/z): 318 ($M^{+1}$)

EXAMPLE 145

In the same manner as Example 144, potassium (Z)-4-(2,4-dioxo-5-thiazolidinylidene)thiochroman 1,1-dioxide was obtained, which had the following physico-chemical properties:

(i) m.p.: 200°C or higher

(ii) Elementary analysis (in the form of $C_{12}H_8NO_4S_2K$):

| | C (%) | H (%) | N (%) | S (%) |
|---|---|---|---|---|
| Theoretical value | 43.23 | 2.42 | 4.20 | 19.23 |
| Measured value | 43.22 | 2.39 | 4.17 | 19.35 |

(iii) Mass spectrometric value (m/z): 334 ($M^{+1}$)

## EXAMPLE 146

In the same manner as Example 55, 3-(4-oxo-2-thioxo-3-thiazolidinylidene)-2-indolinone was obtained using isatin as a raw material. Physico-chemical properties of the product are shown in the following Table.

| | |
|---|---|
| Example No. | 146 |
| Chemical name | 3-(4-oxo-2-thioxo-3-thiazolidinylidene)-2-indolinone |
| Structural formula | 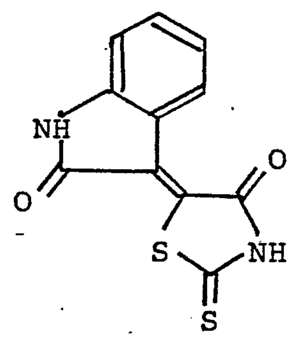 |
| m.p. (°C) | 300 |
| Solvent for recrystallization | AcOH |
| Molecular formula | $C_{11}H_6N_2O_2S_2$ |

| Elementary analysis | C | H | N | S |
|---|---|---|---|---|
| Calculated value (%) | 50.37 | 2.31 | 10.68 | 24.45 |
| Measured value (%) | 50.49 | 2.10 | 10.74 | 24.30 |

| | |
|---|---|
| Mass spectrometric value | (m/z) 262($M^+$), 175 |

EXAMPLE 147

| | |
|---|---|
| Product obtained in Example 99 (B) | 100.0 mg |
| Hydroxypropyl cellulose with low substitution degree | 35.9 mg |
| Lactose | 18.0 mg |
| Methyl cellulose | 5.4 mg |
| Crystalline cellulose | 18.0 mg |
| Calcium stearate | 0.9 mg |
| Talc | 1.8 mg |
| | 180.0 mg |

The above active compound, hydroxypropyl cellulose with low substitution degree and lactose were blended in the above proportions and aqueous methyl cellulose was added thereto. After kneading and drying the crystalline cellulose, calcium stearate and talc were added and blended, to obtain tablets having a weight of 180 mg and a diameter of 8 mm.

EXAMPLE 148

| | |
|---|---|
| Product obtained in Example 99 (B) | 100.0 mg |
| Crystalline Lactose | 99.0 mg |
| Calcium stearate | 1.0 g |
| | 200.0 mg |

The above active compound, crystalline lactose and calcium stearate were blended in the above proportions and the resulting mixture was filled in No. 3 gelatin capsule

coats to obtain capsules.

EXAMPLES 149 TO 151

In the same manner as Example 1, the compounds of Examples 149 to 151 were obtained. Physico-chemical properties of these compounds are given in the following Table.

| Example No. | 149 | | | | |
|---|---|---|---|---|---|
| Chemical name | 5-(6,7-dichloro-4-thiochromanylidene)-4-oxo-2-thioxothiazolidine | | | | |
| Structural formula | 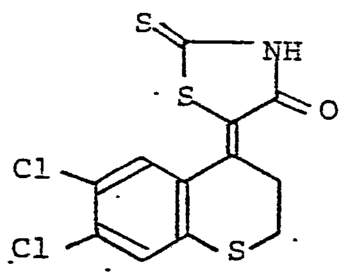 | | | | |
| m.p. (°C) | 226-228 | | | | |
| Solvent for recrystallization | MeOH | | | | |
| Molecular formula | $C_{12}H_7NOS_3Cl_2$ | | | | |
| Elementary analysis | C | H | N | S | Cℓ |
| Calculated value (%) | 41.38 | 2.03 | 4.02 | 27.62 | 20.36 |
| Measured value (%) | 41.28 | 2.11 | 3.93 | 27.64 | 20.26 |
| Mass spectrometric value | (m/z) 347($M^+$) | | | | |

| Example No. | 150 | | | | |
|---|---|---|---|---|---|
| Chemical name | 5-(7-bromo-4-thiochromanylidene)-4-oxo-2-thioxothiazolidine | | | | |
| Structural formula | 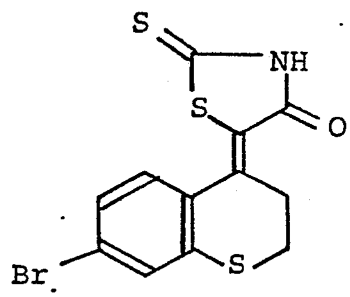 | | | | |
| m.p. (°C) | 210-211 | | | | |
| Solvent for recrystallization | MeOH | | | | |
| Molecular formula | $C_{12}H_8NOS_3Br$ | | | | |
| Elementary analysis | C | H | N | S | Br |
| Calculated value (%) | 40.23 | 2.25 | 3.91 | 26.85 | 22.30 |
| Measured value (%) | 40.17 | 2.17 | 3.91 | 26.84 | 22.43 |
| Mass spectrometric value | (m/z) 357($M^+$), 359($M^{+2}$) | | | | |

| Example No. | 151 | | | | |
|---|---|---|---|---|---|
| Chemical name | 5-(7-chloro-4-thiochromanylidene)-4-oxo-2-thioxothiazolidine | | | | |
| Structural formula | 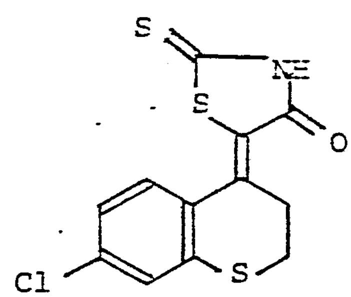 | | | | |
| m.p. (°C) | 194-195 | | | | |
| Solvent for recrystallization | MeOH | | | | |
| Molecular formula | $C_{12}H_8NOS_3Cl$ | | | | |
| Elementary analysis | C | H | N | S | Cl |
| Calculated value (%) | 45.92 | 2.57 | 4.46 | 30.65 | 11.30 |
| Measured value (%) | 45.83 | 2.49 | 4.39 | 30.58 | 11.55 |
| Mass spectrometric value | (m/z) 313($M^+$) | | | | |

While the invention has been described in detail and with reference to specific embodiments thereof, it will be apparent to one skilled in the art that various changes and modifications can be made therein without departing from the spirit and scope thereof.

0237138

CLAIMS:

1. A heterocyclic compound selected from those of formula (I), tautomers thereof, and pharmaceutically acceptable salts and esters of formula (I) compounds and tautomers:

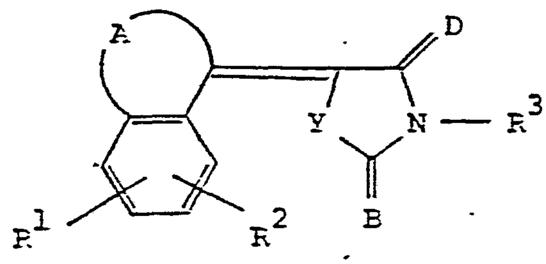

(I)

wherein A represents an alkylene or alkenylene chain which has 1 to 4 carbon atoms and which may have a hetero atom; B, D and Y are selected independently from oxygen and sulfur atoms and NH; $R^1$ and $R^2$ are selected independently from hydrogen and halogen atoms and $C_1$ to $C_5$ alkyl, $C_1$ to $C_5$ alkoxy, $C_1$ to $C_5$ alkylthio, phenyl, nitro, hydroxyl, sulfo, imidazolyl-$C_2$ to $C_5$ acylamimo, amino and carboxyl groups; and $R^3$ represents a hydrogen atom, an amino group, a $C_1$ to $C_5$ alkyl group which may be substituted by a carboxyl group, or a phenyl group which may be substituted by a carboxyl, a carboxy-$C_1$ to $C_5$ alkyl, or an imidazolyl-$C_1$ to $C_5$ alkylamide group.

2. A compound according to claim 1 wherein A represents an alkylene or alkenylene chain having an oxygen or sulfur atom or a sulfinyl (-SO-), sulfonyl ($-SO_2$) or imino (-NH-) group, and is preferably $-SO_2CH_2CH_2-$.

3. A compound according to claim 1 or 2 wherein A represents an alkylene or alkenylene chain with a substituent selected from halogen atoms and $C_1$ to $C_5$ alkyl, phenyl, oxo and 2,4-dioxo-5-thiazolidinylidene groups.

4. A compound according to claim 1 selected from those of formula (I-a), tautomers thereof, and pharmaceutically acceptable salts and esters of formula (I-a) compounds and tautomers:

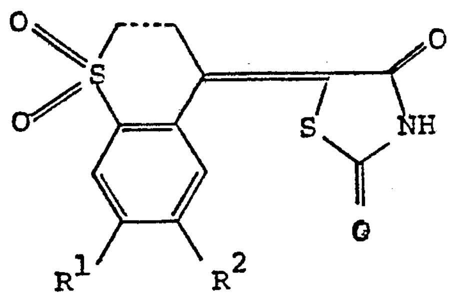

(I-a)

wherein $R^1$ and $R^2$ are selected independently from hydrogen and halogen atoms; and the dotted line means a single or double bond.

5. A compound according to claim 1 selected from

4-(4-oxo-2-thioxo-5-thiazolidinylidene)thiochroman 1,1-dioxide;

4-(2,4-dioxo-5-thiazolidinylidene)thiochroman 1,1-dioxide;

(Z)-4-(2,4-dioxo-5-thiazolidinylidene)thiochroman 1,1-dioxide;

7-chloro-4-(4-oxo-2-thioxo-5-thiazolidinylidene)thiochroman-1,1-dioxide;

4-(4-oxo-2-thioxo-5-thiazolidinylidene)thiochromene 1,1-dioxide;

4-(2,4-dioxo-5-thiazolidinylidene)thiochromene 1,1-dioxide;

7-chloro-4-(2,4-dioxo-5-thiazolidinylidene)thiochromene 1,1-dioxide;

7-chloro-4-(4-oxo-2-thioxo-5-thiazolidinylidene)thiochroman-1,1-dioxide;

7-chloro-4-(2,4-dioxo-5-thiazolidinylidene)thiochroman 1,1-dioxide;

7-chloro-4-(2,4-dioxo-5-thiazolidinylidene)thiochroman;

6,7-dichloro-4-(2,4-dioxo-5-thiazolidinylidene)thiochroman;

7-bromo-4-(2,4-dioxo-5-thiazolidinylidene)thiochroman;

7-chloro-4-(2-thio-4-oxo-5-thiazolidinylidene)thiochroman;

(E)-4-(2,4-dioxo-5-thiazolidinylidene)thiochroman 1,1-dioxide;

8-chloro-4-(2,4-dioxo-5-thiazolidinylidene)thiochroman 1,1-dioxide;

6-chloro-4-)2,4-dioxo-5-thiazolidinylidene)thiochroman 1,1-dioxide;

tautomers of said compounds; and pharmaceutically acceptable salts and esters of said compounds and tautomers.

6. A method for the preparation of a heterocyclic compound selected from those of formula (I), tautomers thereof, and pharmaceutically acceptable salts and esters of said formula (I) compounds and tautomers:

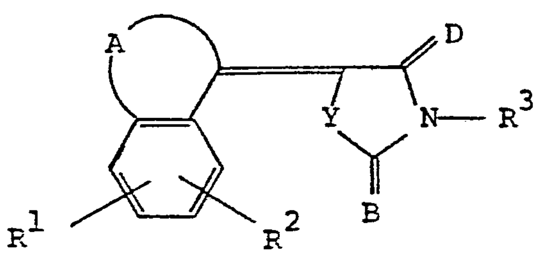

(I)

which comprises reacting (preferably in the presence of base or Lewis acid) ketone compound (II):

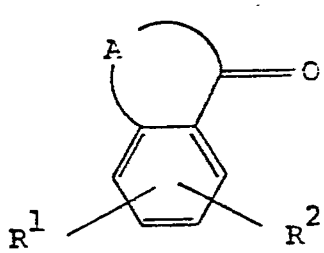

(II)

with heterocyclic compound (III):

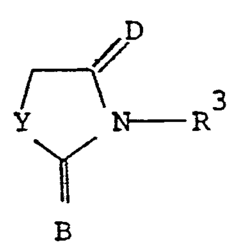

(III)

and optionally tautomerising the product and/or converting to or from salt or ester form, wherein A represents an alkylene or alkenylene chain which has 1 to 4 carbon atoms and which may have a hetero atom; B, D and Y are selected independently from oxygen and sulfur atoms and NH; $R^1$ and $R^2$ are selected independently from hydrogen and halogen atoms and $C_1$ to $C_5$ alkyl, $C_1$ to $C_5$ alkoxy, $C_1$ to $C_5$ alkylthio, phenyl, nitro, hydroxyl, sulfo, imidazolyl-$C_2$ to $C_5$ acylamimo, amino and carboxyl groups; and $R^3$ represents a hydrogen atom, an amino group, a $C_1$ to $C_5$ alkyl group which may be substituted by a carboxyl group, or a phenyl group which may be substituted by a carboxyl, a carboxy-$C_1$ to $C_5$ alkyl, or an imidazolyl-$C_1$ to $C_5$ alkylamide group.

7. A method for the preparation of a heterocyclic compound selected from those of formula (I-1), tautomers thereof and pharmaceutically acceptable salts and esters of said formula (I-1) compounds and tautomers:

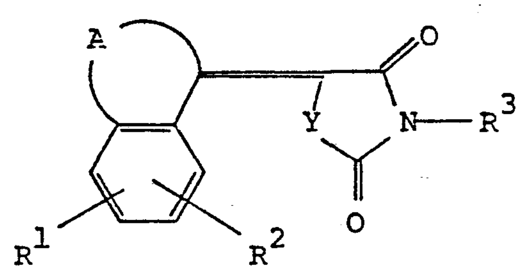

(I-1)

which comprises hydrolysing the imino group(s) of imine compound (IV):

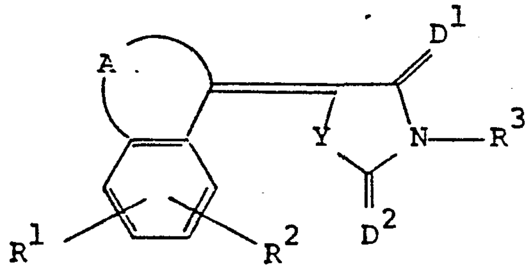

(IV)

and optionally tautomerising the product and/or converting to or from salt or ester form, wherein A, Y, $R^1$, $R^2$ and $R^3$ are as defined in claim 6; and one of $D^1$ and $D^2$ represents an imino group and the other represents an oxygen atom, or both represent imino groups.

8. A method for the preparation of a heterocyclic compound selected from those of formula (I-2), tautomers thereof, and pharmaceutically acceptable salts and esters of said formula (I-2) compounds and tautomers:

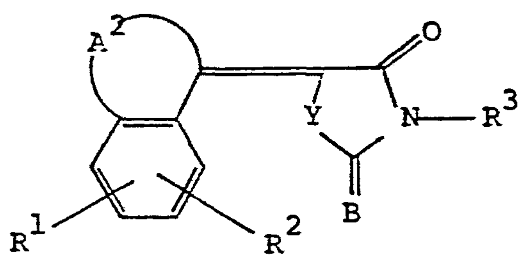

(I-2)

which comprises oxidizing compound (V):

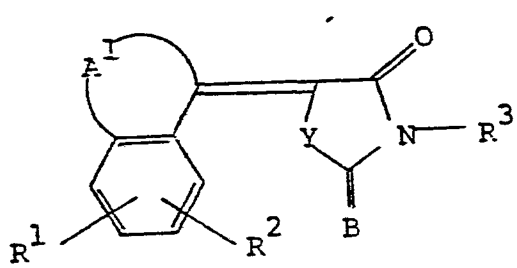

(V)

and optionally tautomerising the product and/or converting to or from salt or ester form, wherein B, Y, $R^1$, $R^2$ and $R^3$ are as defined in claim 6; $A^2$ represents an alkylene or alkenylene chain which has 1 to 4 carbon atoms and a sulfinyl or sulfonyl group; and $A^1$ represents an alkylene or alkenylene chain which has 1 to 4 carbon atoms and a sulfur atom or a sulfinyl group.

9. A method for the preparation of a heterocyclic compound selected from those formula (I-4), tautomers thereof, and pharmaceutically acceptable salts and esters of said formula (I-4) compounds and tautomers:

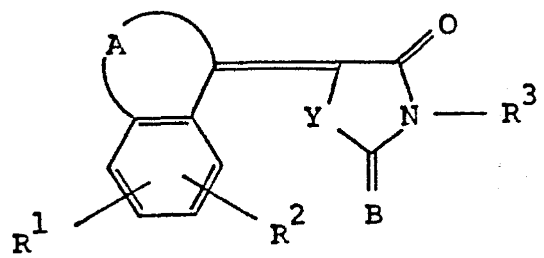

(I-4)

which comprises oxidizing compound (VI):

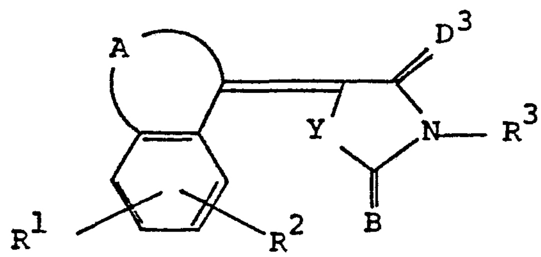

(VI)

and optionally tautomerising the product and/or converting to or from salt or ester form, wherein A, B, Y, $R^1$, $R^2$ and $R^3$ are as defined in claim 6; and $D^3$ represents an oxygen atom or a sulfur atom provided that it represents a sulfur atom when B represents an oxygen atom or NH.

10. A method for the preparation of a heterocyclic compound selected from those of formulae (I-3) and (VII), and pharmaceutically acceptable salts and esters thereof, which comprises isomerising formula (I-3) compound to formula (VII) compound or vice-versa and optionally converting to or from salt or ester form:

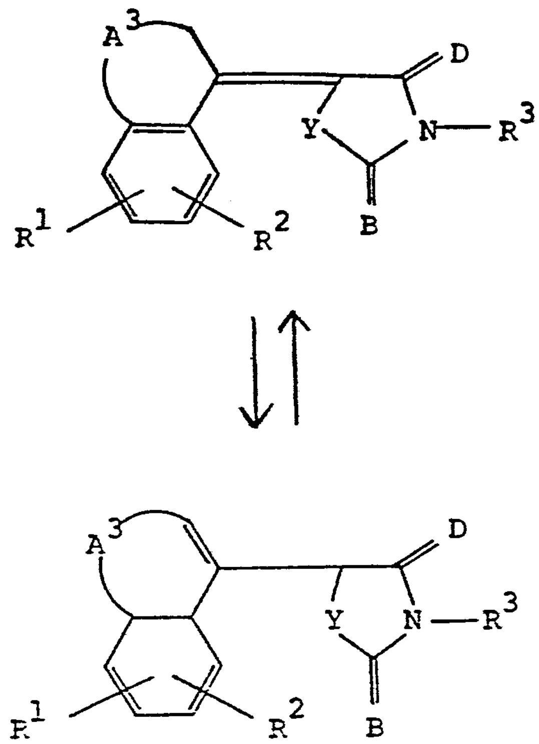

(I-3)

(VII)

wherein $A^3$ represents an alkylene or alkenylene chain which has 1 to 3 carbon atoms and which may have a hetero atom; and B, D, Y, $R^1$, $R^2$ and $R^3$ are as defined in claim 6.

11. A pharmaceutical composition containing active ingredient selected from heterocyclic compounds according to any of claims 1 to 5 (preferably wherein D is an oxygen atom).

12. The use of heterocyclic compound according to any of claims 1 to 5, preferably wherein D is oxygen, for the manufacture of a medicament for aldose reductase inhibition or for platelet aggregation inhibition or for lipid lowering.

European Patent Office

# EUROPEAN SEARCH REPORT

0237138

Application number

EP 87 30 0109

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | CHEMICAL AND PHARMACEUTICAL BULLETIN, vol. 24, no. 10, 1976, pages 2305-2311, Tokyo, JP; T. SAWAYAMA et al.: "The structures of isatylidene-3-mercaptoacetic acid and its related compounds" * page 2306, chart 2, formation of compound (X) * | 1-3,6 | C 07 D 417/04<br>C 07 D 277/36<br>C 07 D 417/14<br>C 07 D 403/04<br>C 07 D 409/04<br>C 07 D 413/04<br>A 61 K 31/425<br>A 61 K 31/415<br>A 61 K 31/55<br>A 61 K 31/42 |
| X,D | CHEMICAL ABSTRACTS, vol. 74, no. 5, 1st February 1971, page 305, column 2, abstract no. 22751a, Columbus, Ohio, US; V. OSKAJA et al.: "Condensation of dicarboxylic acid anhydrides with compounds containing active methylene groups. XIII. Condensation of phthalic anhydride with 2-thio-4-thiazolidone and 2,4-thiazolidinedione", & LATV. PSR ZINAT. AKAD. VESTIS, KIM. SER. 1970, (4), 475-478 | 1-3 | TECHNICAL FIELDS SEARCHED (Int. Cl.4)<br>C 07 D 277/00<br>C 07 D 403/00<br>C 07 D 409/04<br>C 07 D 413/04<br>C 07 D 417/04<br>C 07 D 417/14 |
| X | US-A-4 110 536 (H.J. HAVERA et al.) * example 1 * | 1,2 | |
| X | US-A-4 381 308 (R.C. SCHNUR) * claim 1; column 11, table I, compound with 3-benzo(b)thienyl group * | 1,2 | |

--- -/-

The present search report has been drawn up for all claims

| Place of search | Date of completion of the search | Examiner |
|---|---|---|
| BERLIN | 06-04-1987 | HASS C V F |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number 0237138

EP 87 30 0109

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | EP-A-0 047 109 (ONO PHARMACEUTICAL CO., LTD.) * claims 1, 5, 53, 54 * --- | 1,6,11,12 | |
| A | US-A-4 177 282 (R. SARGES) * claim 1 * --- | 1,11,12 | |
| A | EP-A-0 091 761 (SENJU SEIYAKU K.K.) * claim 1; abstract * --- | 1,11,12 | |
| A,D | CHEMICAL ABSTRACTS, vol. 92, no. 11, 17th March 1980, page 582, column 1, abstract no. 94286x, Columbus, Ohio, US; P.N. STEBLYUK et al.: "Synthesis, fungistatic, and antimicrobial activity of compounds containing two thiazolidine rings in the molecules", & FIZIOL. AKT. VESHCHESTVA 1979, 11, 97-101 ----- | 1,11 | TECHNICAL FIELDS SEARCHED (Int. Cl.4) |

The present search report has been drawn up for all claims

| Place of search | Date of completion of the search | Examiner |
|---|---|---|
| BERLIN | 06-04-1987 | HASS C V F |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document